# EUROPEAN PATENT SPECIFICATION

(11) **EP 1 879 884 B1**
(45) Date of publication and mention of the grant of the patent: **25.08.2010**
(21) Application number: 06727571.9
(22) Date of filing: 03.05.2006
(51) Int. Cl.: C07D 413/12, A61K 31/4439, A61P 11/00, A61P 31/00

(54) **AZOLE-BASED PHOSPHODIESTERASE INHIBITORS**
PHOSPHODIESTERASEINHIBITOREN AUF AZOLBASIS
INHIBITEURS DE LA PHOSPHODIESTERASE A BASE D'AZOLE

(30) Priority: 03.05.2005 IN DE11012005
(43) Date of publication of application: 23.01.2008
(73) Proprietor: Ranbaxy Laboratories Limited, Haryana 122001, Delhi (IN)
(72) Inventor: PALLE, Venkata G901, Sylvan Heights Sanewade, Maharashtra 411007 (IN); BALACHANDRAN, Sarala D-1302 Lakshachandi Apts., Mumbai 400063 (IN); MUTHUKAMA, Nagarajan 20/21 Malayamman Kovil St., Erode Dt., Tamil Nadu 638151 (IN); RAY, Abhijit Sector C-1, Flat No. 1408, New Delhi, Delhi 110070 (IN); DASTIDAR, Sunanda Ghose B-138, Sarita Vihar, New Delhi, Delhi 110044 (IN); RAMAIAH, Raghu, Andra Pradesh 522018 (IN)
(74) Representative: Cronin, Brian Harold John
(86) International application number: PCT/IB2006/001133
(87) International publication number: WO 2006/117653

(56) References cited:
- EP-A- 0 497 564
- WO-A-01/53274
- WO-A-95/14681
- WO-A-96/00218
- WO-A-97/03967
- US-B1- 6 303 789
- US-B1- 6 548 514

## Description

### Field of the Invention

The present invention relates to phosphodiesterase (PDE) type IV selective inhibitors.

Compounds disclosed herein can be useful in the treatment of AIDS, asthma, arthritis, bronchitis, chronic obstructive pulmonary disease (COPD), psoriasis, allergic rhinitis, shock, atopic dermatitis, Crohn's disease, adult respiratory distress syndrome (ARDS), eosinophilic granuloma, allergic conjunctivitis, osteoarthritis, ulcerative colitis or other inflammatory diseases, especially in humans.

Processes for preparing compounds described herein, pharmaceutical compositions comprising compounds described herein and their use as PDE type IV selective inhibitors are also provided.

### Background of the Invention

It is known that cyclic adenosine-3',5'-monophosphate (cAMP) exhibits an important role of acting as an intracellular secondary messenger (E.W. SFutherland, and T.W. Roll, Pharmacol. Rev, 1960,12, 265). Intracellular hydrolysis of cAMP to adenosine 5'-monophosphate (AMP) causes a number of inflammatory conditions including psoriasis, allergic rhinitis, shock, atopic dermatitis, Crohn's disease, adult respiratory distress syndrome (ARDS), eosinophilic granuloma, allergic conjunctivitis, osteoarthritis, ulcerative colitis. The most important role in the control of cAMP (as well as of cGMP) level is played by cyclic nucleotide phosphodiesterases (PDE), which represent a biochemically and functionally highly variable super family of enzyme; eleven distinct families with more than 15 gene products are currently recognized. Although PDE I, PDE II, PDE III, PDE IV, and PDE VII all use cAMP as a substrate, only PDE IV and PDE VII are highly selective for hydrolysis of cAMP. Inhibitors of PDE, particularly the PDE IV inhibitors, such as rolipram or Ro-1724, are therefore known as cAMP-enhancers. Immune cells contain type IV and type III PDE, the PDE IV type being prevalent in human mononuclear cells. Thus the inhibition of phosphodiesterase type IV has been a target for modulation and, accordingly, for therapeutic intervention in a range of disease processes.

The initial observation that xanthine derivatives, theophylline and caffeine inhibit the hydrolysis of cAMP led to the discovery of the required hydrolytic activity in the cyclic nucleotide phosphodiesterase (PDE) enzymes. More recently, distinct classes of PDE's have been recognized (J.A. Bervo and D.H. Reifsnyder, TIPS, 1990, 11, 150), and their selective inhibition has led to improved drug therapy (C.D. Nicholus, R.A. Challiss and M. Shahid, TIPS, 1991, 12, 19). Thus it was recognized that inhibition of PDE IV could lead to inhibition of inflammatory mediator release (M.W. Verghese et. al, J. Mol. Cell. Cardiol. 1989, 12 (Suppl.II), S 61) and airway smooth muscle relaxation.

Various phosphodiesterase and/or inhibitors have been disclosed in, for example, WO 94/02465, WO 95/14680, 95/14681, U.S. Patent No. 5,686,434, WO 95/24398, U.S. Patent No. 6,114,367.WO 95/20578, WO 95/04046, WO 95/04045, WO 97/03967,

WO 99/32433 discloses thiourea and benzamide compounds, compositions and methods of treating or preventing inflammatory diseases and atherosclerosis. WO 99/38845 discloses substituted diaryl compounds, which have been stated as PPAR-Gamma modulators. WO 00/55120 discloses amide derivatives and their use in the treatment of diseases or medical conditions mediated by cytokines. WO 01/93909 discloses a method for treating cancer including administering to a mammal therapeutically effective amount of an anti-neoplastic agent and a PDE IV inhibitor. WO 02/100332 discloses isoxazoline compounds, which have been stated as having macrophage inhibitory factor (MIF) antagonist activity. U.S. Patent No. 5, 935,978 discloses pharmaceutical use of compounds containing phenyl linked to aryl or heteroaryl by an aliphatic or heteroatom containing linking group for inhibiting cyclic AMP phosphodiesterase. U.S. Patent No. 6,303,789 discloses benzamides with tetrahydrofuranyloxy substituents, which have been stated as PDE IV inhibitors.

However in view of this, there remains a need for novel phosphodiesterase inhibitors.

### Summary of the Invention

In one aspect, provided are compounds having the structure of Formula I, 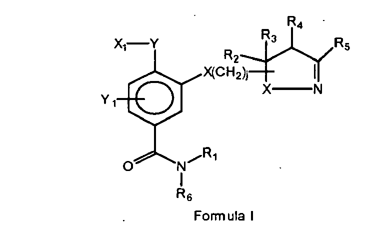 or a pharmaceutically acceptable salt, pharmaceutically acceptable solvate, stereoisomer, tautomer, racemate, polymorph or N-oxide thereof, wherein
R₁ and R₆ can independently be hydrogen, alkyl, alkenyl, alkynyl, (un)saturated)cycloalkyl, aryl, aralkyl, heterocyclyl, heteroaryl, (heteroaryl)alkyl or (heterocyclyl)alkyl,
X can be oxygen, sulfur, or NRₕ, wherein
Rₕ can be hydrogen, alkyl, alkenyl, alkynyl, (un) saturated cycloalkyl, acyl, aryl, aralkyl, heteroaryl, heterocyclyl, (heteroaryl)alkyl, (heterocyclyl)alkyl, S(O)ₙR_{d}, wherein
R_{d} can be alkyl, aryl, heterocyclyl or heteroaryl and
n can be an integer from 0-2,
Y can be no atom, oxygen, sulfur, NRₕ, wherein Rₕ is the same as defined above,
X₁ can be hydrogen, alkyl, alkenyl, alkynyl, cycloalkyl, aryl, aralkyl, heteroaryl, heterocyclyl, (heteroaryl)alkyl, (heterocyclyl)alkyl,
Y₁ can be hydrogen, alkyl, alkenyl, alkynyl, nitro, cyano, halogen, ORₕ, SRₕ, NHRₕ, COOR', COR', CONHR', S(O)ₙR_{d}, or Y₁ may be fused to benzene ring if Y₁ is aryl, heteroaryl or heterocyclyl, wherein
Rₕ and R_{d} are the same as defined above and
R' can be hydrogen, alkyl, alkenyl, alkynyl, (un)saturated cycloalkyl, aryl, aralkyl, heterocyclyl, heteroaryl, (heterocyclyl)alkyl or (heteroaryl)alkyl,
j can be an integer from 1 to 3,
R₂ and R₃ can independently be hydrogen, alkyl, alkenyl, alkynyl, cycloalkyl, nitro, cyano, amino, substituted amino, alkoxy, aryloxy, COR', COOR', aryl, aralkyl,
heteroaryl, heterocyclyl, (heteroaryl)alkyl, (heterocyclyl)alkyl, (CH₂)₁₋₄OR''', C(=O)NRₓR_{y}, (CH₂)ₘ-C(=O)R_{z}, wherein
R' is the same as defined above,
R''' can be hydrogen, alkyl, alkenyl, alkynyl, (un)saturated cycloalkyl, aryl, aralkyl, heterocyclyl, heteroaryl, (heterocyclyl)alkyl, (heteroaryl)alkyl or hydroxy,
Rₓ and R_{y} can independently be hydrogen, alkyl, alkenyl of three to six carbon atoms, alkynyl of three to six carbon atoms, (un)saturated cycloalkyl, aryl, aralkyl, heteroaryl, heterocyclyl, (heteroaryl)alkyl, or (heterocyclyl)alkyl,
m can be an integer from 0-2 and
R_{z} can be Rₚ or R_{q}, wherein
Rₚ can be a 4-12 membered (un)saturated monocyclic or bicyclic ring containing 1-4 heteroatom(s) selected from N, O or S, wherein the said ring can be attached to (CH₂)ₘC(=O) through N, and
R_{q} can be a 4-12 membered monocyclic or bicyclic ring containing 0-4 heteroatom(s) selected from N, O or S, wherein the said ring can be attached to (CH₂)ₘC(=O) through C,
R₄ can be hydrogen, alkyl, halogen, hydroxy, cyano, carboxy, nitro, C(=O)NRₓR_{y}, wherein
Rₓ and R_{y} are the same as defined above, and
R₅ can be hydrogen, alkyl, aryl, aralkyl, (un) saturated cycloalkyl, heteroaryl, heterocyclyl, (heteroaryl)alkyl, or (heterocyclyl)alkyl,
wherein R₂, R₃, R₄ or R₅ are optionally replaced by a bond, represented for example by the compounds of Formula X and Formula XXII 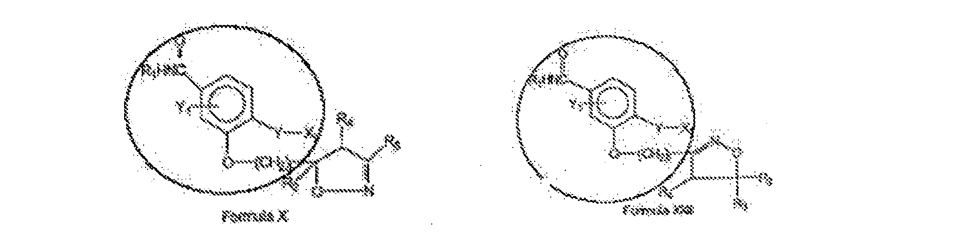 wherein R₃ and R₅ are replaced by the bond respectively.

The compounds described herein can include one or more of the following embodiments. For example, R₁ can be 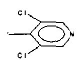 , R₄, R₆ and Y₁ can be hydrogen, X can be oxygen, Y can be no atom or oxygen, R₂ can be methyl, j can be 1, R₃ can be replaced by said bond, X₁ can be selected from hydrogen, cycloalkyl, alkyl, heterocyclyl, heteroaryl, (heterocyclyl)alkyl or (heteroaryl)alkyl and R₅ can be selected from alkyl, aryl, cycloalkyl, heterocyclyl, heteroaryl, (heterocyclyl)alkyl or (heteroaryl)alkyl.

In another embodiment, X₁ can be selected from methyl, difluoromethyl, ethyl, propyl, isopropyl, cyclopropyl, butyl, cyclopentyl, benzyl or cycloheptyl and R₅ is selected from methyl, phenyl, 3-pyridyl, 3-cyclopentyloxy-4-methoxy-phenyl, 4-difluoromethoxy-phenyl, 2-fluorophenyl, 2,6-difluorophenyl, 4-fluorophenyl, 3-fluorophenyl, 1-phenyl ethyl, cyclohexyl, p-tolyl, 4-tert-butyl phenyl, O-tolyl, 3-methoxy phenyl, piperidine-1-carboxylic acid tert-butyl ester, 3,4-difluorophenyl, 2,4-difluorophenyl, 4-fluoro-3-methyl phenyl or 3,5-difluoro phenyl.

In another embodiment, R₁ is 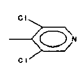 , R₄, R₆ and Y₁ can be hydrogen, X and Y can be oxygen, R₃ and X₁ can be methyl, j can be 1, R₅ can be replaced by said bond and R₂ can be selected from cyano, -COOR' or C(=O)NRₓR_{y}, wherein R', Rₓ and R_{y} can be selected from hydrogen, alkyl, alkenyl, alkynyl, (un)saturated cycloalkyl, aryl, aralkyl, heterocyclyl, heteroaryl, (heterocyclyl)alkyl or (heteroaryl)alkyl.

In another embodiment, R', Rₓ and R_{y} can be selected from methyl and ethyl.

In another aspect, provided herein are compounds selected from:
3-[3-(3-Cyclopentyloxy-4-methoxy-phenyl)-5-methyl-4,5-dihydro-isoxazol-5-ylmethoxy]-N-(3,5-dichloro-pyridin-4-yl)-4-methoxy-benzamide,
N-(3,5-dichloro-pyridin-4-yl)-3-(3,5-dimethyl-4,5-dihydro-isoxazol-5-ylmethoxy)-4-methoxy-benzamide,
3-(5-Cyano-5-methyl-4,5-dihydro-isoxazol-4-ylmethoxy)-N-(3,5-dichloro-pyridin-4-yl)-4-methoxy-benzamide,
4-[5-(3,5-Dichloro-pyridin-4-ylcarbomoyl)-2-methoxy-phenoxymethyl]-5-methyl-4,5-dihydro-isoxazole-5-carboxylic acid methyl ester,
4-[5-(3,5-Dichloro-pyridin-4-ylcarbomoyl)-2-methoxy-phenoxymethyl]-5-methyl-4,5-dihydro-isoxazole-5-carboxylic acid ethyl amide,
3-[5-(3,5-Dichloro-pyridin-4-ylcarbomoyl)-2-methoxy-phenoxymethyl]-5-methyl-4,5-dihydro-isoxazole-5-carboxylic acid,
3-[5-(3,5-Dichloro-pyridin-4-ylcarbomoyl)-2-methoxy-phenoxymethyl]-5-methyl-4,5-dihydro-isoxazole-5-carboxylic acid amide,
3-[5-(3,5-Dichloro-pyridin-4-ylcarbomoyl)-2-methoxy-phenoxymethyl]-5-methyl-4,5-dihydro-isoxazole-5-carboxylic acid methyl amide,
3-[5-(3,5-Dichloro-pyridin-4-ylcarbomoyl)-2-methoxy-phenoxymethyl]-5-methyl-4,5-dihydro-isoxazole-5-carboxylic acid dimethyl amide,
N-(3,5-dichloro-pyridin-4-yl)-3-(5-methyl-3-phenyl-4,5-dihydro-isoxazol-5-ylmethoxy)-4-methoxy-benzamide,
N-(3,5-dichloro-pyridin-4-yl)-3-(5-methyl-3-pyridin-3-yl-4,5-dihydro-isoxazol-5-ylmethoxy)-4-methoxy-benzamide,
N-(3,5-dichloro-pyridin-4-yl)-3-(3-(4-difluoromethoxy-phenyl)-5-methyl-4,5-dihydro-isoxazol-5-ylmethoxy)-4-methoxy-benzamide,
N-(3,5-dichloro-pyridin-4-yl)-4-difluoromethoxy-3-(3,5-dimethyl-4,5-dihydro-isoxazol-5-ylmethoxy)-benzamide,
N-(3,5-dichloro-pyridin-4-yl)-3-(5-methyl-3-phenyl-4,5-dihydro-isoxazol-5-ylmethoxy)-4-hydroxy-benzamide;
N-(3,5-dichloro-pyridin-4-yl)-3-[3-(2-fluoro-phenyl)-5-methyl-4,5-dihydro-isoxazol-5-ylmethoxy)-4-methoxy-benzamide,
N-(3,5-dichloro-pyridin-4-yl)-3-[3-(2,6-difluoro-phenyl)-5-methyl-4,5-dihydro-isoxazol-5-ylmethoxy)-4-methoxy-benzamide,
N-(3,5-dichloro-pyridin-4-yl)-3-[3,5-dimethyl-4,5-dihydro-isoxazol-5-ylmethoxy)-4-hydroxy-benzamide,
N-(3,5-dichloro-pyridin-4-yl)-3-[3-(4-fluoro-phenyl)-5-methyl-4,5-dihydro-isoxazo1-5-ylmethoxy)-4-methoxy-benzamide,
N-(3,5-dichloro-pyridin-4-yl)-3-[3-(3-fluoro-phenyl)-5-methyl-4,5-dihydro-isoxazol-5-ylmethoxy)-4-hydroxy-benzamide,
N-(3,5-dichloro-pyridin-4-yl)-3-[3-(3-fluoro-phenyl)-5-methyl-4,5-dihydro-isoxazol-5-ylmethoxy)-4-methoxy-benzamide,
N-(3,5-dichloro-pyridin-4-yl)-4-ethoxy-3-(5-methyl-3-phenyl-4,5-dihydro-isoxazol-5-ylmethoxy)-benzamide,
N-(3,5-dichloro-pyridin-4-yl)-4-ethoxy-3-[3-(3-fluoro-phenyl)-5-methyl-4,5-dihydro-isoxazol-5-ylmethoxy]-benzamide,
N-(3,5-dichloro-pyridin-4-yl)-3-(5-methyl-3-phenyl-4,5-dihydro-isoxazol-5-ylmethoxy)-4-propoxy-benzamide,
N-(3,5-diehloro-pyridin-4-yl)-3-[3-(3-fluoro-phenyl)-5-methyl-4,5-dihydro-isoxazol-5-ylmethoxy]-4-propoxy-benzamide,
N-(3,5-dichloro-pyridin-4-yl)-4-isopropoxy-3-(5-methyl-3-phenyl-4,5-dihydro-isoxazol-5-ylmethoxy)-benzamide,
N-(3,5-dichloro-pyridin-4-yl)-3-[3-(3-fluoro-phenyl)-5-methyl-4,5-dihydro-isoxazol-5-ylmethoxy]-4-isopropoxy-benzamide,
4-Cyclopropoxy-N-(3,5-dichloro-pyridin-4-yl)-3-(5-methyl-3-phenyl-4,5-dihydro-isoxazol-5-ylmethoxy)-benzamide,
4-Cyclopropoxy-N-(3,5-dichloro-pyridin-4-yl)-3-[3-(3-fluoro-phenyl)-5-methyl-4,5-dihydro-isoxazol-5-ylmethoxy]-benzamide,
4-Butoxy-N-(3,5-dichloro-pyridin-4-yl)-3-(5-methyl-3-phenyl-4,5-dihydro-isoxazol-5-ylmethoxy)-benzamide,
4-Butoxy-N-(3,5-dichloro-pyridin-4-yl)-3-[3-(3-fluoro-phenyl)-5-methyl-4,5-dihydro-isoxazol-5-ylmethoxy]-benzamide,
4-Cyclopentyloxy-N-(3,5-dichloro-pyridin-4-yl)-3-(5-methyl-3-phenyl-4,5-dihydro-isoxazol-5-ylmethoxy)-benzamide,
4-Cyclopentyloxy-N-(3,5-dichloro-pyridin-4-yl)-3-[3-(3-fluoro-phenyl)-5-methyl-4,5-dihydro-isoxazol-5-ylmethoxy]-benzamide,
N-(3,5-dichloro-pyridin-4-yl)-3-(3,5-dimethyl-4,5-dihydro-isoxazol-5-ylmethoxy)-4-ethoxy-benzamide,
N-(3,5-dichloro-pyridin-4-yl)-3-(3,5-dimethyl-4,5-dihydro-isoxazol-5-ylmethoxy)-4-propoxy-benzamide,
N-(3,5-dichloro-pyridin-4-yl)-4-isopropoxy-3-(5-methyl-3-pyridin-3-yl-4,5-dihydro-isoxazol-5-ylmethoxy)-benzamide,
N-(3,5-dichloro-pyridin-4-yl)-3-(3,5-dimethyl-4,5-dihydro-isoxazol-5-ylmethoxy)-4-isopropoxy-benzamide,
4-Cyclopropoxy-N-(3,5-dichloro-pyridin-4-yl)-3-(5-methyl-3-pyridin-3-yl-4,5-dihydro-isoxazol-5-ylmethoxy)-benzamide,
4-Cyclopropoxy-N-(3,5-dichloro-pyridin-4-yl)-3-(3,5-dimethyl-4,5-dihydro-isoxazol-5-ylmethoxy)-benzamide,
4-Butoxy-N-(3,5-dichloro-pyridin-4-yl)-3-(5-methyl-3-pyridin-3-yl-4,5-dihydro-isoxazol-5-ylmethoxy)-benzamide,
4-Butoxy-N-(3,5-dichloro-pyridin-4-yl)-3-(3,5-dimethyl-4,5-dihydro-isoxazol-5-ylmethoxy)-benzamide,
4-Cyclopentyloxy-N-(3,5-dichloro-pyridin-4-yl)-3-(5-methyl-3-pyridin-3-yl-4,5-dihydro-isoxazol-5-ylmethoxy)-benzamide,
4-Cyclopentyloxy-N-(3,3-dichloro-pyridin-4-yl)-3-(3,5-dimethyl-4,5-dihydro-isoxazol-5-ylmethoxy)-benzamide,
N-(3,5-dichloro-pyridin-4-yl)-4-hydroxy-3-(5-methyl-3-pyridin-3-yl-4,5-dihydro-isoxazol-5-ylmethoxy)-benzamide,
N-(3,5-dichloro-pyridin-4-yl)-4-methoxy-3-[5-methyl-3-(1-phenyl-ethyl)-4,5-dihydro-isoxazol-5-ylmethoxy]-benzamide,
3-(3-Cyclohexyl-5-methyl-4,5-dihydro-isoxazol-5-ylmethoxy)-N-(3,5-dichloro-pyridin-4-yl)-4-methoxy-benzamide,
N-(3,5-dichloro-pyridin-4-yl)-4-methoxy-3-(5-methyl-3-p-tolyl-4,5-dihydro-isoxazol-5-ylmethoxy)-benzamide,
3-[3-(4-tert-Butyl-phenyl)-5-methyl-4,5-dihydro-isoxazol-5-ylmethoxy]-N-(3,5-dichloro-pyridin-4-yl)-4-methoxy-benzamide,
N-(3,5-dichloro-pyridin-4-yl)-4-methoxy-3-(5-methyl-3-O-tolyl-4,5-dihydroisoxazol-5-ylmethoxy)-benzamide,
N-(3,5-dichloro-pyridin-4-yl)-4-methoxy-3-[3-(3-methoxy-phenyl)-5-methyl-4,5-dihydro-isoxazol-5-ylmethoxy]-benzamide,
2-{5-[5-(3,5-Dichloro-pyridin-4-ylcarbamoyl)-2-methoxy-phenoxymethyl]-5-methyl-4,5-dihydro-isoxazol-3-yl}-piperidine-1-carboxylic acid tert-butyl ester,
N-(3,5-dichloro-pyridin-4-yl)-3-[3-(3,4-difluoro-phenyl)-5-methyl-4,5-dihydro-isoxazol-5-ylmethoxy]-4-methoxy-benzamide,
N-(3,5-dichloro-pyridin-4-yl)-3-[3-(2,4-difluoro-phenyl)-5-methyl-4,5-dihydro-isoxazol-5-ylmethoxy]-4-methoxy-benzamide,
N-(3,5-dichloro-pyridin-4-yl)-3-[3-(4-fluoro-3-methyl-phenyl)-5-methyl-4,5-dihydro-isoxazol-5-ylmethoxy]-4-methoxy-benzamide,
N-(3,5-dichloro-pyridin-4-yl)-3-[3-(3,5-difluoro-phenyl)-5-methyl-4,5-dihydro-isoxazol-5-ylmethoxy]-4-methoxy-benzamide,
4-Benzyloxy-N-(3,5-dichloro-pyridin-4-yl)-3-(3,5-dimethyl-4,5-dihydro-isoxazol-5-ylmethoxy)-benzamide,
4-Cycloheptyloxy-N-(3,5-dichloro-pyridin-4-yl)-3-(3,5-dimethyl-4,5-dihydro-isoxazol-5-ylmethoxy)-benzamide,
3-[3-(3-Cyclopentyloxy-4-methoxy-phenyl)-5-methyl-4,5-dihydro-isoxazol-5-ylmethoxy]-N-(3,5-dichloro-pyridin-4-yl)-benzamide,
or a pharmaceutically acceptable salt, pharmaceutically acceptable solvate, stereoisomer, tautomer, racemate, polymorph or N-oxide thereof.

In yet another aspect, provided are pharmaceutical compositions comprising a therapeutically effective amount of one or more compounds of Formula I defined above and one or more pharmaceutically acceptable carriers, excipients or diluents

In one embodiment, the pharmaceutical compositions can further comprise one or more the therapeutic agents selected from corticosteroids, beta agonists, leukotriene antagonists, 5-lipoxygenase inhibitors, chemokine inhibitors or muscarinic receptor antagonists.

In one aspect, provided are one or more compounds of Formula I or one or more pharmaceutical compositions comprising one or more compounds of Formula I along with one or more pharmaceutically acceptable carriers, excipients or diluents, wherein Formula I is defined above for use in treating, preventing, inhibiting or suppressing an inflammatory condition or disease in a patient.

In one aspect, provided are methods one or more compounds of Formula I and one or more pharmaceutically acceptable carriers, excipients or diluents, wherein Formula I is defined above, for use in the treatment, prevention, inhibition or suppression of AIDS, asthma, arthritis, bronchitis, chronic obstructive pulmonary disease (COPD), psoriasis, allergic rhinitis, shock, atopic dermatitis, Crohn's disease, adult respiratory distress syndrome (ARDS), eosinophilic granuloma, allergic conjunctivitis, osteoarthritis, ulcerative colitis or other inflammatory diseases in a patient.

In another aspect, provided are methods for preparing a compound of Formula X, 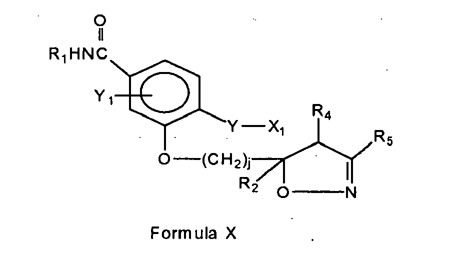 it's a pharmaceutically acceptable salt, pharmaceutically acceptable solvate, stereoisomer, tautomer, racemate, polymorph or N-oxide thereof comprising the steps of: a) oxidizing a compound of Formula II, 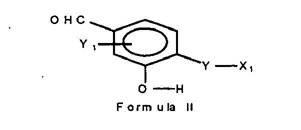 to form a compound of Formula II (a), 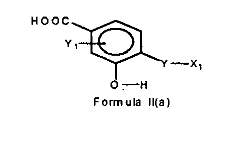 b) esterifying the compound of Formula II (a) to form a compound of Formula III, 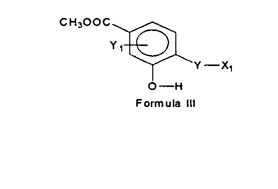 c) reacting the compound of Formula III with a compound of Formula IV (wherein X₃ is halogen 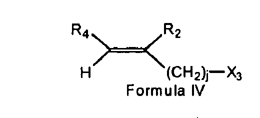 to form a compound of Formula V, 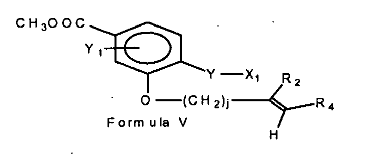 d) ester hydrolyzing the compound of Formula V to form a compound of Formula VI, 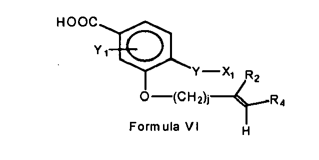 e) reacting the compound of Formula VI with a compound of Formula VII to form a compound of Formula VIII 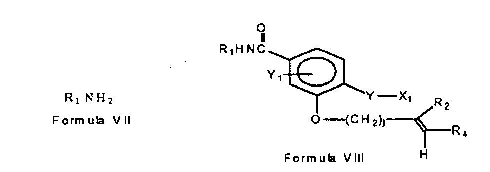 f) reacting the compound of Formula VIII with a compound of Formula IX,

R₅HC=NOH Formula IX

to form a compound of Formula X,
wherein the substituents are all as defined above.

In another aspect, provided are methods for preparing a compound of Formula XXII, 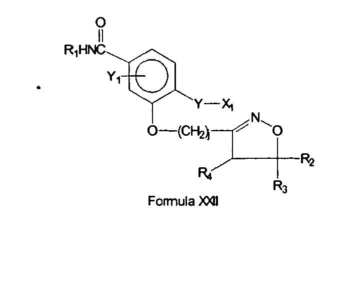 a pharmaceutically acceptable salt, pharmaceutically acceptable solvate, stereoisomer, tautomer, racemate, polymorph or N-oxide thereof comprising the steps of:
a) protecting a compound of Formula II (a), 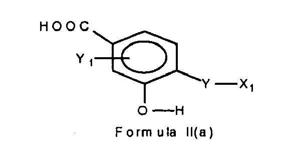 with a compound of Formula RX₃ (wherein X₃ can be halogen and R can be a hydroxy protecting group) to form a compound of Formula XI, 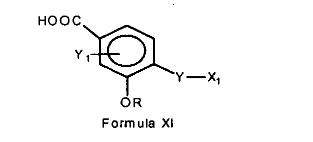 b) reacting the compound of Formula XI with a compound of Formula VII to form a compound of Formula XII, 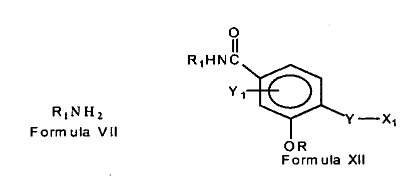 c) deprotecting the compound of Formula XII to form a compound of Formula XIII, 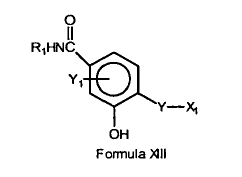 d) N-protecting the compound of Formula XIII to form a compound of Formula XIV (wherein Pr is a amino protecting group), 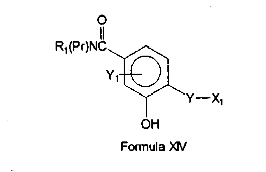 e) reacting the compound of Formula XIV with a compound of Formula XV (wherein X₃ is the same as defined earlier) to form a compound of Formula XVI, 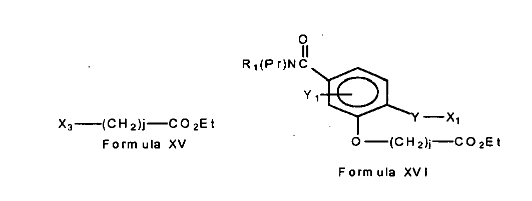 f) reducing the compound of Formula XVI to form a compound of Formula XVII, 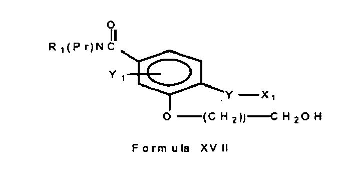 g) oxidizing the compound of Formula XVII to form a compound of Formula XVIII, 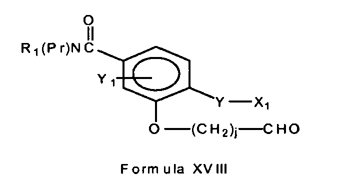 h) reacting the compound of Formula XVIII with hydroxylamine hydrochloride to form a compound of Formula XIX, 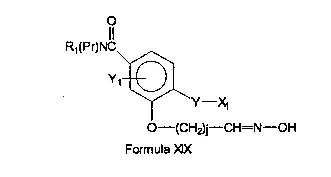 i) reacting the compound of Formula XIX with a compound of Formula XX to form a compound of Formula XXI, and 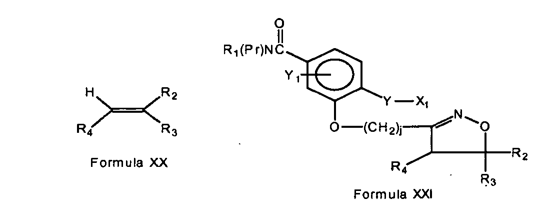 j) deprotecting the compound of Formula XXI to form a compound of Formula XXII, wherein the substituents are all as defined above.

In another aspect, provided are methods for preparing a compound of Formula XXVI, 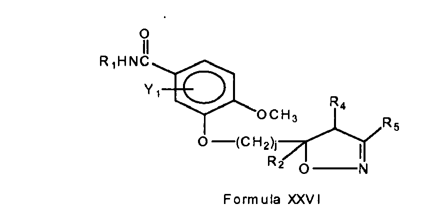 a pharmaceutically acceptable salt, pharmaceutically acceptable solvate, stereoisomer, tautomer, racemate, polymorph or N-oxide thereof comprising the steps of:
a) reacting a compound of Formula XXIII with a compound of Formula IX, 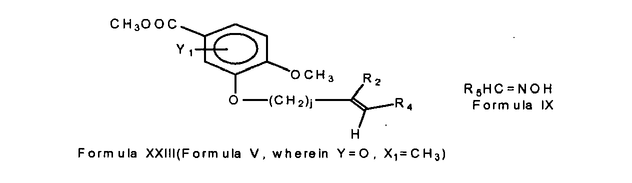 to form a compound of Formula XXIV, 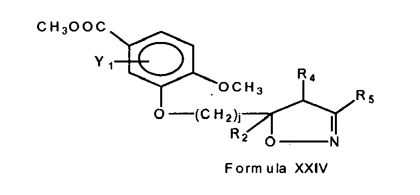
b) ester hydrolyzing the compound of Formula XXIV to form a compound of Formula XXV, and 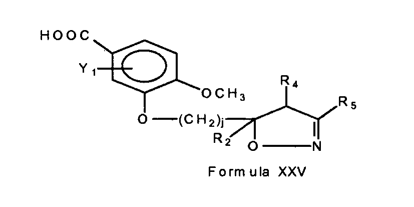
c) reacting the compound of Formula XXV with a compound of Formula VII,

   R₁NH₂ Formula VII

   to form a compound of Formula XXVI
   wherein the substituents are all as defined above.

In yet another aspect, provided are methods for preparing a compound of Formula XXVII, 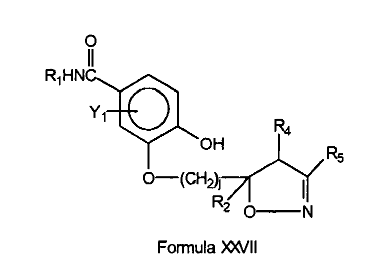 a pharmaceutically acceptable salt, pharmaceutically acceptable solvate, stereoisomer, tautomer, racemate, polymorph or N-oxide thereof comprising:
a) demethylation of a compound of Formula XXVI 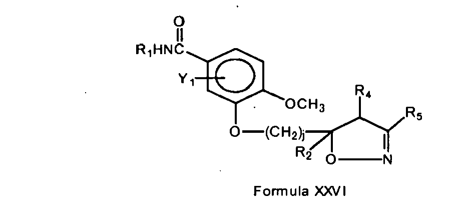 to form a compound of Formula XXVII,
   wherein the substituents are all as defined above.

In another aspect, provided are methods for preparing a compound of Formula XXIX, 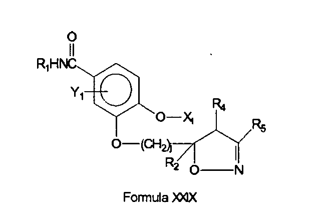 a pharmaceutically acceptable salt, pharmaceutically acceptable solvate, stereoisomer, tautomer, racemate, polymorph or N-oxide thereof comprising:
(a) reacting a compound of Formula XXVII, 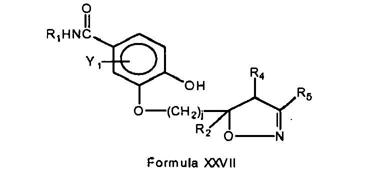 with a compound of Formula XXVIII,

   X₁-halide Formula XXVIII

   to form a compound of Formula XXIX; or
(b) 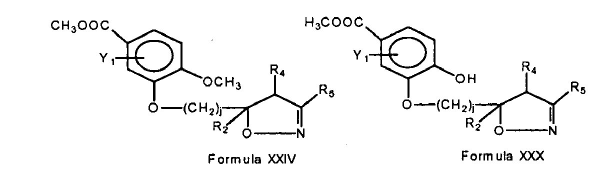
   (i) demethylating a compound of Formula XXIV to form a compound of Formula
      XXX,
   (ii) reacting the compound of Formula XXX with a compound of Formula XXVIII to form a compound of Formula XXXI, 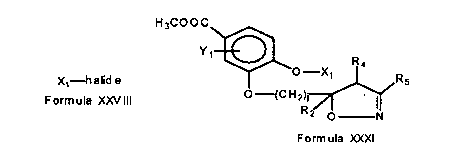
   (iii) ester hydrolyzing the compound of Formula XXXI to form a compound of Formula XXXII, and 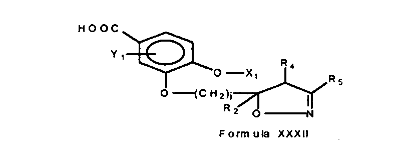
   (iv) reacting the compound of Formula XXXII with a compound of Formula VII

      R₁NH₂ Formula VII

      to form a compound of Formula XXIX,
      wherein the substituents are all as defined above.

### Detailed description of the Invention

The present invention provides phosphodiesterase inhibitors, which can be used for the treatment of AIDS, asthma, arthritis, bronchitis, chronic obstructive pulmonary disease (COPD), psoriasis, allergic rhinitis, shock, atopic dermatitis, Crohn's disease, adult respiratory distress syndrome (ARDS), eosinophilic granuloma, allergic conjunctivitis, osteoarthritis, ulcerative colitis or other inflammatory diseases. Also provided are processes for synthesizing compounds described herein.

Pharmaceutically acceptable salts, pharmaceutically acceptable solvates, stereoisomers, tautomers, racemates, polymorphs or N-oxides of compounds described herein having the same type of activity are also provided.

Pharmaceutical compositions comprising compounds described herein, which may also contain one or more pharmaceutically acceptable carriers or diluents, are also provided. Such pharmaceutical compositions can be used for treating AIDS, asthma, arthritis, bronchitis, chronic obstructive pulmonary disease (COPD), psoriasis, allergic rhinitis, shock, atopic dermatitis, Crohn's disease, adult respiratory distress syndrome (ARDS), eosinophilic granuloma, allergic conjunctivitis, osteoarthritis, ulcerative colitis or other inflammatory diseases.

Other aspects will be set forth in the accompanying description which follows and in part will be apparent from the description or may be learnt by the practice of the invention.

In accordance with one aspect, there are provided compounds having the structure of Formula **I**: 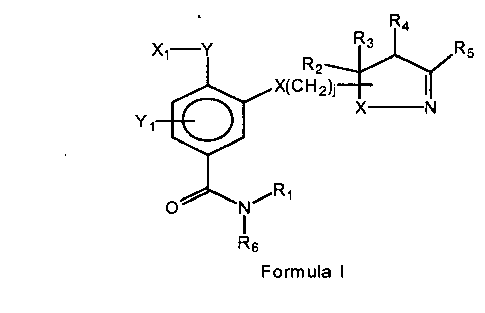 and pharmaceutically acceptable salts, pharmaceutically acceptable solvates, stereoisomers, tautomers, racemates, polymorphs or N-oxides thereof, wherein the substituents are all as defined above.

The following definitions apply to terms as used herein.

The term "alkyl" unless and otherwise specified refers to a monoradical branched or unbranched saturated hydrocarbon chain having from 1 to 20 carbon atoms. This term is exemplified by groups such as methyl, ethyl, n-propyl, iso-propyl, n-butyl, iso-butyl, t-butyl, n-hexyl, n-decyl, tetradecyl and the like. Alkyl groups may further be substituted with one or more substituents selected from the group alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, thiol, alkylthio, aryloxy, aminosulfonyl, aminocarbonylamino, hydroxylamino, alkoxyamino, nitro, -SO-alkyl, SO-aryl, -SO-heteroaryl, -SO₂-alkyl, SO₂-aryl and -SO₂-heteroaryl. Unless otherwise constrained by the definition, all 1-3 substituents chosen from alkyl, carboxy, carboxy-alkyl, aminocarbonyl, hydroxy, alkoxy, halogen, CF₃, amino, substituted amino, cyano, and -S(O)ₙR_{d}, where R_{d} is alkyl, aryl, or heteroaryl and n is 0, 1 or 2; or an alkyl group as defined above that is interrupted by 1-5 atoms of groups independently chosen from oxygen, sulfur, a phenylene, sulphinyl, sulphonyl group and NR_{c}-, where R_{c} is chosen from hydrogen, alkyl, cycloalkyl, alkenyl, cycloalkenyl, alkynyl, aryl. Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR_{d}, where n and R_{d} are the same as defined earlier; or an alkyl group as defined above that has both substituents as defined above and is also interrupted by 1-5 atoms or groups as defined above.

The term "alkenyl" unless and otherwise specified refers to a monoradical of a branched or unbranched unsaturated hydrocarbon group preferably having from 2 to 20 carbon atoms with cis or trans geometry. Preferred alkenyl groups include ethenyl or vinyl (CH=CH₂), 1-propylene or allyl (-CH₂CH=CH₂), iso-propylene (-C(CH₃)=CH₂), bicyclo[2.2.1]heptene, and the like. In the event that an alkenyl group is attached to the heteroatom, the double bond cannot be alpha to the heteroatom. Alkenyl groups may further be substituted with one or more substituents selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, thiol, alkylthio, aryl, aryloxy, aminosulfonyl, aminocarbonylamino, hydroxyamino, alkoxyamino, nitro, -SO-alkyl, -SO-aryl, -SO-heteroaryl, -SO₂-alkyl SO₂-aryl and -SO₂-heteroaryl or an alkenyl group as defined above that is interrupted by 1-5 atoms of groups independently chosen from oxygen, sulfur, a phenylene, sulphinyl, sulphonyl group and -NR_{c}-, where R_{c} is chosen from hydrogen, alkyl, cycloalkyl, alkenyl, cycloalkenyl, alkynyl, aryl. Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR_{d}, where R_{d} is alkyl, aryl, or heteroaryl and n is 0, 1 or 2.

The term "alkynyl" unless and otherwise specified refers to a monoradical of an unsaturated hydrocarbon, preferably having from 2 to 20 carbon atoms (Preferred alkynyl groups include ethynyl, (-C ≡CH), propargyl (or propynyl, -CH₂C ≡CH), and the like) and it can be interrupted by an oxygen or sulfur atom, a phenylene, imino (-NH-) or alkyliminolinkage, or a sulphinyl or sulphonyl group. In the event that alkynyl is attached to the heteroatom, the triple bond cannot be alpha to the heteroatom. It may further be substituted with one or more substituents selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, thiol, alkylthio, aryl, aryloxy, aminosulfonyl, aminocarbonylamino, hydroxyamino, alkoxyamino, nitro, -SO-alkyl, -SO-aryl, -SO-heteroaryl, -SO₂-alkyl SO₂-aryl and -SO₂-heteroaryl or an alkynyl group as defined above that is interrupted by 1-5 atoms of groups independently chosen from oxygen, sulfur, a phenylene, sulphinyl, sulphonyl group and -NR_{c}-, where R_{c} is chosen from hydrogen, alkyl, cycloalkyl, alkenyl, cycloalkenyl, alkynyl, aryl. Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR_{d}, where R_{d} is alkyl, aryl, or heteroaryl and n is 0, 1 or 2.

The term "cycloalkyl" refers to (un)saturated cyclic alkyl groups of from 3 to 20 carbon atoms having a single cyclic ring or multiple condensed rings. Such cycloalkyl groups include, by way of example, single ring structures such as cyclopropyl, cyclobutyl, cyclopentyl, cyclohexyl, cycloheptyl, cyclooctyl, cyclopropylene, cyclobutylene and the like, or multiple ring structures such as adamantanyl, and bicyclo [2.2.1]heptane, or cyclic alkyl groups to which is fused an aryl group, for example indane, and the like. Cycloalkyl groups may further be substituted with one or more substituents selected from the group consisting of alkyl, alkenyl, alkynyl, alkoxy, cycloalkyl, cycloalkenyl, acyl, acylamino, acyloxy, amino, aminocarbonyl, alkoxycarbonylamino, azido, cyano, halogen, hydroxy, keto, thiocarbonyl, carboxy, carboxyalkyl, arylthio, thiol, alkylthio, aryl, aryloxy, aminosulfonyl, aminocarbonylamino, hydroxyamino, alkoxyamino, nitro, -SO-alkyl, -SO-aryl, -SO-heteroaryl, -SO₂-alkyl -SO₂-aryl and -SO₂-heteroaryl. Unless otherwise constrained by the definition, all substituents may optionally be further substituted by 1-3 substituents chosen from alkyl, carboxy, carboxyalkyl, aminocarbonyl, hydroxy, alkoxy, halogen, CF₃, amino, substituted amino, cyano, and -S(O)ₙR_{d}, where R_{d} is alkyl, aryl or heteroaryl and n is 0, 1 or 2.

The term "alkoxy" denotes the group O-alkyl, wherein alkyl is the same as defined above.

The term "aryloxy" refers to the group O-aryl, wherein aryl is the same as defined below.

The term "cycloalkoxy" refers to the group O-cycloalkyl, wherein cycloalkyl is the same as defined above.

The term "halogen" refers to fluorine, chlorine, bromine or iodine.

The term "aralkyl" refers to (CH₂)ₚ aryl, wherein p is an integer from 1-6 and aryl is as defined below.

The term "aryl" herein refers to phenyl or naphthyl ring and the like optionally substituted with 1 to 3 substituents selected from the group consisting of halogen, hydroxy, alkyl, alkenyl, alkynyl, cycloalkyl, cycloalkoxy, alkoxy, aryloxy, cyano, nitro, carboxy, heterocyclyl, heteroaryl, heterocyclylalkyl, heteroarylalkyl, C(=O)R", wherein R" is selected from hydrogen, alkyl, cycloalkyl, aryl, aralkyl, hydroxy, alkoxy, heteroaryl, heterocyclyl; (CH₂)₀₋₃C(=O)NRₓR_{y}, wherein Rₓ and R_{y} are same as defined earlier.

The term "carboxy" as defined herein refers to -C(=O)O-R_{f}, wherein R_{f} is selected from hydrogen, alkyl, alkenyl, alkynyl or cycloalkyl.

The term "heteroaryl" unless and otherwise specified refers to an aromatic ring structure containing 5 or 6 carbon atoms, or a bicyclic aromatic group having 8 to 10 carbon atoms, with one or more heteroatom(s) independently selected from the group consisting ofN, O and S optionally substituted with 1 to 3 substituent(s) selected from the group consisting of halogen, hydroxy, alkenyl, alkynyl, cycloalkyl, aryl, heteroaryl, heterocyclyl, alkoxy, aralkyl, cyano, nitro, optionally substituted amino, wherein the substituents are selected from the group consisting of alkyl, alkenyl, alkynyl, cycloalkyl, aryl; carboxy, -C(=O)R", wherein R" is the same as defined earlier and C(=O)NRₓR_{y}, wherein Rₓ and R_{y} are the same as defined earlier. Examples of heteroaryl groups include pyridinyl, pyridazinyl, pyrimidinyl, pyrrolyl, oxazolyl, thiazolyl, thienyl, isoxazolyl, triazinyl, furanyl, benzofuranyl, indolyl, benzothiazolyl, benzoxazolyl, and the like.

The term 'heterocyclyl" unless and otherwise specified refers to a non aromatic cycloalkyl group having 5 to 14 atoms in which one or more carbon atom(s) in a ring are replaced by heteroatoms selected from O, S or N, in which the point of attachment can be carbon or nitrogen and are optionally benzofused or fused heteroaryl of 5-6 ring members and/or are optionally substituted, wherein the substituents are selected from the group consisting of halogen, hydroxy, alkyl, alkenyl, alkynyl, cycloalkyl, aryl, alkoxy, alkaryl, heteroaryl, cyano, nitro, optionally substituted amino, wherein the substituents are selected from the group consisting of alkyl, alkenyl, alkynyl, cycloalkyl, aryl; carboxy, C(=O)R", wherein R" is the same as defined earlier; C(=O)NRₓR_{y}, wherein Rₓ and R_{y} the same as defined earlier. One or more carbon(s) of heterocyclyl can also be replaced by carbonyl or thionyl group. Examples of heterocyclyl groups include tetrahydrofuranyl, dihydrofuranyl, dihydropyridinyl, piperidinyl, piperazinyl, dihydrobenzofuryl, dihydroindolyl, and the like.

"(Heteroaryl)alkyl" refers to alkyl-heteroaryl group, wherein the alkyl and heteroaryl are the same as defined earlier.

"(Heterocyclyl)alkyl" refers to alkyl-heterocyclyl group, wherein the alkyl and heterocyclyl are the same as defined earlier.

The term "acyl" as defined herein refers to -C(=O)R", wherein R" is the same as defined earlier.

The term "hydroxy protecting group" includes, but are not limited to, acyl, aroyl, alkyl, aryl, butyldiphenylsilyl, tert-butyldimethylsilyl, methoxymethyl, methylthiomethyl, and the like.

The term "amino protecting group" includes, but is not limited to, acyl, tert-butoxycarbonyl, methoxycarbonyl, ethoxycarbonyl, benzyloxycarbonyl, aroyl, and the like.

The compounds described herein can be used for treating AIDS, asthma, arthritis, bronchitis, chronic obstructive pulmonary disease, psoriasis, allergic rhinitis, shock, atopic dermatitis, Crohn's disease, adult respiratory distress syndrome (ARDS), eosinophilic granuloma, allergic conjunctivitis, osteoarthritis, ulcerative colitis or other inflammatory diseases.

In accordance with yet another aspect, there are provided processes for the preparation of the compounds as described herein.

The compounds described herein may be prepared by techniques well known in the art and familiar to the average synthetic organic chemist. In addition, the compounds of present invention may be prepared by the following reaction sequences as depicted in Schemes I, II, III and IV.

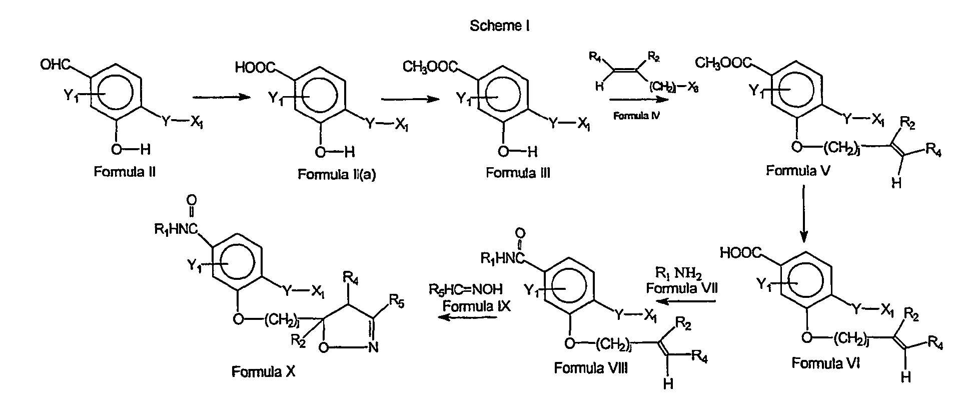

The compound of Formula X can be prepared by following Scheme I. Thus, oxidizing a compound of Formula II forms a compound of Formula II (a) (wherein X₁, Y₁ and Y are the same as defined earlier). The compound of Formula II (a) can be esterified to form a compound of Formula III. The compound of Formula can be reacted with a compound of Formula IV (wherein X₃ can be halogen and j is the same as defined earlier) to form a compound of Formula V (wherein R₂, R₄ and j are the same as defined earlier). The compound of Formula V can undergo ester hydrolysis to form a compound of Formula VI. The compound of Formula VI can be reacted with a compound of Formula VII to form a compound of Formula VIII (wherein R₁ is the same as defined earlier). The compound of Formula VIII can be reacted with a compound of Formula IX to form a compound of Formula X (wherein R₅ is the same as defined earlier).

The oxidation of a compound of Formula II to form a compound of Formula II (a) can be carried out in one or more solvents, for example, polar solvents. Suitable solvents include C₁₋₄ branched or unbranched alcohols (e.g., t-butanol), water, acetonitrile or mixtures thereof, in the presence of one or more organic acids, for example, trifluoroacetic acid, acetic acid, formic acid or mixtures thereof.

The oxidation of a compound of Formula II to form a compound of Formula II (a) can be carried out in the presence of one or more oxidation agents, for example, sodium hypochlorite, sodium chlorite, potassium permanganate, chromium trioxide, potassium dichromate, or mixtures thereof, and a co-reagent known in the prior art to prevent liberation of chlorine dioxide or hypochlorous acid, for example, sulphamic acid, resorcinol, or mixtures thereof.

The esterification of a compound of Formula II (a) to form a compound of Formula III can be carried out in the presence of an alcohol, for example, methanol, and one or more mineral acids, for example, sulfuric acid, hydrochloric acid or mixtures thereof.

The reaction of a compound of Formula III with a compound of Formula IV to form a compound of Formula V can be carried out in one or more organic solvents, for example, dimethylformamide, dimethylsulfoxide, tetrahydrofuran or mixtures thereof.

The reaction of a compound of Formula III to form a compound of Formula V can be carried out in the presence of one or more inorganic bases, for example, Group IA and IIA carbonates, e.g., potassium carbonate, sodium carbonate, potassium bicarbonate, sodium bicarbonate or mixtures thereof.

The ester hydrolysis of a compound of Formula V to form a compound of Formula VI can be carried out in one or more alcohols, for example, C₁₋₄ alcohols, e.g., methanol, ethanol or mixtures thereof; or an alcohol and water mixture.

The ester hydrolysis of a compound of Formula V to form a compound of Formula VI can be carried out in the presence of one or more inorganic bases, for example, Group IA and IIA hydroxides, e.g., potassium hydroxide, sodium hydroxide, lithium hydroxide or mixtures thereof; Group IA and IIA carbonates, e.g., cesium carbonate; Group IA or IIA alkoxides, e.g., potassium t-butoxide; or mixtures thereof.

The reaction of a compound of Formula VI with a compound of Formula VII to form a compound of Formula VIII can be carried out in the presence of one or more activating agents known in the prior art to transform a carboxylic acid to an amide, for example, oxalyl chloride, thionyl chloride, phosphoroxy chloride, mixed anhydride, alkyl chloroformate, allyl chloroformate or carbodiimides. The reaction can be carried out in one or more organic solvents, for example, dimethylformamide, dimethylsulfoxide, halogenated solvents, tetrahydrofuran or mixtures thereof.

The reaction of a compound of Formula VI with a compound of Formula VII can be carried out in the presence of one or more inorganic or organic bases, for example, Group IA or IIA hydrides, Group IA or IIA carbonates, pyridine, or amines, e.g., sodium hydride, lithium hydride, potassium hydride, 4-dimethylaminopyridine (DMAP), triethyl amine, trimethyl amine or mixtures thereof.

The reaction of a compound of Formula VIII with a compound of Formula IX to form a compound of Formula X can be carried out in one or more organic solvents, for example, tetrahydrofuran, halogenated solvents, dimethylformamide, dimethylsulfoxide or mixtures thereof.

The reaction of a compound of Formula VIII with a compound of Formula IX to form a compound of Formula X can be carried out in the presence of one or more organic bases, for example, triethyl amine, pyridine, 2,6-lutidine or mixtures thereof thereof.

The reaction of a compound of Formula VIII with a compound of Formula IX can be carried out in the presence of one or more halogenating reagents, for example, sodium hypochlorite, N-chlorosuccinimide, N-bromochlorosuccinimide or mixtures thereof.

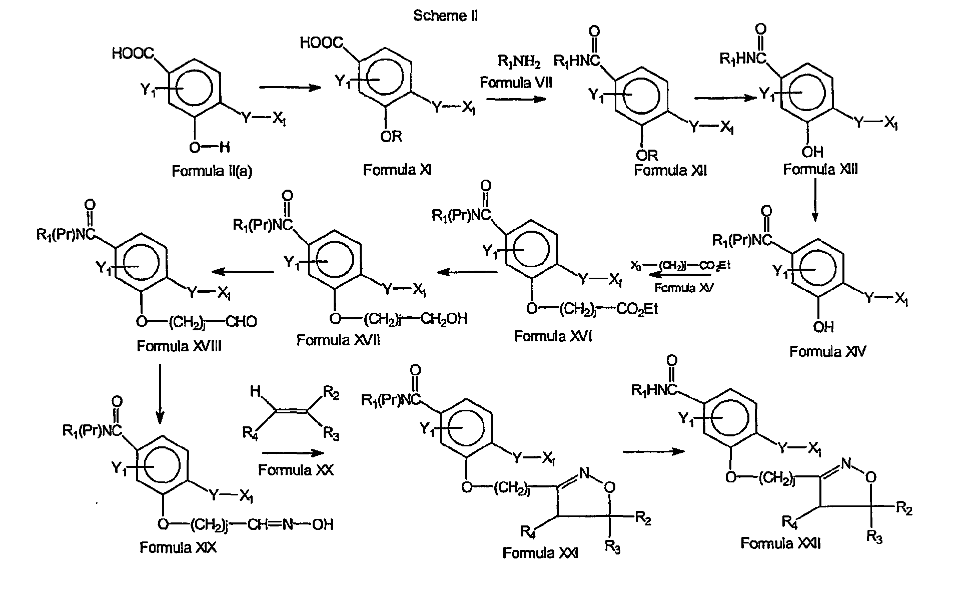

The compound of Formula XXII can be prepared by following Scheme II. Thus, a compound of Formula II(a) can be protected with a compound of Formula RX₃ (wherein X₃ can be halogen) to form a compound of Formula XI (wherein Y, Y₁ and X₁ are the same as defined earlier and R is a hydroxy protecting group). The compound of Formula XI can be reacted with a compound of Formula VII to form a compound of Formula XII (wherein R₁ is the same as defined earlier). The compound of Formula XII can be deprotected to form a compound of Formula XIII. The compound of Formula XIII can be N-protected to form a compound of Formula XIV (wherein Pr is a amino protecting group). The compound of Formula XIV can be reacted with a compound of Formula XV (wherein X₃ is the same as defined earlier) to form a compound of Formula XVI, (wherein j is the same as defined earlier). The compound of Formula XVI can be reduced to form a compound of Formula XVII. The compound of Formula XVII can be oxidized to form a compound of Formula XVIII. The compound of Formula XVIII can be reacted with hydroxylamine hydrochloride to form a compound of Formula XIX. The compound of Formula XIX can be reacted with a compound of Formula XX to form a compound of Formula XXI (wherein R₂,R₃ and R₄ are the same as defined earlier). The compound of Formula XXI can be deprotected to form a compound of Formula XXII.

The protection of a compound of Formula II (a) with a compound of Formula RX₃, wherein R is a hydroxy protecting group known in the prior art, for example, tert-butyl dimethyl silyl, triisopropylsilyl, trimethyl silyl, triethyl silyl, tert-butyldiphenylsilyl, benzyl or O-boc to form a compound of Formula XI can be carried out in one or more solvents, for example, dimethylsulfoxide, dimethylformamide, halogenated solvents, chloroform , tetrahydrofuran or mixtures thereof.

The protection of a compound of Formula II(a) to form a compound of Formula XI can be carried out in the presence of one or more organic bases, for example, imidazole, triethylamine, 4-dimethylaminopyridine, N-ethyldiisopropylamine or mixtures thereof.

The reaction of a compound of Formula XI with a compound of Formula VII to form a compound of Formula XII can be carried out in the presence of one or more activating agents known in the prior art to convert a carboxylic acid to an amide, for example, oxalyl chloride, thionyl chloride, phosphoroxy chloride, mixed anhydride, alkyl chloroformate, allyl chloroformate or carbodiimides. The reaction can be carried out in one or more organic solvents, for example, halogenated solvents, dimethylsulfoxide, dimethylformamide, tetrahydrofuran or mixtures thereof.

The reaction of a compound of Formula XI with a compound of Formula VII can be carried out in the presence of one or more inorganic or organic bases, for example, Group IA or IIA hydrides, Group IA or IIA carbonates, pyridine, or amines, e.g., sodium hydride, lithium hydride, potassium hydride, 4-dimethylaminopyridine (DMAP), triethyl amine, trimethyl amine or mixtures thereof.

The deprotection of a compound of Formula XII to form a compound of Formula XIII can be carried out in one or more organic solvents, for example, tetrahydrofuran, dimethylformamide, dimethylsulfoxide, halogenated solvents or mixtures thereof.

The deprotection of a compound of Formula XII to form a compound of Formula XIII can be carried out in the presence of one or more deprotecting agents known in the prior art, for example, tetrabutylammonium fluoride, hydrogen fluoride-pyridine, potassium fluoride, borontrifluoride-diethylether or mixtures thereof.

The N-protection of a compound of Formula XIII to form a compound of Formula XIV can be carried out in the presence of one or more N-protecting reagents known in prior art, for example, di-tert.-butyl dicarbonate, benzyl, 9-fluoromethoxycarbonyl, allyl or mixtures thereof, in one or more organic solvents, for example, halogenated solvents, tetrahydrofuran, dimethylformamide, dimethylsulfoxide or mixtures thereof.

The N-protection of a compound of Formula XIII to form a compound of Formula XIV can be carried out in the presence of one or more organic bases, for example, triethylamine, trimethylamine or diisopropylamine.

The reaction of a compound of Formula XIV with a compound of Formula XV to form a compound of Formula XVI can be carried out in one or more organic solvents, for example, dimethylsulfoxide, tetrahydrofuran, dimethylformamide, halogenated solvents or mixtures thereof.

The reaction of a compound of Formula XIV with a compound of Formula XV can be carried out in the presence of one or more bases, for example, Group IA or IIA carbonates or bicarbonates, e.g., potassium carbonate, sodium carbonate, sodium bicarbonate or mixtures thereof, Group IA or IIA hydrides, or mixtures thereof.

The reduction of a compound of Formula XVI to form a compound of Formula XVII can be carried out in one or more organic solvents, for example, alcoholic solvents, tetrahydrofuran, dimethylformamide or mixtures thereof.

The reduction of a compound of Formula XVI to form a compound of Formula XVII can be carried out in the presence of one or more reducing agents, for example, sodium borohydride, lithium borohydride or lithium aluminium hydride.

The oxidation of a compound of Formula XVII to form a compound of Formula XVIII can be carried out in the presence of one or more organic bases, for example, pyridine, DMAP, 2,6-lutidine, quinoline or mixtures thereof.

The oxidation of a compound of Formula XVII to form a compound of Formula XVIII can be carried out in the presence of an oxidizing agent, for example, chromium trioxide, pyridinium chlorochromate, quinolinium chlorochromate desmartin reagent, manganese dioxide, oxalyl chloride-dimethylsulfoxide (Swem reagent) or mixtures thereof.

The oxidation of a compound of Formula XVII to form a compound of Formula XVIII can be carried out in one or more organic solvents, for example, dimethylformamide, tetrahydrofuran, dimethylsulfoxide, acetone, halogenated solvents or mixtures thereof.

The reaction of a compound of Formula XVIII to form a compound of Formula XIX can be carried out in the presence of one or more neutralizing reagents, for example, sodium acetate or molecular sieves. The reaction can also be carried out in one or more organic solvents, for example, alcoholic solvents, toluene, pyridine, 2,6-lutidine or mixtures thereof.

The reaction of a compound of Formula XIX with a compound of Formula XX to form a compound of Formula XXI can be carried out in one or more organic solvents, for example, tetrahydrofuran, halogenated solvents, dimethylsulfoxide, dimethylformamide or mixtures thereof.

The reaction of a compound of Formula XIX with a compound of Formula XX can be carried out in the presence of one or more halogenating reagents, for example, sodium hypochlorite or N-bromosuccinimide, N-chlorosuccinimide or mixtures thereof.

The reaction of a compound of Formula XXI to form a compound of Formula XXII can be carried out in one or more organic solvents, for example, tetrahydrofuran, halogenated solvents, dimethylsulfoxide, dimethylformamide or mixtures thereof.

The deprotection of a compound of Formula XXI to form a compound of Formula XXII can be carried out in the presence of organic, inorganic or Lewis acids, for example, trifluoroacetic acid, formic acid, p-toluene sulfonic acid, hydrobromic acid, hydrochloric acid, sulfuric acid, boron trifluoride-diethyl ether, or mixtures thereof.

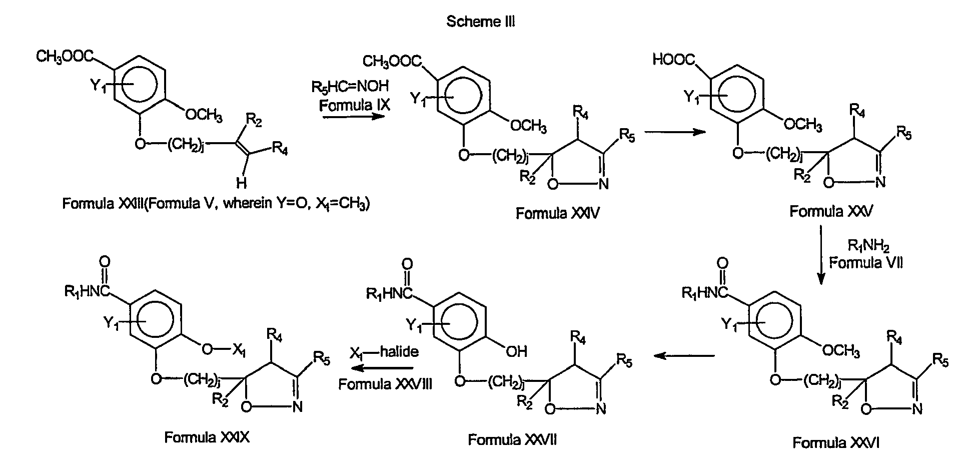

The compound of Formula XXIX can be prepared by following Scheme III. Thus, a compound of Formula XXIII can be reacted with a compound of Formula IX to form a compound of Formula XXIV (wherein R₂, R₄, R₅, j and Y₁ are the same as defined earlier). The compound of Formula XXIV can undergo ester hydrolysis to form a compound of Formula XXV. The compound of Formula XXV can be reacted with a compound of Formula VII to form a compound of Formula XXVI (wherein R₁ is the same as defined earlier). The compound of Formula XXVI can be demethylated to form a compound of Formula XXVII. The compound of Formula XXVII can be reacted with a compound of Formula XXVIII to form a compound of Formula XXIX (wherein X₁ is the same as defined earlier).

The reaction of a compound of Formula XXIII with a compound of Formula IX to form a compound of Formula XXIV can be carried out in one or more organic solvents, for example, tetrahydrofuran, halogenated solvents, dimethylformamide, dimethylsulfoxide or mixtures thereof.

The reaction of a compound of Formula XXIII with a compound of Formula IX to form a compound of Formula XXIV can be carried out in the presence of one or more organic bases, for example, triethyl amine, diisopropylethylamine, pyridine, 2,6-lutidine or mixtures thereof thereof.

The reaction of a compound of Formula XXIII with a compound of Formula IX can be carried out in the presence of one or more halogenating reagents, for example, sodium hypochlorite, N-chlorosuccinimide, N-bromochlorosuccinimide or mixtures thereof.

The ester hydrolysis of a compound of Formula XXIV to form a compound of Formula XXV can be carried out in one or more alcohols, for example, methanol, ethanol, or an alcohol and water mixture.

The ester hydrolysis of a compound of Formula XXIV to form a compound of Formula XXV can be carried out in the presence of one or more inorganic bases, for example, Group IA and IIA hydroxides, e.g., potassium hydroxide, sodium hydroxide, lithium hydroxide or mixtures thereof; Group IA and IIA carbonates, e.g., cesium carbonate; Group IA or IIA alkoxides, e.g., potassium t-butoxide; or mixtures thereof.

The reaction of a compound of Formula XXV with a compound of Formula VII to form a compound of Formula XXVI can be carried out in the presence of one or more activating agents known in the prior art to convert a carboxylic acid to an amide, for example, oxalyl chloride, thionyl chloride, phosphoroxy chloride, mixed anhydride, alkyl chloroformate, allyl chloroformate or carbodiimides in one or more organic solvents, for example, halogenated solvents, dimethylsulfoxide, dimethylformamide, tetrahydrofuran or mixtures thereof.

The reaction of a compound of Formula XXV with a compound of Formula VII can be carried out in the presence of one or more inorganic or organic bases, for example, Group IA or IIA hydrides, Group IA or IIA carbonates, pyridine, or amines, e.g., sodium hydride, lithium hydride, potassium hydride, 4-dimethylaminopyridine (DMAP), triethyl amine, trimethyl amine or mixtures thereof.

The demethylation of a compound of Formula XXVI to form a compound of Formula XXVII can be carried out in one or more solvents, for example, tetrahydrofuran, halogenated solvents, dimethylformamide, dimethylsulfoxide or mixtures thereof.

The demethylation of a compound of Formula XXVI to form a compound of Formula XXVII can be carried out in the presence of one or more cleavage reagents, for example, aluminium chloride-bromonitrobenzene, boron trihalide-dimethyl sulphide, boron triiodide-N,N-diethylaniline, lewis acid-sodium iodide or sodium salt of N-methylaniline.

The reaction of a compound of Formula XXVII with a compound of Formula XXVIII to form a compound of Formula XXIX can be carried out in one or more solvents, for example, acetonitrile, acetone, ethanol, tetrahydrofuran, dimethylformamide, dimethylsulfoxide, toluene or mixtures thereof.

The reaction of a compound of Formula XXVII with a compound of Formula XXVIII can be carried out in the presence of one or more inorganic bases, for example, Group IA or IIA carbonates or bicarbonates, e.g., potassium carbonate, sodium carbonate, sodium bicarbonate or mixtures thereof; Group IA or IIA hydrides, e.g., lithium hydride, sodium hydride, potassium hydride or mixtures thereof; or mixtures thereof.

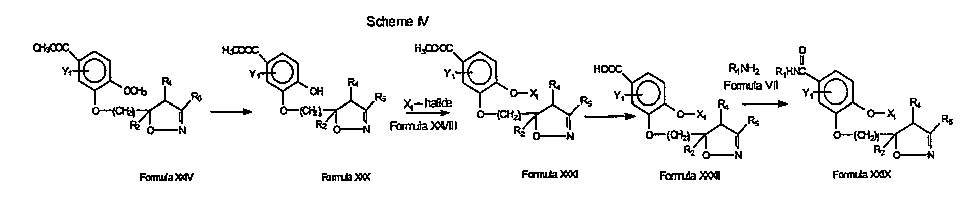

The compound of Formula XXIX can also be prepared by following Scheme IV. Thus, a compound of Formula XXIV can be demethylated to form a compound of Formula XXX (wherein R₂, R₄, R₅, j and Y₁ are the same as defined earlier). The compound of Formula XXX can be reacted with a compound of Formula XXVIII to form a compound o Formula XXXI (wherein X₁ is the same as defined earlier). The compound of Formula XXXI can undergo ester hydrolysis to form a compound of Formula XXXII. The compound of Formula XXXII can be reacted with a compound of Formula VII to form a compound of Formula XXIX (wherein R₁ is the same as defined earlier).

The demethylation of a compound of Formula XXIV to form a compound of Formula XXX can be carried out in one or more solvents, for example, tetrahydrofuran, halogenated solvents, dimethylformamide, dimethylsulfoxide or mixtures thereof.

The demethylation of a compound of Formula XXIV to form a compound of Formula XXX can be carried out in the presence of one or more cleavage reagent, for example, aluminium chloride-bromonitrobenzene, boron trihalide-dimethyl sulphide, boron triiodide-N,N-diethylaniline, lewis acid-sodium iodide or sodium salt of N-methylaniline.

The reaction of a compound of Formula XXX with a compound of Formula XXVIII to form a compound of Formula XXXI can be carried out in one or more solvents, for example, acetonitrile, acetone, ethanol, tetrahydrofuran, dimethylformamide, dimethylsulfoxide, toluene or mixtures thereof.

The reaction of a compound of Formula XXX with a compound of Formula XXVIII can be carried out in the presence of one or more inorganic bases, for example, Group IA or IIA carbonates or bicarbonates, e.g., potassium carbonate, sodium carbonate, sodium bicarbonate or mixtures thereof; Group IA or IIA hydrides, e.g., lithium hydride, sodium hydride, potassium hydride or mixtures thereof; or mixtures thereof.

The ester hydrolysis of a compound of Formula XXXI to form a compound of Formula XXXII can be carried out in one or more alcohols, for example, methanol, ethanol or an alcohol and water mixture.

The ester hydrolysis of a compound of Formula XXXI to form a compound of Formula XXXII can be carried out in the presence of one or more inorganic bases, for example, Group IA and IIA hydroxides, e.g., potassium hydroxide, sodium hydroxide, lithium hydroxide or mixtures thereof; Group IA and IIA carbonates, e.g., cesium carbonate; Group IA or IIA alkoxides, e.g., potassium t-butoxide; or mixtures thereof.

The reaction of a compound of Formula XXXII with a compound of Formula VII to form a compound of Formula XXIX can be carried out in the presence of one or more activating agents known in the prior art to convert a carboxylic acid to an amide, for example, oxalyl chloride, thionyl chloride, phosphoroxy chloride, mixed anhydride, alkyl chloroformate, allyl chloroformate or carbodiimides in one or more organic solvents, for example, dimethylformamide, dimethylsulfoxide, halogenated solvents, tetrahydrofuran or mixtures thereof.

The reaction of a compound of Formula XXXII with a compound of Formula VII can be carried out in the presence of one or more inorganic or organic bases, for example, Group IA or IIA hydrides, Group IA or IIA carbonates, pyridine, or amines, e.g., sodium hydride, lithium hydride, potassium hydride, 4-dimethylaminopyridine (DMAP), triethyl amine, trimethyl amine or mixtures thereof.

In the above schemes, where the specific solvents, bases, reducing agents, oxidizing agents etc., are disclosed, it is to be understood that other solvents, bases, reducing agents, oxidizing agents etc., known to those skilled in the art may be used. Similarly, the reaction temperature and duration may be adjusted according to the desired needs.

Examples of compounds described herein include, but are not limited to:
3-[3-(3-Cyclopentyloxy-4-methoxy-phenyl)-5-methyl-4,5-dihydro-isoxazol-5-ylmethoxy]-N-(3,5-dichloro-pyridin-4-yl)-4-methoxy-benzamide (Compound No. 1),
N-(3,5-dichloro-pyridin-4-yl)-3-(3,5-dimethyl-4,5-dihydro-isoxazol-5-ylmethoxy)-4-methoxy-benzamide (Compound No. 2),
3-(5-Cyano-5-methyl-4,5-dihydro-isoxazol-4-ylmethoxy)-N-(3,5-dichloro-pyridin-4-yl)-4-methoxy-benzamide (Compound No. 3),
4-[5-(3,5-Dichloro-pyridin-4-ylcarbomoyl)-2-methoxy-phenoxymethyl]-5-methyl-4,5-dihydro-isoxazole-5-carboxylic acid methyl ester (Compound No. 4),
4-[5-(3,5-Dichloro-pyridin-4-ylcarbomoyl)-2-methoxy-phenoxymethyl]-5-methyl-4,5-dihydro-isoxazole-5-carboxylic acid ethyl amide (Compound No. 5),
3-[5-(3,5-Dichloro-pyridin-4-ylcarbomoyl)-2-methoxy-phenoxymethyl]-5-methyl-4,5-dihydro-isoxazole-5-carboxylic acid (Compound No. 6),
3-[5-(3,5-Dichloro-pyridin-4-ylcarbomoyl)-2-methoxy-phenoxymethyl]-5-methyl-4,5-dihydro-isoxazole-5-carboxylic acid amide (Compound No. 7),
3-[5-(3,5-Dichloro-pyridin-4-ylcarbomoyl)-2-methoxy-phenoxymethyl]-5-methyl-4,5-dihydro-isoxazole-5-carboxylic acid methyl amide (Compound No. 8),
3-[5-(3,5-Dichloro-pyridin-4-ylcarbomoyl)-2-methoxy-phenoxymethyl]-5-methyl-4,5-dihydro-isoxazole-5-carboxylic acid dimethyl amide (Compound No. 9),
N-(3,5-dichloro-pyridin-4-yl)-3-(5-methyl-3-phenyl-4,5-dihydro-isoxazol-5-ylmethoxy)-4-methoxy-benzamide (Compound No. 10),
N-(3,5-dichloro-pyridin-4-yl)-3-(5-methyl-3-pyridin-3-yl-4,5-dihydro-isoxazol-5-ylmethoxy)-4-methoxy-benzamide (Compound No. 11),
N-(3,5-dichloro-pyridin-4-yl)-3-(3-(4-difluoromethoxy-phenyl)-5-methyl-4,5-dihydro-isoxazol-5-ylmethoxy)-4-methoxy-benzamide (Compound No. 12),
N-(3,5-dichloro-pyridin-4-yl)-4-difluoromethoxy-3-(3,5-dimethyl-4,5-dihydro-isoxazol-5-ylmethoxy)-benzamide (Compound No. 13),
N-(3,5-dichloro-pyridin-4-yl)-3-(5-methyl-3-phenyl-4,5-dihydro-isoxazol-5-ylmethoxy)-4-hydroxy-benzamide (Compound No. 14),
N-(3,5-dichloro-pyridin-4-yl)-3-[3-(2-fluoro-phenyl)-5-methyl-4,5-dihydro-isoxazol-5-ylmethoxy)-4-methoxy-benzamide (Compound No. 15),
N-(3,5-dichloro-pyridin-4-yl)-3-[3-(2,6-difluoro-phenyl)-5-methyl-4,5-dihydro-isoxazol-5-ylmethoxy)-4-methoxy-benzamide (Compound No. 16),
N-(3,5-dichloro-pyridin-4-yl)-3-[3,5-dimethyl-4,5-dihydro-isoxazol-5-ylmethoxy)-4-hydroxy-benzamide (Compound No. 17),
N-(3,5-dichloro-pyridin-4-yl)-3-[3-(4-fluoro-phenyl)-5-methyl-4,5-dihydro-isoxazol-5-ylmethoxy)-4-methoxy-benzamide (Compound No. 18),
N-(3,5-dichloro-pyridin-4-yl)-3-[3-(3-fluoro-phenyl)-5-methyl-4,5-dihydro-isoxazol-5-ylmethoxy)-4-hydroxy-benzamide (Compound No. 19),
N-(3,5-dichloro-pyridin-4-yl)-3-[3-(3-fluoro-phenyl)-5-methyl-4,5-dihydro-isoxazol-5-ylmethoxy)-4-methoxy-benzamide (Compound No. 20),
N-(3,5-dichloro-pyridin-4-yl)-4-ethoxy-3-(5-methyl-3-phenyl-4,5-dihydro-isoxazol-5-ylmethoxy)-benzamide (Compound No.21),
N-(3,5-dichloro-pyridin-4-yl)-4-ethoxy-3-[3-(3-fluoro-phenyl)-5-methyl-4,5-dihydro-isoxazol-5-ylmethoxy]-benzamide (Compound No. 22),
N-(3,5-dichloro-pyridin-4-yl)-3-(5-methyl-3-phenyl-4,5-dihydro-isoxazol-5-ylmethoxy)-4-propoxy-benzamide (Compound No. 23),
N-(3,5-dichloro-pyridin-4-yl)-3-[3-(3-fluoro-phenyl)-5-methyl-4,5-dihydro-isoxazol-5-ylmethoxy]-4-propoxy-benzamide (Compound No. 24),
N-(3,5-dichloro-pyridin-4-yl)-4-isopropoxy-3-(5-methyl-3-phenyl-4,5-dihydro-isoxazol-5-ylmethoxy)-benzamide (Compound No. 25),
N-(3,5-dichloro-pyridin-4-yl)-3-[3-(3-fluoro-phenyl)-5-methyl-4,5-dihydro-isoxazol-5-ylmethoxy]-4-isopropoxy-benzamide (Compound No. 26),
4-Cyclopropoxy-N-(3,5-dichloro-pyridin-4-yl)-3-(5-methyl-3-phenyl-4,5-dihydro-isoxazol-5-ylmethoxy)-benzamide (Compound No. 27),
4-Cyclopropoxy-N-(3,5-dichloro-pyridin-4-yl)-3-[3-(3-fluoro-phenyl)-5-methyl-4,5-dihydro-isoxazol-5-ylmethoxy]-benzamide (Compound No. 28),
4-Butoxy-N-(3,5-dichloro-pyridin-4-yl)-3-(5-methyl-3-phenyl-4,5-dihydro-isoxazol-5-ylmethoxy)-benzamide (Compound No. 29),
4-Butoxy-N-(3,5-dichloro-pyridin-4-yl)-3-[3-(3-fluoro-phenyl)-5-methyl-4,5-dihydro-isoxazol-5-ylmethoxy]-benzamide (Compound No. 30),
4-Cyclopentyloxy-N-(3,5-dichloro-pyridin-4-yl)-3-(5-methyl-3-phenyl-4,5-dihydro-isoxazol-5-ylmethoxy)-benzamide (Compound No. 31),
4-Cyclopentyloxy-N-(3,5-dichloro-pyridin-4-yl)-3-[3-(3-fluoro-phenyl)-5-methyl-4,5-dihydro-isoxazol-5-ylmethoxy]-benzamide (Compound No. 32),
N-(3,5-dichloro-pyridin-4-yl)-3-(3,5-dimethyl-4,5-dihydro-isoxazol-5-ylmethoxy)-4-ethoxy-benzamide (Compound No. 33),
N-(3,5-dichloro-pyridin-4-yl)-3-(3,5-dimethyl-4,5-dihydro-isoxazol-5-ylmethoxy)-4-propoxy-benzamide (Compound No. 34),
N-(3,5-dichloro-pyridin-4-yl)-4-isopropoxy-3-(5-methyl-3-pyridin-3-yl-4,5-dihydro-isoxazol-5-ylmethoxy)-benzamide (Compound No. 35),
N-(3,5-dichloro-pyridin-4-yl)-3-(3,5-dimethyl-4,5-dihydro-isoxazol-5-ylmethoxy)-4-isopropoxy-benzamide (Compound No. 36),
4-Cyclopropoxy-N-(3,5-dichloro-pyridin-4-yl)-3-(5-methyl-3-pyridin-3-yl-4,5-dihydro-isoxazol-5-ylmethoxy)-benzamide (Compound No. 37),
4-Cyclopropoxy-N-(3,5-dichloro-pyridin-4-yl)-3-(3,5-dimethyl-4,5-dihydro-isoxazol-5-ylmethoxy)-benzamide (Compound No. 38),
4-Butoxy-N-(3,5-dichloro-pyridin-4-yl)-3-(5-methyl-3-pyridin-3-yl-4,5-dihydro-isoxazol-5-ylmethoxy)-benzamide (Compound No. 39),
4-Butoxy-N-(3,5-dichloro-pyridin-4-yl)-3-(3,5-dimethyl-4,5-dihydro-isoxazol-5-ylmethoxy)-benzamide (Compound No. 40),
4-Cyclopentyloxy-N-(3,5-dichloro-pyridin-4-yl)-3-(5-methyl-3-pyridin-3-yl-4,5-dihydro-isoxazol-5-ylmethoxy)-benzamide (Compound No. 41),
4-Cyclopentyloxy-N-(3,5-dichloro-pyridin-4-yl)-3-(3,5-dimethyl-4,5-dihydro-isoxazol-5-ylmethoxy)-benzamide (Compound No. 42),
N-(3,5-dichloro-pyridin-4-yl)-4-hydroxy-3-(5-methyl-3-pyridin-3-yl-4,5-dihydro-isoxazol-5-ylmethoxy)-benzamide (Compound No. 43),
N-(3,5-dichloro-pyridin-4-yl)-4-methoxy-3-[5-methyl-3-(1-phenyl-ethyl)-4,5-dihydro-isoxazol-5-ylmethoxy]-benzamide (Compound No. 44),
3-(3-Cyclohexyl-5-methyl-4,5-dihydro-isoxazol-5-ylmethoxy)-N-(3,5-dichloro-pyridin-4-yl)-4-methoxy-benzamide (Compound No. 45),
N-(3,5-dichloro-pyridin-4-yl)-4-methoxy-3-(5-methyl-3-p-tolyl-4,5-dihydro-isoxazol-5-yhnethoxy)-benzamide (Compound No. 46),
3-[3-(4-tert-Butyl-phenyl)-5-methyl-4,5-dihydro-isoxazol-5-ylmethoxy]-N-(3,5-dichloro-pyridin-4-yl)-4-methoxy-benzamide (Compound No. 47),
N-(3,5-dichloro-pyridin-4-yl)-4-methoxy-3-(5-methyl-3-O-tolyl-4,5-dihydro-isoxazol-5-ylmethoxy)-benzamide (Compound No. 48),
N-(3,5-dichloro-pyridin-4-yl)-4-methoxy-3-[3-(3-methoxy-phenyl)-5-methyl-4,5-dihydro-isoxazol-5-ylmethoxy]-benzamide (Compound No. 49),
2-{5-[5-(3,5-Dichloro-pyridin-4-ylcarbamoyl)-2-methoxy-phenoxymethyl]-5-methyl-4,5-dihydro-isoxazol-3-yl)-piperidine-1-carboxylic acid tert-butyl ester (Compound No. 50),
N-(3,5-dichloro-pyridin-4-yl)-3-[3-(3,4-difluoro-phenyl)-5-methyl-4,5-dihydro-isoxazol-5-ylmethoxy]-4-methoxy-benzamide (Compound No. 51),
N-(3,5-dichloro-pyridin-4-yl)-3-[3-(2,4-difluoro-phenyl)-5-methyl-4,5-dihydro-isoxazol-5-ylmethoxy]-4-methoxy-benzamide (Compound No. 52),
N-(3,5-dichloro-pyridin-4-yl)-3-[3-(4-fluoro-3-methyl-phenyl)-5-methyl-4,5-dihydro-isoxazol-5-ylmethoxy]-4-methoxy-benzamide (Compound No. 53),
N-(3,5-dichloro-pyridin-4-yl)-3-[3-(3,5-difluoro-phenyl)-5-methyl-4,5-dihydro-isoxazol-5-ylmethoxy]-4-methoxy-benzamide (Compound No. 54),
4-Benzyloxy-N-(3,5-dichloro-pyridin-4-yl)-3-(3,5-dimethyl-4,5-dihydro-isoxazol-5-ylmethoxy)-benzamide (Compound No. 55),
4-Cycloheptyloxy-N-(3,5-dichloro-pyridin-4-yl)-3-(3,5-dimethyl-4,5-dihydro-isoxazol-5-ylmethoxy)-benzamide (Compound No. 56),
3-[3-(3-Cyclopentyloxy-4-methoxy-phenyl)-5-methyl-4,5-dihydro-isoxazol-5-ylmethoxy]-N-(3,5-dichloro-pyridin-4-yl)-benzamide (Compound No. 57),
pharmaceutically acceptable salts, pharmaceutically acceptable solvates, stereoisomers, tautomers, racemates, polymorphs or N-oxides, thereof.

The term "pharmaceutically acceptable" means approved by regulatory agency of the federal or a state government or listed in the U.S. Pharmacopoeia or other generally recognized pharmacopeia for use in animals, and more particularly in humans.

The term "pharmaceutically acceptable salts" refers to derivatives of compounds that can be modified by forming their corresponding acid or base salts. Examples of pharmaceutically acceptable salts include, but are not limited to, mineral or organic acids salts of basic residues (such as amines), or alkali or organic salts of acidic residues (such as carboxylic acids), and the like.

The salt forms differ from the compound described herein in certain physical properties such as solubility, but the salts are otherwise equivalent for purposes of this invention.

The term "pharmaceutically acceptable solvates" refers to solvates with water (i.e. hydrates, hemihydrate or sesquihydrate) or pharmaceutically acceptable solvents, for example solvates with common organic solvents as ethanol and the like. Such solvates are also encompassed within the scope of the disclosure.

The term "polymorphs" includes all crystalline form as well as amorphous form for compounds described herein and as such are intended to be included in the present invention.

All stereoisomers of the compounds described herein are encompassed, either in admixture or in pure or substantially pure form. The compounds described herein can have asymmetric centers at any of the carbon atoms including all the substituents.

Consequently, compounds of present invention can exist in enantiomeric or diastereomeric forms or in mixtures thereof. The processes for the preparation can utilize racemates, enantiomers, or diastereomers as starting materials. When diastereomeric or enantiomeric products are prepared, they can be separated by conventional methods, for example, chromatographic or fractional crystallization.

Tautomers and all other possible isomers of compounds described herein are encompassed.

In another aspect, the present invention provides pharmaceutical compositions comprising, as an active ingredient, at least one of the compounds described herein or a pharmaceutically acceptable salt thereof together with a one or more pharmaceutically acceptable carriers or diluents. Compounds disclosed herein may be administered to human or animal for treatment by any route, which effectively transports the active compound to the appropriate or desired site of action such as oral, nasal, pulmonary, transdermal or parenteral (rectal, subcutaneous, intravenous, intraurethral, intramuscular, intranasal). Pharmaceutical compositions comprise a pharmaceutically effective amount of one or more compounds described herein formulated together with one or more pharmaceutically acceptable carriers or diluents. The term "pharmaceutically acceptable carriers" is intended to include non-toxic, inert solid, semi-solid or liquid filler, diluents, encapsulating material or formulation of any type.

The term "patient" means all mammals including humans.

Where desired, the compounds of Formula I and/ or their pharmaceutically acceptable salts, pharmaceutically acceptable solvates, stereoisomers, tautomers, racemates, polymorphs or N-oxides may be advantageously used in combination with one or more other therapeutic agents. Examples of other therapeutic agents, which may be used in combination with compounds of Formula I of this invention and/ or their pharmaceutically acceptable salts, pharmaceutically acceptable solvates, stereoisomers, tautomers, racemates, polymorphs or N-oxides include corticosteroids, beta agonists, leukotriene antagonists, 5-lipoxygenase inhibitors, chemokine inhibitors and muscarinic receptor antagonists.

While the present invention has been described in terms of its specific embodiments, certain modifications and equivalents will be apparent to those skilled in the art and are included within the scope of the present invention. The examples are provided to illustrate particular aspects of the disclosure and do not limit the scope of the present invention as defined by the claims.

### Examples

### Example 1: Preparation of 4-difluoromethoxy benzaldehyde

To a stirred solution of 4-hydroxy benzaldehyde (1.00 g, 8.19 mmol) in dimethylformamide was added benzyltriethyl ammonium chloride (0.93 g, 4.00 mmol) at room temperature. Freon gas was purged along with simultaneous addition of sodium hydroxide solution (0.82 g in 2.3 mL water, 20.5 mmol) for about 5-10 minutes. Freon gas was purged again for about 5 minutes along with simultaneous addition of sodium hydroxide solution (0.82 g in 2.3 mL water, 20.5 mmol). Water was added to the reaction mixture and the compound was extracted with ethyl acetate. The organic layer was washed with brine, dried over sodium sulfate and concentrated to yield oily residue. The residue was purified by column chromatography on silica gel (hexane:ethyl acetate, 95:5) to form the title compound.

### Yield: 0.83 g (59%)

The following compound was similarly prepared using the appropriate corresponding reagents:
4-Difluoromethoxy -3-hydroxy benzaldehyde

### Example 2: Preparation of 4-difluoromethoxy-3-hydroxy benzoic acid

To a previously cooled and stirred mixture of 4-difluoromethoxy -3-hydroxy benzaldehyde (0.3 g, 1.59 mmol) (Example 1) and sulphamic acid (0.23 g, 2.39 mmol) in 80% acetic acid (6.7 mL), was added aqueous solution of sodium chlorite (0.22 g in 0.7 mL water, 2.39 mmol) dropwise maintaining the temperature at 10 °C. The reaction mixture was stirred at room temperature for overnight, water was added to the reaction mixture and then stirred for about 15 minutes. The mixture was extracted with ethyl acetate. The organic layer was washed with brine, dried over anhydrous sodium sulfate and concentrated under vacuum to provide white solid.

### Yield: 0.25 g (77%)

### Example 3: Preparation of 3-hydroxy-4-methoxy-benzoic acid methyl ester

To a stirred solution of 3-hydroxy-4-methoxy benzoic acid (4.00 g, 23.7 mmol) (commercially available) in methanol, was added sulfuric acid (catalytic amount) and the reaction mixture was heated at 65-70 °C for about 15 hours. Methanol was removed by evaporation; water was added and the reaction mixture was extracted with ethyl acetate. The organic layer was separated, washed with water, dried over anhydrous sodium sulfate, filtered and the solvent was evaporated to yield the title compound.

### Yield: 4.0 g (93%)

The following compound was similarly prepared using the appropriate corresponding reagents:
4-Difluoromethoxy -3-hydroxy benzoic acid methyl ester

### Yield: 0.25 g (94%)

### Example 4: Preparation of 4-methoxy-3-(2-methyl-allyloxy)-benzoic acid methyl ester

To a solution of 3-hydroxy-4-methoxy benzoic acid methyl ester (4.0 g, 20.0 mmol) (Example 3) in dry dimethylformamide, potassium carbonate (6.0 g, 43.0 mmol) and ß-methallyl chloride (3.89 g, 43.0 mmol) were added. The reaction mixture was stirred at 60 °C for about 5 hours. The reaction mixture was filtered, diluted with water and extracted with ethyl acetate. The organic layer was separated, washed with brine, dried over anhydrous sodium sulfate, filtered and the solvent was evaporated to yield the title compound.

### Yield: 4.20 g (82%)

The following compound was similarly prepared using the appropriate corresponding reagents:
4-Difluoromethoxy-3-(2-methyl-allyloxy)-benzoic acid methyl ester

### Yield: 0.30 g (97%)

### Example 5: Preparation of 4-methoxy-3-(2-methyl-allyloxy)-benzoic acid

To a stirred solution of 4-methoxy-3-(2-methyl-allyloxy)-benzoic acid methyl ester (4.2 g, 17.7 mmol) (Example 4) in methanol-water (80:20), potassium hydroxide (2.9 g, 53.0 mmol) was added and the reaction mixture was heated at 60 °C for about 2 hours. Methanol was removed by evaporation, water was added, and the reaction mixture was acidified with hydrochloric acid and the product was extracted with ethyl acetate. The organic layer was washed with brine, dried over anhydrous sodium sulfate, filtered and the solvent was evaporated to yield the title compound.

### Yield: 3.75 g (95%)

The following compound was similarly prepared using the appropriate corresponding reagents:
4-Difluoromethoxy-3-(2-methyl-allyloxy)-benzoic acid

### Yield: 0.256 g (90%)

### Example 6: Preparation of N-(3,5-dichloro-pyridin-4-yl)-4-methoxy-3-(2-methyl-allyloxy)-benzamide

Oxalyl chloride (0.33 g, 2.20 mmol) was added dropwise to a stirred solution of 4-methoxy-3-(2-methyl-allyloxy)-benzoic acid (0.5 g, 2.2 mmol) (Example 5) in dichloromethane (3 mL) and dimethylformamide (2 drops) at 0 °C. The reaction mixture was further stirred at room temperature for about 3 hours. The solvent was removed by evaporation and the residue thus obtained was dissolved in dry dimethylformamide under nitrogen atmosphere and added dropwise to the pre-stirred suspension of 3,5-dichloro-4-amino pyridine (0.36 g, 2.2 mmol) and sodium hydride (0.11 g, 4.40 mmol) in dry dimethylformamide at 0 °C under anhydrous condition. After completion of the reaction, the reaction mixture was acidified with dilute hydrochloric acid, water was added and the mixture was extracted with ethyl acetate. The organic layer was separated, washed with brine, dried over anhydrous sodium sulfate, filtered and the solvent was evaporated. The residue thus obtained was purified by column chromatography to yield the title compound as white solid.

### Yield: 0.22 g (27%), m/z: 367.00 (M⁺+1), 389 (M⁺+23)

The following compound was similarly prepared using the appropriate corresponding reagents:
N-(3,5-dichloro-pyridin-4-yl)-4-difluoromethoxy-3-(2-methyl-allyloxy)-benzamide Yield: 0.32 g (73%)

### Example 7: Preparation of 3-cyclopentyloxy-4-methoxybenzaldehyde

To a solution of 3-hydroxy-4-methoxy benzaldehyde (25.0 g, 160 mmol) (commercially available) in dimethylformamide, potassium carbonate (45.3 g, 320 mmol) and potassium iodide (2.73 g, 16 mmol) were added. The reaction mixture was heated to 60 °C and further cyclopentyl bromide (35.8 g, 240 mmol) was added slowly over a period of 20 minutes and the reaction mixture was stirred at that temperature for about 22 hours. The reaction mixture was extracted with ethyl acetate. The combined organic layer was separated, washed with brine, dried over anhydrous sodium sulfate, filtered and the solvent was evaporated to yield the title compound.

### Yield: 32.0 g (89%)

### Example 8: Preparation of 3-cyclopentyloxy-4-methoxybenzaldehyde oxime

To a solution of 3-cyclopentyloxy-4-methoxybenzaldehyde (9.5 g, 43.0 mmol) (Example 7) in ethanol, hydroxylamine hydrochloride (6.0 g, 86.0 mmol) and sodium acetate (7.0 g, 86.0 mmol) were added. The reaction mixture was stirred at room temperature for about 2 hours. The solvent was evaporated, water was added and the reaction mixture was extracted with ethyl acetate. After extraction, the combined organic layer was separated, washed with brine, dried over anhydrous sodium sulfate, filtered and the solvent was evaporated to form the title compound.

### Yield: 9.4 g (95%)

The following compounds were prepared similarly
4-Difluoromethoxy benzaldehyde oxime, Yield: 1.74 g (99%)
4-Methyl benzaldehyde oxime, Yield: 1.0 g (74%)
4-tert-Butyl benzaldehyde oxime, Yield: 1.15 g (95%)
2-Phenyl propionaldehyde oxime, Yield: 5.9 g (93 %)
Cyclohexanecarbaldehyde oxime, Yield: 5.1 g (91%)

### Example 9: Preparation of 3-[3-(3-cyclopentyloxy-4-methoxy-phenyl-5-methyl-4,5-dihydro-isoxazol-5-ylmethoxy]-N-(3,5-dichloro-pyridin-4-yl)-4-methoxy-benzamide (Compound No. 1)

To a solution of N-(3,5-dichloro-pyridin-4-yl)-4-methoxy-3-(2-methyl-allyloxy)-benzamide (0.2 g, 0.54 mmol) (Example 6) and 3-cyclopentyloxy-4-methoxybenzaldehyde oxime (0.25 g, 1.08 mmol) (Example 8) in tetrahydrofuran, sodium hypochlorite (4% solution, 2 mL) was added dropwise over a period of 30 minutes. After stirring the reaction mixture for about 2 hours, tetrahydrofuran was removed by evaporation. The reaction mixture was diluted with water and extracted with ethyl acetate. The combined organic layer was washed with brine, dried over anhydrous sodium sulfate, and filtered. The solvent was removed by evaporation and the residue thus obtained was purified by column chromatography to yield the title compound.

### Yield: 0.04 g (13%), mp: 108 °C, m/z: 600 (M⁺+1), 601 (M⁺+2)

The following compound was similarly prepared using the appropriate corresponding reagents:
N-(3,5-dichloro-pyridin-4-yl)-3-(3-(4-difluoromethoxy-phenyl)-5-methyl-4,5-dihydro-isoxazol-5-ylmethoxy)-4-methoxy-benzamide (Compound No. 12)

### Yield: 10 mg (7%), m/z: 552 (M⁺+1)

### Example 10: Preparation of N-(3,5-dichloro-pyridin-4-yl)-3-(5-methyl-3-phenyl-4,5-dihydro-isoxazol-5-ylmethoxy)-4-methoxy-benzamide (Compound No. 10)

To a stirred solution of N-(3,5-dichloro-pyridin-4-yl)-4-methoxy-3-(2-methyl-allyloxy)-benzamide (0.1 g, 0.27 mmol) (Example 6), benzaldehyde oxime (0.098 g, 0.81 mmol) and triethylamine (4 drops) in dichloromethane, sodium hypochlorite (4%, 2 mL) was added dropwise over a period of about 20 minutes at room temperature. The reaction mixture was stirred for about 3 hours and water was added. The organic layer was washed with water, brine and dried over sodium sulfate. The reaction mixture was evaporated under vacuum and purified by column chromatography to yield the title compound as white solid.

### Yield: 0.04 g (31%), m/z: 486 (M⁺+1)

The following compound was similarly prepared using the appropriate corresponding reagents:
N-(3,5-dichloro-pyridin-4-yl)-3-(5-methyl-3-pyridin-3-yl-4,5-dihydro-isoxazol-5-ylmethoxy)-4-methoxy-benzamide (Compound No. 11)

### Yield: 0.030 g (23%), m/z: 487 (M⁺+1)

### Example 11: Preparation of N-(3,5-dichloro-pyridin-4-yl)-3,5-dimethyl-4,5-dihydro-isoxazol-5-ylmethoxy)-4-methoxy-benzamide (Compound No.2)

To a solution of acetaldoxime (0.03 g, 0.49 mmol, commercially available) in dichloromethane was added N-chloro succinimide (0.06 g, 0.49 mmol) and pyridine (1 drop). The reaction mixture was stirred at room temperature for about 20 minutes. N-(3,5-dichloro-pyridin-4-yl)-4-methoxy-3-(2-methyl-allyloxy)-benzamide (0.18 g, 0.49 mmol) (Example 6) in dichloromethane (2 mL) was added and the reaction mixture was stirred at 40-50 °C. Triethylamine (0.05 g, 0.51 mmol) was added dropwise at 40-50 °C over a period of 10 minutes. Heating was continued for about 30 minutes at the same temperature. The reaction mixture was cooled to room temperature, water was added and the mixture was extracted with dichloromethane. The organic layer was separated, washed with brine, dried over anhydrous sodium sulfate, filtered and the solvent was evaporated. The residue thus obtained was purified by column chromatography to yield the title compound.

### Yield: 0.03 g (15%), m.p.: 232 °C, m/z: 424 (M⁺+1)

The following compound was similarly prepared using the appropriate corresponding reagents:
N-(3,5-dichloro-pyridin-4-yl)-4-difluoromethoxy-3-(3,5-dimethyl-4,5-dihydro-isoxazol-5-ylmethoxy)-benzamide (Compound No. 13)

### Yield: 10 mg (3%), m/z: 460 (M⁺+1)

### Example 12: Preparation of 3-(tert-butyl-dimethyl-silanyloxy)-4-methoxy-benzoic acid

To a solution of 3-hydroxy-4-methoxybenzoic acid (0.5 g, 2.98 mmol) (commercially available) in dry dimethylformamide was added imidazole (0.3 g, 4.46 mmol) at room temperature. The reaction mixture was stirred for about 10 minutes and tert-butyldimethylsilyl chloride (0.54 g, 3.57 mmol) was added portion wise over a period of about 15 minutes. After stirring the reaction mixture for about 15 hours, ice-cold water was added. After extracting with ethyl acetate, the organic layer was washed with brine, dried over anhydrous sodium sulfate, the solvent was evaporated and the residue was purified by column chromatography to yield the title compound.

### Yield: 0.65 g (72%), m/z: 305 (M⁺+23)

### Example 13: Preparation of -3-(tert-butyl-dimethyl-silanyloxy)-N-(3,5-dichloro-pyridin-4 yl)-4-methoxy-benzamide

Oxalyl chloride (3.0 g, 23.70 mmol) was added dropwise to a stirred solution of 3-(tert-butyl-dimethyl-silanyloxy)-4-methoxy-benzoic acid (4.1 g, 14.5 mmol) (Example 12) in dichloromethane (40 mL) and dimethylformamide (4 drops) at 0 °C under an inert atmosphere. The reaction mixture was then warmed to room temperature and the stirring was continued for about 2 hours. The solvent was removed by evaporation and the residue thus obtained was dissolved in dry dimethylformamide and then added dropwise to a pre-stirred suspension of 3,5-dichloro-4-aminopyridine (2.96 g, 18.1 mmol) and sodium hydride (1.45 g, 36.3 mmol) in dry dimethylformamide at 0 °C under an inert atmosphere. The stirring was continued for about 30 minutes and then quenched with water. After extracting with ethyl acetate, the organic layer was separated, washed with brine, dried over sodium sulfate and concentrated under vacuum. The reaction mixture was purified using column chromatography to yield the title compound.

### Yield: 2.90 g (51 %)

### Example 14: Preparation of N-(3,5-dichloro-pyridin-4-yl)-3-hydroxy-4-methoxybenzamide

To a stirred solution of 3-(tert-butyl-dimethyl-silanyloxy)-N-(3,5-dichloro-pyridin-4-yl)-4-methoxy-benzamide (2.93 g, 6.86 mmol) (Example 13) in tetrahydrofuran, tetrabutyl ammonium fluoride (1.79 g, 6.86 mmol) was added at 0 °C under an inert atmosphere. After stirring the reaction mixture for about 30 minutes, water was added. The mixture was extracted with ethyl acetate. The combined organic layer was washed with brine, dried over sodium sulfate and evaporated. The compound was further purified using column chromatography to yield the title compound.

### Yield: 1.82 g (85%)

### Example 15: Preparation of (3,5-dichloro-pyridin-4-yl]-(3-hydroxy-4-methoxy-benzoyl)-carbamic acid tert-butyl ester

To a solution of N-(3,5-dichloro-pyridin-4-yl)-3-hydroxy-4-methoxy-benzamide (0.40 g, 1.28 mmol) (Example 14) in dichloromethane, triethylamine (0.39 g, 3.85 mmol) was added. The reaction mixture was stirred for about 10 minutes and ditertiary butyl dicarbonate (0.42 g, 1.92 mmol) was added dropwise. After stirring the reaction mixture for additional 2 hours, water was added and the reaction mixture was extracted with dichloromethane. The combined organic layer was washed with brine, dried over anhydrous sodium sulfate and the solvent was evaporated. The residue was purified using column chromatography to yield the title compound.

### Yield: 0.45 g (85%)

### Example 16: Preparation of {5-[tert-butoxycarbonyl-(3,5-dichloro-pyridin-4-yl)-aminocarbonyl]-2-methoxy-phenoxy}-acetic acid ethyl ester

To a solution of (3,5-dichloro-pyridin-4-yl)-(3-hydroxy-4-methoxy-benzoyl)-carbamic acid tert-butyl ester (0.45 g, 1.09 mmol) (Example 15) in dimethylformamide, potassium carbonate (0.45 g, 3.28 mmol) was added. The reaction mixture was stirred for about 10 minutes and ethylbromo acetate (0.27 g, 1.64 mmol) was added. After stirring the reaction mixture for about 16 hours at room temperature, water was added and the mixture was extracted with ethyl acetate. The combined organic layer was washed with brine, dried over anhydrous sodium sulfate and the solvent was evaporated to yield the title compound.

### Yield: 0.44 g (81%), m/z: 499(M⁺+1)

### Example 17: Preparation of (3,5-dichloro-pyridin-4-yl)-[3-(2-hydroxy-ethoxy)-4-methoxybenzoyl]-carbamic acid tert-butyl ester

Sodium borohydride (0.34 g, 8.80 mmol) was added slowly to a stirred solution of {5-[tert-butoxycarbonyl-(3,5-dichloro-pyridin-4-yl)-aminocarbonyl]-2-methoxy-phenoxy}-acetic acid ethyl ester (0.44 g, 0.88 mmol) (Example 16) in methanol at 0 °C under an inert atmosphere. After stirring the reaction mixture for about 2 hours, saturated solution of ammonium chloride was added followed by ethyl acetate and water. The organic layer was separated, washed with brine, dried over sodium sulfate and the solvent was evaporated. The residue was purified using column chromatography to yield the title compound.

### Yield: 0.35 g (90%), m/z: 457 (M⁺+1)

### Example 18: Preparation of (3,5-dichloro-pyridin-4-yl)-[3-(2-hydroxyimino-ethoxy)-4-methoxy-benzoyl]-carbamic acid tert-butyl ester

To a solution of chromium trioxide (0.45 g, 4.47 mmol) in dichloromethane and pyridine (0.71 g, 8.95 mmol) was added dropwise the solution of (3,5-dichloro-pyridin-4-yl)-[3-(2-hydroxy-ethoxy)-4-methoxy-benzoyl]-carbamic acid tert-butyl ester (0.34 g, 0.75 mmol) (Example 17) in dichloromethane. After stirring the reaction mixture for about 15 minutes, dichloromethane was removed by evaporation to reduce the volume by half, supernatant was decanted into another flask and hydroxylamino hydrochloride (0.21 g, 2.99 mmol) sodium acetate, (0.25 g, 2.99 mmol) and ethanol were added. After stirring the reaction mixture for about 15 hours, solvent was removed by evaporation and water was added. The mixture was extracted with ethyl acetate and organic layer was separated, washed, dried and the solvent was evaporated to yield the title compound.

### Yield: 0.24 g (52%)

### Example 19: Preparation of 4-[5-(3,5-dichloro-pyridin-4-ylcarbamol)-2-methoxy-phenoxymethyl]-5-methyl-4,5-dihydro-isoxazole-5-carboxylic acid methyl ester (Compound No. 4)

To a solution of (3,5-dichloro-pyridin-4-yl)-[3-(2-hydroxyimino-ethoxy)-4-methoxy-benzoyl]-carbamic acid tert-butyl ester (0.19 g, 0.41 mmol) (Example 18) in tetrahydrofuran, methylmethacrylate (0.08 g, 0.81 mmole) and sodium hypochlorite (2 mL) was added dropwise over a period of 30 minutes under an inert atmosphere. After stirring the reaction mixture for about 30 minutes, tetrahydrofuran was removed by evaporation and water was added. The mixture was extracted with ethyl acetate and the organic layer was separated, washed with brine, dried over sodium sulfate and solvent was evaporated. The residue, thus obtained, was taken in dichloromethane (5 mL) and to this was added trifluoroacetic acid (0.1 mL). After stirring the reaction mixture for about 30 minutes, residue was adsorbed on silica gel and purified using column chromatography to yield the title compound.

### Yield: 25 mg (11%), m/z: 457 (M⁺+23)

### Example 20: Preparation of 3-(5-cyano-5-methyl-4,5-dihydro-isoxazol-4-ylmethoxy)-N-(3,5-dichloro-pyridin-4-yl)-4-methoxy-benzamide (Compound No. 3)

To a solution of (3,5-dichloro-pyridin-4-yl)-[3-(2-hydroxyimino-ethoxy)-4-methoxy-benzoyl]-carbamic acid tert-butyl ester (0.07 g, 0.14 mmol) (Example 18) and methacrylonitrile (0.02 g, 0.27 mmol) in tetrahydrofuran, sodium hypochlorite (2 mL) was added dropwise over a period of about 30 minutes under an inert atmosphere. After stirring the reaction mixture for about 30 minutes at an ambient temperature, solvent was removed by evaporation and water was added. The reaction mixture was extracted with ethyl acetate and the organic layer was separated, washed with brine, dried over anhydrous sodium sulfate and the solvent was evaporated. The residue thus obtained was taken in dichloromethane and to this trifluoroacetic acid (0.1 mL) was added. After stirring the reaction mixture for about 30 minutes, the residue was adsorbed on silica gel and purified by column chromatography to yield the title compound.

### Yield: 0.02 g (28%), m/z: 469 (M⁺+1)

### Example 21: Preparation of 4-[5-(3,5-dichloro-pyridin-4-ylcarbamoyl)-2-methoxy-phenoxymethyl]-5-methyl-4,5-dihydro-isoxazole-5-carboxylic acid ethyl amide (Compound No. 5)

Ethyl amine (0.5 mL) was added to a stirred solution of 4-{5-[tert-butoxycarbonyl-(3,5-dichloro-pyridin-4-yl)-aminocarbonyl]-2-methoxy-phenoxymethyl}-5-methyl-4,5-dihydro-isoxazole-5-carboxylic acid methyl ester (0.03 g, 0.05 mmol) in tetrahydrofuran and the reaction mixture was stirred for about 16 hours at room temperature. The solvent was evaporated, and the residue thus obtained was adsorbed on silica gel and purified using column chromatography to yield the title compound.

### Yield: 14 mg (44%), m/z: 481(M⁺+1)

The following compound was similarly prepared using the appropriate corresponding reagents:
3-[5-(3,5-Dichloro-pyridin-4-ylcarbomoyl)-2-methoxy-phenoxymethyl]-5-methyl-4,5-dihydro-isoxazole-5-carboxylic acid (Compound No. 6)
3-[5-(3,5-Dichloro-pyridin-4-ylcarbomoyl)-2-methoxy-phenoxymethyl]-5-methyl-4,5-dihydro-isoxazole-5-carboxylic acid amide (Compound No. 7)
3-[5-(3,5-Dichloro-pyridin-4-ylcarbomoyl)-2-methoxy-phenoxymethyl]-5-methyl-4,5-dihydro-isoxazole-5-carboxylic acid methyl amide (Compound No. 8)
3-[5-(3,5-Dichloro-pyridin-4-ylcarbomoyl)-2-methoxy-phenoxymethyl]-5-methyl-4,5-dihydro-isoxazole-5-carboxylic acid dimethyl amide (Compound No. 9)

### Example 22: Preparation of 4-methoxy -3-[(5-methyl-3-phenyl-4,5-dihydroisoxazol-5-yl) methoxy] benzoic acid methyl ester

To a solution of 4-methoxy-3-(2-methyl-allyloxy)-benzoic acid methyl ester (1.0 g, 4.20 mmol) (Example 4) and commercially available benzaldehyde oxime (0.76 g, 6.30 mmol) in tetrahydrofuran, sodium hypochlorite (4% w/w, 2 mL) was added dropwise over a period of 30 minutes. After stirring the reaction mixture for about 2 hours, tetrahydrofuran was removed by evaporation; water and ethyl acetate were added. After extraction, the organic layer was separated, washed with brine, dried over anhydrous sodium sulfate, and filtered. The solvent was removed by evaporation and the residue thus obtained was purified by column chromatography to yield the title compound.

### Yield: 1.0 g (67%)

The following compound was similarly prepared using the appropriate corresponding reagents:
4-Methoxy-3-[(5-methyl-3-pyridin-4-yl-4,5-dihydroisoxazol-5-yl) methoxy]benzoic acid methyl ester
3-{[3-(2-fluorophenyl)-5-methyl-4,5-dihydroisoxazol-5-yl]methoxy}-4-methoxybenzoic acid methyl ester
3-{[3-(2,6-difluorophenyl)-5-methyl-4,5-dihydroisoxazol-5-yl]methoxy}-4-methoxybenzoic acid methyl ester
3-{[3-(4-fluorophenyl)-5-methyl-4,5-dihydroisoxazol-5-yl]methoxy}-4-methoxybenzoic acid methyl ester
3-{[3-(3-fluorophenyl)-5-methyl-4,5-dihydroisoxazol-5-yl]methoxy}-4-methoxybenzoic acid methyl ester

### Example 23: Preparation of 4-methoxy -3-[(5-methyl-3 phenyl-4,5-dihydroisoxazol-5-yl) methoxy] benzoic acid

To a solution of 4-methoxy -3-[(5-methyl-3-phenyl-4, 5-dihydroisoxazol-5-yl)methoxy] benzoic acid methyl ester (1.0 g, 2.80 mmol) (Example 22) in ethanol-water (90:10), sodium hydroxide (0.33 g, 8.40 mmol) was added and the reaction mixture was stirred with heating at 60 °C for about 2 hours. Ethanol was removed by evaporation, water was added, and the reaction mixture was acidified with hydrochloric acid and extracted with ethyl acetate. The organic layer was washed with brine, dried over anhydrous sodium sulfate, filtered and the solvent was evaporated to yield the title compound.

### Yield: 0.85 g (89%)

The following compound was similarly prepared using the appropriate corresponding reagents:
4-Methoxy-3-[(5-methyl-3-pyridin-4-yl-4,5-dihydroisoxazol-5-yl)methoxy]benzoic acid 3-{[3-(2-fluorophenyl)-5-methyl-4,5-dihydroisoxazol-5-yl]methoxy}-4-methoxybenzoic acid
3-{[3-(2,6-difluorophenyl)-5-methyl-4,5-dihydroisoxazol-5-yl]methoxy}-4-methoxybenzoic acid
3-{[3-(4-fluorophenyl)-5-methyl-4,5-dihydroisoxazol-5-yl]methoxy}-4-methoxybenzoic acid
-3-{[3-(3-fluorophenyl)-5-methyl-4,5-dihydroisoxazol-5-yl]methoxy}-4-methoxybenzoic acid

### Example 24: Preparation of N-(3,5-dichloro-pyridin-4-yl)-3-[3-(2-fluoro-phenyl)-5-methyl-4,5-dihydro-isoxazol-5-ylmethoxy)-4-methoxy-benzamide (Compound No. 15)

To a stirred solution of 3-{[3-(2-fluorophenyl)-5-methyl-4,5-dihydroisoxazol-5-yl]methoxy}-4-methoxybenzoic acid (0.64g, 1.70 mmol) (Example 23) and triethyl amine (0.36 mL, 0.25 mmol) in dimethylformamide, isobutyl chloroformate (0.24 mL, 1.90 mmol) was added dropwise at 0 °C over a period of 5 minutes and the stirring was continued for about 1 hours at room temperature. The resulting solution was added to a suspension of 3,5 dichloro-4-amino pyridine (0.56 g, 3.40 mmol) and sodium hydride (0.17 g, 4.2 mmol, 60% w/w) in dimethylformamide at 0 °C over a period of 10 minutes under an inert atmosphere and the stirring was continued for about 1 hours at 0-5 °C. The reaction mixture was quenched with dilute hydrochloride solution (10%, 20 mL) and extracted with dichloromethane. The combined organic layer was washed with brine, dried over sodium sulfate and concentrated to form the title compound.

### Yield: 0.32 g (38%), mp: 197°C, m/z: 503.9(M⁺ +1)

The following compound was similarly prepared using the appropriate corresponding reagents:
N-(3,5-dichloro-pyridin-4-yl)-3-[3-(2,6-difluoro-phenyl)-5-methyl-4,5-dihydro-isoxazol-5-ylmethoxy)-4-methoxy-benzamide (Compound No. 16) mp: 170 °C, m/z: 522.1 (W +1)
N-(3,5-dichloro-pyridin-4-yl)-3-[3-(4-fluoro-phenyl)-5-methyl-4,5-dihydro-isoxazol-5-ylmethoxy)-4-methoxy-benzamide (Compound No. 18) mp: 235 °C, m/z: 504(M⁺ +1)
N-(3,5-dichloro-pyridin-4-yl)-3-[3-(3-fluoro-phenyl)-5-methyl-4,5-dihydro-isoxazol-5-ylmethoxy)-4-methoxy-benzamide (Compound No. 20) mp: 234 °C, m/z: 504(M⁺ +1)

The following compounds were prepared following this example also
N-(3,5-dichloro-pyridin-4-yl)-3-(5-methyl-3-phenyl-4,5-dihydro-isoxazol-5-ylmethoxy)-4-methoxy-benzamide (Compound No. 10)
N-(3,5-dichloro-pyridin-4-yl)-3-(5-methyl-3-pyridin-3-yl-4,5-dihydro-isoxazol-5-ylmethoxy)-4-methoxy-benzamide (Compound No. 11)

### Example 25: Preparation of N-(3,5-dichloro-pyridin-4-yl-(5-methyl-3-phenyl-4,5-dihydro-isoxazol-5-ylmethoxy)-4-hydroxy-benzamide (Compound No. 14)

To a stirred solution of N-(3,5-dichloro-pyridin-4-yl)-3-(5-methyl-3-phenyl-4, 5-dihydro-isoxazol-5-ylmethoxy)-4-methoxy-benzamide (0.22 g, 0.45 mmol) (Example 10 and 24) and 1-bromo-4-nitrobenzene (0.18g, 0.90 mmol) in dichloromethane, aluminium chloride (0.89 g, 6.70 mmol) was added slowly at room temperature and the stirring was continued for 10 hours at room temperature. The reaction mixture was poured into ice-cold water (30 mL) and extracted with dichloromethane. The combined organic layer was washed with brine, dried over anhydrous sodium sulfate, filtered and concentrated. The residue thus obtained was adsorbed on silica gel and purified using column chromatography to yield the title compound.

### Yield: 0.17 g (81 %), m/z: 471.97(M⁺ +1)

The following compound was similarly prepared using the appropriate corresponding reagents:
N-(3,5-dichloro-pyridin-4-yl)-3-[3,5-dimethyl-4,5-dihydro-isoxazol-5-ylmethoxy)-4-hydroxy-benzamide (Compound No. 17) mp: 115.5 °C, m/z: 410.0 (M⁺ +1)
N-(3,5-dichloro-pyridin-4-yl)-3-[3-(3-fluoro-phenyl)-5-methyl-4,5-dihydro-isoxazol-5-ylmethoxy)-4-hydroxy-benzamide (Compound No. 19) mp: 219°C, m/z: 490.0 (M⁺+1)

### Example 26: Preparation of 3-[3-(4-tert-Butyl-phenyl)-5-methyl-4,5-dihydro-isoxazol-5-ylmethoxy]-N-(3,5-dichloro-pyridin-4-yl)-4-methoxy-benzamide (Compound No. 47)

A mixture of N-(3,5-dichloro-pyridin-4-yl)-4-methoxy-3-(2-methyl-allyloxy)-benzamide (0.10 g, 0.27 mmol) (Example 6) and 4-tert-Butyl benzaldehyde oxime (0.06 g, 0.35 mmol) (Example 8) was taken in dichloromethane. Pyridine (2-3 drops) was added to it followed with the addition of sodium hypochlorite (5 mL) (dropwise) at 0 °C. The reaction mixture was then allowed to stir at room temperature for about 2 days. The mixture was extracted with dichloromethane. The organic layer was washed with brine, dried over sodium sulfate and concentrated to yield the title compound.

### Yield: 0.046 g (31%), m/z: 542 (M⁺+1)

The following compound was similarly prepared using the appropriate corresponding reagents:
N-(3,5-dichloro-pyridin-4-yl)-4-methoxy-3-(5-methyl-3-O-tolyl-4,5-dihydro-isoxazol-5-ylmethoxy)-benzamide (Compound No. 48)

### Yield: 30 mg (22%), m/z: 500 (M⁺+1)

N-(3,5-dichloro-pyridin-4-yl)-4-methoxy-3-[3-(3-methoxy-phenyl)-5-methyl-4,5-dihydro-isoxazol-5-ylmethoxy]-benzamide (Compound No. 49)

### Yield: 39 mg (28%), m/z: 516 (M⁺+1)

### Example 27: Preparation of 4-Methoxy-3-[(5-methyl-3-p-tolyl-4,5-dihydroisoxazol-5-yl)methoxy]benzoic acid methyl ester

A mixture of 4-methoxy-3-(2-methyl allyloxy)benzoic acid methyl ester (1.8 g, 7.62 mmol) (Example 4) and 4-methyl benzaldehyde oxime (1.3 g, 9.10 mmol) was taken in dichloromethane. Pyridine (2 drops) was added to the stirred reaction mixture, the reaction mixture cooled to 0 °C and sodium hypochlorite (8 mL) was added dropwise. The reaction mixture was then allowed to stir at room temperature for 24 hours, poured into water (50 mL) and extracted with dichloromethane. The organic layer was dried over sodium sulfate and concentrated to yield the title compound.

### Yield: 0.60 g (21%).

### Example 28: Preparation of 4-Methoxy-3-[(5-methyl-3-p-tolyl-4,5-dihydroisoxazol-5-yl)methoxy]benzoic acid

4-Methoxy-3-[(5-methyl-3-p-tolyl-4,5-dihydroisoxazol-5-yl)methoxy]benzoic acid methyl ester (0.60 g, 1.63 mmol) (Example 27) was taken in methanol. Sodium hydroxide (aqueous) (0.13 g, 3.25 mmol) was added to it. The reaction mixture was then heated at 60 °C overnight. Methanol was evaporated and water was poured in to the residue. Aqueous layer was washed with ethyl acetate to remove organic impurities. The aqueous portion was then neutralized with 1.5 N hydrochloride solution to make pH = 5. The aqueous layer was then extracted with ethyl acetate (2 x 50 mL). The combined organic layer was dried over sodium sulfate and concentrated to yield the title compound Yield: 0.47 g (81%)

### Example 29: Preparation of N-(3,5-dichloro-pyridin-4-yl)-4-methoxy-3-(5-methyl-3 -p-tolyl-4,5-dihydro-isoxazol-5-ylmethoxy)-benzamide (Compound No. 46)

4-Methoxy-3-[(5-methyl-3-p-tolyl-4,5-dihydroisoxazol-5-yl)methoxy]benzoic acid (0.044 g, 0.124 mmol) (Example 28) was taken in 3-necked flask, fitted with septum, condenser under an inert atmosphere. Dry-dichloroethane (6 mL) and thionyl chloride (0.022 g, 0.19 mmol) were added. The reaction mixture was refluxed at 80 °C for about 1.5 hours. Excess of dichloroethane and thionyl chloride were removed under vacuum from the reaction mixture. In another round bottom flask, sodium hydride (0.08 g, 0.2106 moles) was taken in dry tetrahydrofuran (12 mL) at 0 °C and 3,5-dichloro-4-amino pyridine (0.03 g, 0.19 mmol) was added. After about half an hour, reaction mixture was cooled to 0 °C and the previously prepared acid chloride residue was dissolved in dry tetrahydrofuran (5 mL) and was slowly added to it at 0 °C. The reaction mixture was stirred at 0 °C to room temperature for 2 hours. Water (40 mL) was poured into reaction mixture. The mixture was extracted with ethyl acetate. The organic layer was concentrated and the crude product was crystallized using 2% dichloromethane in hexane to yield the title compound.

### Yield: 15 mg (24%), m/z: 500(M⁺+1).

### Example 30: Preparation of 2-hydroxymethyl-piperidine-1-carboxylic acid butyl ester

Pipridin-2-yl -methanol (1.3 g, 11.3 mmol) was taken in 10 mL of tetrahydrofuran. Aqueous sodium carbonate (10% w/v, 20 mL) was added to it followed by di-tert-butyl dicarbonate (4.93 g, 22.6 mmol). The reaction mixture was stirred at room temperature for about 1 hour, cooled, then neutralized with 1.5 N hydrochloric acid solution till the solution attained a pH value of 6-6.5. The mixture was extracted with ethyl acetate. The organic layer was dried and concentrated to yield the title compound.

### Yield: 1.9 g (79%).

### Example 31: Preparation of 2-formyl-pipridine-1-carboxylic acid tert-butvl ester

2-Hydroxymethyl-piperidine-1-carboxylic acid butyl ester (1.0 g, 4.65 mmol) (Example 30) was taken in dichloromethane. Celite and pyridinium chlorochromate (2.0 g, 9.30 mmol) was added to it. The reaction mixture was stirred at room temperature for about 2 hours. The reaction mixture was filtered through a celite pad. The filtrate was then concentrated to yield the title compound.

### Yield: 0.55 g (56%).

### Example 32: Preparation of 2-(hydroxyiminomethyl)piperidine-1-carboxylic acid tert-butyl ester

2-Formyl-pipridine-1-carboxylic acid tert-butyl ester (0.55 g, 2.58 mmol) (Example 31) was taken in ethanol. Hydroxylamine hydrochloride (0.54 g, 7.75 mmol) and sodium acetate (0.64 mg, 7.75 mmol) were added to it. The reaction mixture was then stirred for about 2 hours. Methanol was evaporated, water was added and the mixture was extracted with ethyl acetate. The organic layer was dried over sodium sulfate and concentrated to form the title compound.

### Yield: 0.40 g (68%).

### Example 33: Preparation of 2-{5-[5-(3,5-dichloro-pyridin-4-ylcarbamoyl)-2-methoxy phenoxymethyl]-5-methyl-4,5-dihydro-isoxazol-3-yl}-piperidine-1-carboxylic acid tert-butyl ester (Compound No. 50)

A mixture of N-(3,5-dichloro-pyridin-4-yl)-4-methoxy-3-(2-methyl-allyloxy)-benzamide (0.26 g, 0.71 mmol) (Example 6) and 2-(hydroxyiminomethyl)piperidine-1-carboxylic acid tert-butyl ester (Example 32) (0.404 g, 1.77 mmol) was taken in dichloromethane. Pyridine was added to it followed by dropwise addition of sodium hypochlorite (5.0 mL) at 0 °C. The reaction mixture was then allowed to stir at room temperature for 2 days. The mixture was extracted with dichloromethane. The organic layer washed with brine and concentrated to yield the title compound.

### Yield 0.046 g (14%), m/z: 593 (M⁺+1).

### Example 34: Preparation of N-(3,5-dichloro pyridin-4-yl)-4-methoxy-3-[5-methyl-3-(1-phenyl-ethyl)-4,5-dihydro-isoxazol-5-ylmethoxy]-benzamide (Compound No. 44)

To a stirred mixture of 2-phenyl propionaldehyde oxime (0.081 g, 0.54 mmole) (Example 8) and N-(3,5-dichloro-pyridin-4-yl)-4-methoxy-3-(2-methyl-allyloxy)-benzamide (0.10 g, 0.27 mmole) (Example 6) in a mixture of dichloromethane and chloroform (8:2, 20 mL) was added 3-4 drops of pyridine. Sodium hypochlorite solution (0.89 mL, 0.60 mmol) was added dropwise and the reaction mixture was stirred at room temperature for about 24 hours. Water was added to the reaction mixture and the compound was extracted with dichloromethane. The extract was washed successively with water, brine, dried over anhydrous sodium sulfate, and concentrated under reduced pressure to yield the residue. The residue was purified by column chromatography on silica gel (using n-hexane/ethyl acetate: 70/30) to yield the title compound.

### Yield: 10 mg, (7%), m/z: 514 (M⁺+1).

### Example 35: Preparation of 3-(3-Cyclohexyl-5-methyl-4,5-dihydro-isoxazol-5-ylmethoxy)-N-(3,5-dichloro-pyridin-4-yl)-4-methoxy-benzamide (Compound No. 45)

To a stirred mixture of cyclohexane carbaldehyde oxime (0.069 g, 0.55 mmol) (Example 8) and N-(3,5-dichloro-pyridin-4-yl)-4-methoxy-3-(2-methyl-allyloxy)-benzamide (Example 6) (0.10 mg, 0.27 mmol) in a mixture of dichloromethane and chloroform (3:2, 20 mL) was added 3-4 drops of pyridine. Sodium hypochlorite solution (0.87 mL, 0.60 mmol) was added dropwise. The reaction mixture was stirred at room temperature for about 24 hours. Water was added to the reaction mixture and the compound was extracted with dichloromethane. The organic portion was washed with water, brine, dried over anhydrous sodium sulfate and concentrated under reduced pressure to form the residue. The crude residue was purified by column chromatography on silica gel (using n-hexane/ethyl acetate: 70/30) to yield the title compound.

### Yield: 0.03 g (22%), m/z: 492 (M⁺+1).

### Example 36: Preparation of N-(3,5-dichloro-pyridin-4-yl)-4-ethoxy-3-(5-methyl-3-phenyl-4,5-dihydro-isoxazol-5-ylmethoxy)-benzamide (Compound No. 21)

To a stirred solution of the N-(3,5-dichloro-pyridin-4-yl)-3-(5-methyl-3-phenyl-4,5-dihydro-isoxazol-5-ylmethoxy)-4-hydroxy benzamide (0.07 g, 0.14 mmol) (Example 25) and potassium carbonate (0.022 g, 0.14 mmol) in acetonirile at 60 °C, ethyl iodide (0.024 mL, 0.15 mmol) was added. Stirring was continued at 50-60 °C for 10 hours. Acetonitrile was removed by evaporation and water was added to the 62 reaction mixture. The aqueous layer was extracted with ethyl acetate (2 x 30 mL) and washed with water, brine, dried over sodium sulfate, filtered and the solvent was removed by evaporation to form the title compound.

### Yield: 0.035 g (47%)

### Example 37: Preparation of 4-methoxy-3-[(5-methyl-3-pyridin-3-yl-4,5-dihydroisoxazol-5-yl)methoxy]benzoic acid methyl ester

To a stirred solution of 4-methoxy-3-(2-methyl-allyloxy)-benzoic acid methyl ester (2.0 g, 8.00 mmol) (Example 4) and pyridine-3-carbaldehyde oxime (1.46g, 12.0 mmol) in tetrahydrofuran (15mL), sodium hypochlorite solution (5 mL) was added. Stirring was continued at room temperature for about 10 hours Tetrahydrofuran was removed by evaporation from reaction mixture, water was added and the mixture was extracted with ethyl acetate. The organic layer was washed with water, dried over sodium sulfate, filtered and the solvent was removed by evaporation to form the title compound.

### Yield: 1.60 g (51%)

### Example 38: Preparation of 4-hydroxy-3-[(5-methyl-3-pyridin-3-yl-4,5-dihydroisoxazol-5-yl)methoxy]benzoic acid methyl ester

To a stirred solution of 4-methoxy-3-[(5-methyl-3-pyridin-3-yl-4,5-dihydroisoxazol-5-yl)methoxy]benzoic acid methyl ester (1.6 g, 4.30 mmol) (Example 37) in dichloromethane (50 mL), aluminium chloride (5.7 g, 43.0 mmol) was added 1-Bromo-4-nitro benzene (1.3 g, 6.40 mmol) was added at room temperature. Stirring was continued at room temperature for 17 hours. The reaction mixture was poured in to ice water, and the mixture was extracted with dichloromethane. The organic layer was washed with water, dried over sodium sulfate, filtered and the solvent was removed by evaporation. The residue thus obtained was triturated with 5% dichloromethane-hexane, the solvent was decanted to yield the title compound.

### Yield: 1.10 g (73%)

### Example 39: Preparation of 4-butoxy-3-[(5-methyl-3-pyridin-3-yl-4,5-dihydroisoxazol-5-yl)methoxy]benzoic acid methyl ester

To a stirred solution of the 4-hydroxy-3-[(5-methyl-3-pyridin-3-yl-4,5-dihydroisoxazol-5-yl)methoxy]benzoic acid methyl ester (0.15 g, 0.43 mmol) (Example 38) and potassium carbonate (1.1 g, 0.84 mmol) in dimethylformamide at 60°C, n-butyl bromide (0.086 g, 0.63 mmol) was added. Stirring was continued at 60 °C for 10 hours. Water was added to the reaction mixture and the mixture was extracted with ethyl acetate. The organic layer was washed with water, dried over sodium sulfate, filtered and the solvent was removed by evaporation to yield the title compound.

### Yield: 0.15 g (82%)

### Example 40: Preparation of 4-butoxy-3-[(5-methyl-3-pyridin-3-yl-4,5-dihydroisoxazol-5-yl)methoxy]benzoic acid

To a stirred solution of 4-butoxy-3-[(5-methyl-3-pyridin-3-yl-4,5-dihydroisoxazol-5-yl)methoxy]benzoic acid methyl ester (0.145 g, 0.35 mmol) (Example 39) in ethanol-water (95:5, 1mL), sodium hydroxide (0.072 g, 1.70 mmol) was added. Stirring was continued at 60-70 °C for about 10 hours. Ethanol was removed by evaporation and water was added to the reaction mixture. The reaction mixture was acidified with 5% hydrochloric acid solution. The mixture was extracted with ethyl acetate. The organic layer was washed with water, dried over sodium sulfate, filtered and the solvent was removed by evaporation to form the compound.

### Yield: 0.12 g (92%)

### Example 41: Preparation of 4-butoxy-N-(3,5-dichloro-pyridin-4-yl)-3-(5-methyl-3-pyridin-3-yl-4,5-dihydro-isoxazol-5-ymethoxy)-benzamide (Compound No. 39)

To a stirred solution of 4-butoxy-3-[(5-methyl-3-pyridin-3-yl-4,5-dihydroisoxazol-5-yl)methoxy]benzoic acid (0.12 g, 0.31 mmol) (Example 40) and triethyl amine (0.06 mL, 0.40 mmol) in dimethylformamide (3.0 mL), isobutyl chloroformate (0.04 mL, 0.32 mmol) was added dropwise at 0 °C over a period of 5 minutes and the stirring was continued for about 1 hour at room temperature. The resulting solution was added to a suspension of 3,5 dichloro-4-amino pyridine (0.10 g, 0.62 mmol) and sodium hydride (0.026 g, 0.65 mmol, 60% w/w) in dimethylformamide (3.0 mL) at 0 °C over a period of 10 minutes under an inert atmosphere and the stirring was continued for about 1 hour at 0-5°C. The reaction mixture was quenched with dilute hydrochloride solution (10%, 20 mL) and extracted with dichloromethane. The combined organic layer was washed with brine, dried over sodium sulfate and concentrated to form the title compound.

### Yield: 0.019 g (15%), m/z: 530 (M⁺+1).

The following compound was similarly prepared using the appropriate corresponding reagents:
4-Benzyloxy-N-(3,5-dichloro-pyridin-4-yl)-3-(3,5-dimethyl-4,5-dihydro-isoxazol-5-ylmethoxy)-benzamide (Compound No. 55)
4-Cycloheptyloxy-N-(3,5-dichloro-pyridin-4-yl)-3-(3,5-dimethyl-4,5-dihydro-isoxazol-5-ylmethoxy)-benzamide (Compound No. 56)
N-(3,5-dichloro-pyridin-4-yl)-4-isopropoxy-3-(5-methyl-3-pyridin-3-yl-4,5-dihydro-isoxazol-5-ylmethoxy)-benzamide (Compound No. 35); m/z: 515 (M⁺+1).
4-Cyclopropoxy-N-(3,5-dichloro-pyridin-4-yl)-3-(5-methyl-3-pyridin-3-yl-4,5-dihydro-isoxazol-5-ylmethoxy)-benzamide (Compound No. 37); m/z: 527 (M⁺+1).
4-Cyclopentyloxy-N-(3,5-dichloro-pyridin-4-yl)-3-(5-methyl-3-pyridin-3-yl-4,5-dihydro-isoxazol-5-ylmethoxy)-benzamide (Compound No. 41); m/z: 541 (M⁺+1).
N-(3 ,5-dichloro-pyridin-4-yl)-3-(3 ,5-dimethyl-4,5-dihydro-isoxazol-5-ylmethoxy)-4-isopropoxy-benzamide (Compound No. 36); m/z: 452 (M⁺+1).
N-(3,5-dichloro-pyridin-4-yl)-3-(3,5-dimethyl-4,5-dihydro-isoxazol-5-ylmethoxy)-4-propoxy-benzamide (Compound No. 34); m/z: 452 (M⁺+1).
N-(3,5-dichloro-pyridin-4-yl)-3-(3,5-dimethyl-4,5-dihydro-isoxazol-5-ylmethoxy)-4-ethoxy-benzamide (Compound No. 33); m/z: 438 (M⁺+1).

### Example 42: Efficacy of compounds as PDE IV inhibitors PDE-IV Enzyme Assay

The efficacy of compounds of PDE-4 inhibitors is determined by an enzyme assay using U937 cell cytosolic fraction (Biochemical and Biophysical Research Communications, 197: 1126-1131, 1993). Hydrolysis of cAMP to AMP was monitored using HPLC and [³H]cAMP in the sample was detected using FLO-ONE Detector.

The enzyme preparation was incubated in the presence and absence of the test compound for 30 minutes and amount of [³H]cAMP measured in the sample. The IC₅₀ values were found to be from about 0.0001 nM to about 16,900 nM, from about 0.0001 nM to about 2300 nM, from about 0.0001 nM to about 725 nM and even from about 0.0001 nM to about 150 nM.

## Claims

1. A compound having the structure of Formula I, 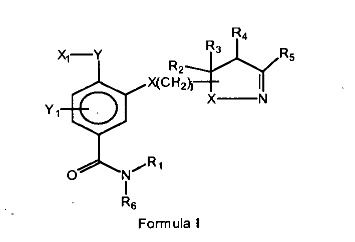 or a pharmaceutically acceptable salt, pharmaceutically acceptable solvate, stereoisomer, tautomer, racemate, polymorph or N-oxide thereof, wherein
R₁ and R₆ are independently hydrogen, alkyl, alkenyl, alkynyl, (un) saturated cycloalkyl,
aryl, aralkyl, heterocyclyl, heteroaryl, (heteroaryl)alkyl or (heterocyclyl)alkyl, X is oxygen, sulfur, or NRₕ, wherein
Rₕ is hydrogen, alkyl, alkenyl, alkynyl, (un) saturated cycloalkyl, acyl, aryl, aralkyl,
heteroaryl, heterocyclyl, (heteroaryl)alkyl, (heterocyclyl)alkyl, S(O)ₙR_{d}, wherein
R_{d} is alkyl, aryl, heterocyclyl or heteroaryl and
n is an integer from 0-2,
Y is no atom, oxygen, sulfur, NRₕ, wherein Rₕ is the same as defined above,
X₁ is hydrogen, alkyl, alkenyl, alkynyl, cycloalkyl, aryl, aralkyl, heteroaryl, heterocyclyl,
(heteroaryl)alkyl, (heterocyclyl)alkyl,
Y₁ is hydrogen, alkyl, alkenyl, alkynyl, nitro, cyano, halogen, ORₕ, SRₕ, NHRₕ, COOR',
COR', CONHR', S(O)ₙR_{d}, or Y₁ may be fused to benzene ring if Y₁ is aryl, heteroaryl or heterocyclyl, wherein
Rₕ and R_{d} are the same as defined above and
R' is hydrogen, alkyl, alkenyl, alkynyl, (un)saturated cycloalkyl, aryl, aralkyl,
heterocyclyl, heteroaryl, (heterocyclyl)alkyl or (heteroaryl)alkyl,
j is an integer from 1 to 3,
R₂ and R₃ are independently hydrogen, alkyl, alkenyl, alkynyl, cycloalkyl, nitro, cyano,
amino, substituted amino, alkoxy, aryloxy, COR', COOR' , aryl, aralkyl, heteroaryl, heterocyclyl, (heteroaryl)alkyl, (heterocyclyl)alkyl, (CH₂)₁₋₄ORR"', C(=O)NRₓR_{y}, (CH₂)ₘ-C(=O)R_{z}, wherein
R' is the same as defined above,
R"' is hydrogen, alkyl, alkenyl, alkynyl, (un)saturated cycloalkyl, aryl, aralkyl,
heterocyclyl, heteroaryl, (heterocyclyl)alkyl, (heteroaryl)alkyl or hydroxy,
Rₓ and R_{y} are independently hydrogen, alkyl, alkenyl of three to six carbon atoms,
alkynyl of three to six carbon atoms, (un)saturated cycloalkyl, aryl, aralkyl, heteroaryl, heterocyclyl, (heteroaryl)alkyl, or (heterocyclyl)alkyl,
m is an integer from 0-2 and
R_{z} is Rₚ or Rq, wherein
Rₚ is a 4-12 membered (un)saturated monocyclic or bicyclic ring containing
1-4 heteroatom(s) selected from N, O or S, wherein the said ring can be attached to (CH₂)ₘC(=O) through N, and
R_{q} is a 4-12 membered monocyclic or bicyclic ring containing 0-4
heteroatom(s) selected from N, O or S, wherein the said ring can be attached to (CH₂)ₘC(=O) through C,
R₄ is hydrogen, alkyl, halogen, hydroxy, cyano, carboxy, nitro, C(=O)NRₓR_{y}, wherein
Rₓ and R_{y} are the same as defined above, and
R₅ is hydrogen, alkyl, aryl, aralkyl, (un) saturated cycloalkyl, heteroaryl, heterocyclyl,
(heteroaryl)alkyl, or (heterocyclyl)alkyl,
wherein R₂, R₃, R₄ or R₅ are optionally replaced by a bond, represented for example by the compounds of Formula X and Formula XXII wherein R₃ and R₅ respectively are replaced by the bond

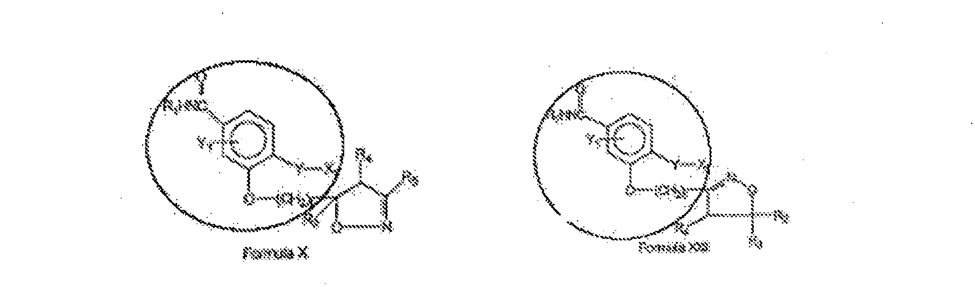

2. A compound according to claim 1, wherein R₁ is 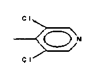 , R₄, R₆ and Y₁ are hydrogen,
X is oxygen, Y is no atom or oxygen, R₂ is methyl, j is 1, R₃ is replaced by said bond, X₁ is selected from hydrogen, cycloalkyl, alkyl, heterocyclyl, heteroaryl, (heterocyclyl)alkyl or (heteroaryl)alkyl and R₅ is selected from alkyl, aryl, cycloalkyl, heterocyclyl, heteroaryl, (heterocyclyl)alkyl or (heteroaryl)alkyl.

3. A compound according to claim 2, wherein X₁ is selected from methyl, difluoromethyl, ethyl, propyl, isopropyl, cyclopropyl, butyl, cyclopentyl, benzyl or cycloheptyl and R₅ is selected from methyl, phenyl, 3-pyridyl, 3-cyclopentyloxy-4-methoxy-phenyl, 4-difluoromethoxy-phenyl, 2-fluorophenyl, 2,6-difluorophenyl, 4-fluorophenyl, 3-fluorophenyl, 1-phenyl ethyl, cyclohexyl, p-tolyl, 4-tert-butyl phenyl, O-tolyl, 3-methoxy phenyl, piperidine-1-carboxylic acid tert-butyl ester, 3,4-difluorophenyl, 2,4-difluorophenyl, 4-fluoro-3-methyl phenyl or 3,5-difluoro phenyl.

4. A compound according to claim 1, wherein R₁ is 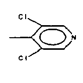 , R₄, R₆ and Y₁ are hydrogen, X and Y are oxygen, R₃ and X₁ are methyl, j is 1, R₅ is replaced by said bond and R₂ is selected from cyano, -COOR' or C(=O)NRₓR_{y}, wherein R', Rₓ and R_{y} are selected from hydrogen, alkyl, alkenyl, alkynyl, (un)saturated cycloalkyl, aryl, aralkyl, heterocyclyl, heteroaryl, (heterocyclyl)alkyl or (heteroaryl)alkyl.

5. A compound according to claim 4, wherein R', Rₓ and R_{y} are selected from methyl and ethyl.

6. A compound according to claim 1 selected from
3-[3-(3-Cyclopentyloxy-4-methoxy-phenyl)-5-methyl-4,5-dihydro-isoxazol-5-ylmethoxy]-N-(3,5-dichloro-pyridin-4-yl)-4-methoxy-benzamide,
N-(3,5-dichloro-pyridin-4-yl)-3-(3,5-dimethyl-4,5-dihydro-isoxazo-1-5-ylmethoxy)-4-methoxy-benzamide,
3-(5-Cyano-5-methyl-4,5-dihydro-isoxazol-4-ylmethoxy)-N-(3,5-dichloro-pyridin-4-yl)-4-methoxy-benzamide,
4-[5-(3,5-Dichloro-pyridin-4-ylcarbomoyl)-2-methoxy-phenoxymethyl]-5-methyl-4,5-dihydro-isoxazole-5-carboxylic acid methyl ester,
4-[5-(3,5-Dichloro-pyridin-4-ylcarbomoyl)-2-methoxy-phenoxymethyl]-5-methyl-4,5-dihydro-isoxazole-5-carboxylic acid ethyl amide,
3-[5-(3,5-Dichloro-pyridin-4-ylcarbomoyl)-2-methoxy-phenoxymethyl]-5-methyl-4,5-dihydro-isoxazole-5-carboxylic acid,
3-[5-(3,5-Dichloro-pyridin-4-ylcarbomoyl)-2-methoxy-phenoxymethyl]-5-methyl-4,5-dihydro-isoxazole-5-carboxylic acid amide,
3-[5-(3,5-Dichloro-pyridin-4-ylcarbomoyl)-2-methoxy-phenoxymethyl]-5-methyl-4,5-dihydro-isoxazole-5-carboxylic acid methyl amide,
3-[5-(3,5-Dichloro-pyridin-4-ylcarbomoyl)-2-methoxy-phenoxymethyl]-5-methyl-4,5-dihydro-isoxazole-5-carboxylic acid dimethyl amide,
N-(3,5-dichloro-pyridin-4-yl)-3-(5-methyl-3-phenyl-4,5-dihydro-isoxazol-5-ylmethoxy)-4-methoxy-benzamide,
N-(3,5-dichloro-pyridin-4-yl)-3-(5-methyl-3-pyridin-3-yl-4,5-dihydro-isoxazol-5-ylmethoxy)-4-methoxy-benzamide,
N-(3,5-dichloro-pyridin-4-yl)-3-(3-(4-difluoromethoxy-phenyl)-5-methyl-4,5-dihydro-isoxazol-5-ylmethoxy)-4-methoxy-benzamide,
N-(3,5-dichloro-pyridin-4-yl)-4-difluoromethoxy-3-(3,5-dimethyl-4,5-dihydro-isoxazol--5-ylmethoxy)-benzamide,
N-(3,5-dichloro-pyridin-4-yl)-3-(5-methyl-3-phenyl-4,5-dihydro-isoxazol-5-ylmethoxy)-4-hydroxy-benzamide,
N-(3,5-dichloro-pyridin-4-yl)-3-[3-(2-fluoro-phenyl)-5-methyl-4,5-dihydro-isoxazol-5-ylmethoxy)-4-methoxy-benzamide,
N-(3,5-dichloro-pyridin-4-yl)-3-[3-(2,6-difluoro-phenyl)-5-methyl-4,5-dihydro-isoxazol-5-ylmethoxy)-4-methoxy-benzamide,
N-(3,5-dichloro-pyridin-4-yl)-3-[3,5-dimethyl-4,5-dihydro-isoxazol-5-ylmethoxy)-4-hydroxy-benzamide,
N-(3,5-dichloro-pyridin-4-yl)-3-[3-(4-fluoro-phenyl)-5-methyl-4,5-dihydro-isoxazol-5-ylmethoxy)-4-methoxy-benzamide,
N-(3,5-dichloro-pyridin-4-yl)-3-[3-(3-fluoro-phenyl)-5-methyl-4,5-dihydro-isoxazol-5-ylmethoxy)-4-hydroxy-benzamide,
N-(3,5-dichloro-pyridin-4-yl)-3-[3-(3-fluoro-phenyl)-5-methyl-4,5-dihydro-isoxazol-5-ylmethoxy)-4-methoxy-benzamide,
N-(3,5-dichloro-pyridin-4-yl)-4-ethoxy-3-(5-methyl-3-phenyl-4,5-dihydro-isoxazol-5-ylmethoxy)-benzamide,
N-(3,5-dichloro-pyridin-4-yl)-4-ethoxy-3-[3-(3-fluoro-phenyl)-5-methyl-4,5-dihydro-isoxazol-5-ylmethoxy]-benzamide,
N-(3,5-dichloro-pyridin-4-yl)-3-(5-methyl-3-phenyl-4,5-dihydro-isoxazol-5-ylmethoxy)-4-propoxy-benzamide,
N-(3,5-dichloro-pyridin-4-yl)-3-[3-(3-fluoro-phenyl)-5-methyl-4,5-dihydro-isoxazol-5-ylmethoxy]-4-propoxy-benzamide,
N-(3,5-dichloro-pyridin-4-yl)-4-isopropoxy-3-(5-methyl-3-phenyl-4,5-dihydroisoxazol-5-ylmethoxy)-benzamide,
N-(3,5-dichloro-pyridin-4-yl)-3-[3-(3-fluoro-phenyl)-5-methyl-4,5-dihydro-isoxazol-5-ylmethoxy]-4-isopropoxy-benzamide,
4-Cyclopropoxy-N-(3,5-dichloro-pyridin-4-yl)-3-(5-methyl-3-phenyl-4,5-dihydro-isoxazol-5-ylmethoxy)-benzamide,
4-Cyclopropoxy-N-(3,5-dichloro-pyridin-4-yl)-3-[3-(3-fluoro-phenyl)-5-methyl-4,5-dihydro-isoxazol-5-ylmethoxy]-benzamide,
4-Butoxy-N-(3,5-dichloro-pyridin-4-yl)-3-(5-methyl-3-phenyl-4,5-dihydro-isoxazol-5-ylmethoxy)-benzamide,
4-Butoxy-N-(3,5-dichloro-pyridin-4-yl)-3-[3-(3-fluoro-phenyl)-5-methyl-4,5-dihydro-isoxazol-5-ylmethoxy]-benzamide,
4-Cyclopentyloxy-N-(3,5-dichloro-pyridin-4-yl)-3-(5-methyl-3-phenyl-4,5-dihydro-isoxazol-5-ylmethoxy)-benzamide,
4-Cyclopentyloxy-N-(3,5-dichloro-pyridin-4-yl)-3-[3-(3-fluoro-phenyl)-5-methyl-4,5-dihydro-isoxazol-5-ylmethoxy]-benzamide,
N-(3,5-dichloro-pyridin-4-yl)-3-(3,5-dimethyl-4,5-dihydro-isoxazol-5-ylmethoxy)-4-ethoxy-benzamide,
N-(3,5-dichloro-pyridin-4-yl)-3-(3,5-dimethyl-4,5-dihydro-isoxazol-5-ylmethoxy)-4-propoxy-benzamide,
N-(3,5-dichloro-pyridin-4-yl)-4-isopropoxy-3-(5-methyl-3-pyridin-3-yl-4,5-dihydro-isoxazol-5-ylmethoxy)-benzamide,
N-(3,5-dichloro-pyridin-4-yl)-3-(3,5-dimethyl-4,5-dihydro-isoxazol-5-ylmethoxy)-4-isopropoxy-benzamide,
4-Cyclopropoxy-N-(3,5-dichloro-pyridin-4-yl)-3-(5-methyl-3-pyridin-3-yl-4,5-dihydro-isoxazol-5-ylmethoxy)-benzamide,
4-Cyclopropoxy-N-(3,5-dichloro-pyridin-4-yl)-3-(3,5-dimethyl-4,5-dihydro-isoxazol-5-ylmethoxy)-benzamide,
4-Butoxy-N-(3,5-dichloro-pyridin-4-yl)-3-(5-methyl-3-pyridin-3-yl-4,5-dihydro-isoxazol-5-ylmethoxy)-benzamide,
4-Butoxy-N-(3,5-dichloro-pyridin-4-yl)-3-(3,5-dimethyl-4,5-dihydro-isoxazol-5-ylmethoxy)-benzamide,
4-Cyclopentyloxy-N-(3,5-dichloro-pyridin-4-yl)-3-(5-methyl-3-pyridin-3-yl-4,5-dihydro-isoxazol-5-ylmethoxy)-benzamide,
4-Cyclopentyloxy-N-(3,5-dichloro-pyridin-4-yl)-3-(3,5-dimethyl-4,5-dihydro-isoxazol-5-ylmethoxy)-benzamide,
N-(3,5-dichloro-pyridin-4-yl)-4-hydroxy-3-(5-methyl-3-pyridin-3-yl-4,5-dihydro-isoxazol-5-ylmethoxy)-benzamide,
N-(3,5-dichloro-pyridin-4-yl)-4-methoxy-3-[5-methyl-3-( 1-phenyl-ethyl)-4,5-dihydro-isoxazol-5-ylmethoxy]-benzamide,
3-(3-Cyclohexyl-5-methyl-4,5-dihydro-isoxazol-5-ylmethoxy)-N-(3,5-dichloro-pyridin-4-yl)-4-methoxy-benzamide,
N-(3,5-dichloro-pyridin-4-yl)-4-methoxy-3-(5-methyl-3-p-tolyl-4,5-dihydro-isoxazol-5-ylmethoxy)-benzamide,
3-[3-(4-tert-Butyl-phenyl)-5-methyl-4,5-dihydro-isoxazol-5-ylmethoxy]-N-(3,5-dichloro-pyridin-4-yl)-4-methoxy-benzamide,
N-(3,5-dichloro-pyridin-4-yl)-4-methoxy-3-(5-methyl-3-O-tolyl-4,5-dihydro-isoxazol-5-ylmethoxy)-benzamide,
N-(3,5-dichloro-pyridin-4-yl)-4-methoxy-3-[3-(3-methoxy-phenyl)-5-methyl-4,5-dihydro-isoxazol-5-ylmethoxy]-benzamide,
2- {5-[5-(3,5-Dichloro-pyridin-4-ylcarbamoyl)-2-methoxy-phenoxymethyl]-5-methyl-4,5-dihydro-isoxazol-3-yl}-piperidine-1-carboxylic acid tert-butyl ester,
N-(3,5-dichloro-pyridin-4-yl)-3-[3-(3,4-difluoro-phenyl)-5-methyl-4,5-dihydro-isoxazol-5-ylmethoxy]-4-methoxy-benzamide,
N-(3,5-dichloro-pyridin-4-yl)-3-[3-(2,4-difluoro-phenyl)-5-methyl-4,5-dihydro-isoxazol-5-ylmethoxy]-4-methoxy-benzamide,
N-(3,5-dichloro-pyridin-4-yl)-3-[3-(4-fluoro-3-methyl-phenyl)-5-methyl-4,5-dihydro-isoxazol-5-ylmethoxy]-4-methoxy-benzamide,
N-(3,5-dichloro-pyridin-4-yl)-3-[3-(3,5-difluoro-phenyl)-5-methyl-4,5-dihydro-isoxazol-5-ylmethoxy]-4-methoxy-benzamide,
4-Benzyloxy-N-(3,5-dichloro-pyridin-4-yl)-3-(3,5-dimethyl-4,5-dihydro-isoxazo 1-5-ylmethoxy)-benzamide,
4-Cycloheptyloxy-N-(3,5-dichloro-pyridin-4-yl)-3-(3,5-dimethyl-4,5-dihydro-isoxazol-5-ylmethoxy)-benzamide,
3-[3-(3-Cyclopentyloxy-4-methoxy-phenyl)-5-methyl-4,5-dihydro-isoxazol-5-ylmethoxy]-N-(3,5-dichloro-pyridin-4-yl)-benzamide,
or a pharmaceutically acceptable salt, pharmaceutically acceptable solvate, stereoisomer, tautomer, racemate, polymorph or N-oxide thereof.

7. A pharmaceutical composition comprising a therapeutically effective amount of one or more compounds of claim 1 and one or more pharmaceutically acceptable carriers, excipients or diluents.

8. The pharmaceutical composition of claim 7 further comprising one or more the therapeutic agents selected from corticosteroids, beta agonists, leukotriene antagonists, 5-lipoxygenase inhibitors, chemokine inhibitors or muscarinic receptor antagonists.

9. Compound according to claim 1 for use in treating, preventing, inhibiting or suppressing an inflammatory condition or disease in a patient.

10. The pharmaceutical composition of claim 7 for use in treating, preventing, inhibiting or suppressing an inflammatory condition or disease in a patient.

11. A compound of Formula I and one or more pharmaceutically acceptable carriers, excipients or diluents for use in the treatment, prevention, inhibition or suppression of AIDS, asthma, arthritis, bronchitis, chronic obstructive pulmonary disease (COPD), psoriasis, allergic rhinitis, shock, atopic dermatitis, Crohn's disease, adult respiratory distress syndrome (ARDS), eosinophilic granuloma, allergic conjunctivitis, osteoarthritis, ulcerative colitis or other inflammatory diseases in a patient.

12. A method for preparing a compound of Formula X, 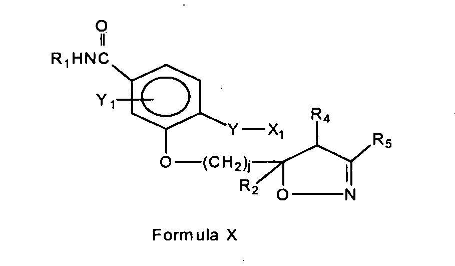 it's pharmaceutically acceptable salt, pharmaceutically acceptable solvate, stereoisomer, tautomer, racemate, polymorph or N-oxide thereof comprising the steps of:
a) oxidizing a compound of Formula II, 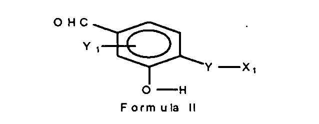 to form a compound of Formula II (a), 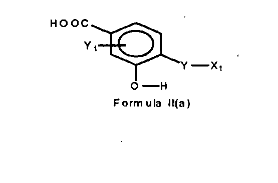
b) esterifying the compound of Formula II (a) to form a compound of Formula III, 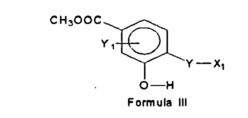
c) reacting the compound of Formula III with a compound of Formula IV (wherein X₃ is halogen 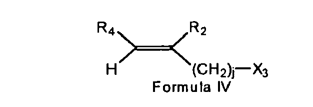 to form a compound of Formula V, 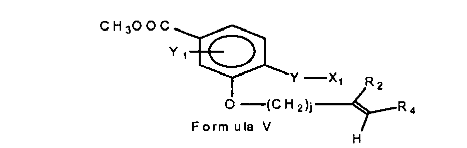
d) ester hydrolyzing the compound of Formula V to form a compound of Formula VI, 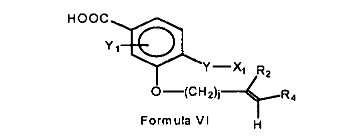
e) reacting the compound of Formula VI with a compound of Formula VII to form a compound of Formula VIII 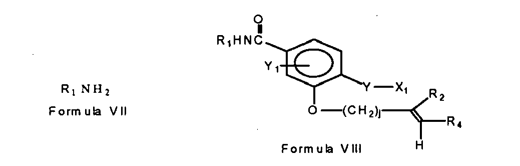
f) reacting the compound of Formula VIII with a compound of Formula IX,
R₅HC=NOH Formula IX
to form a compound of Formula X,
wherein all substituents have the same meaning as in claim 1.

13. A method for preparing a compound of Formula XXII, 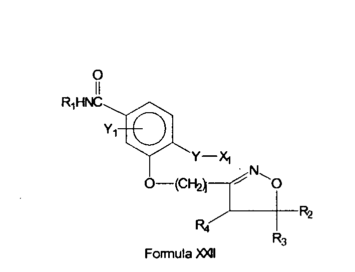 a pharmaceutically acceptable salt, pharmaceutically acceptable solvate, stereoisomer, tautomer, racemate, polymorph or N-oxide thereof comprising the steps of:
a) protecting a compound of Formula II (a), 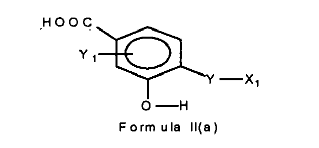 with a compound of Formula RX₃ (wherein X₃ can be halogen and R is a hydroxy protecting group) to form a compound of Formula XI, 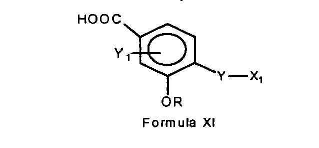
b) reacting the compound of Formula XI with a compound of Formula VII to form a compound of Formula XII, 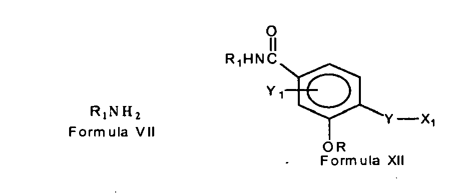
c) deprotecting the compound of Formula XII to form a compound of Formula XIII, 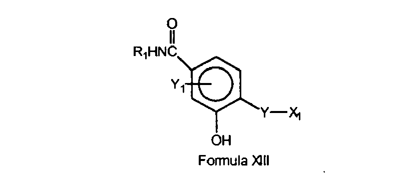
d) N-protecting the compound of Formula XIII to form a compound of Formula XIV (wherein Pr is a amino protecting group), 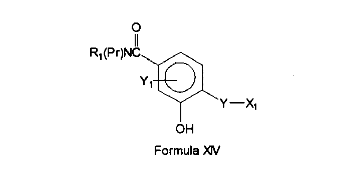
e) reacting the compound of Formula XIV with a compound of Formula XV (wherein X₃ is the same as defined earlier) to form a compound of Formula XVI, 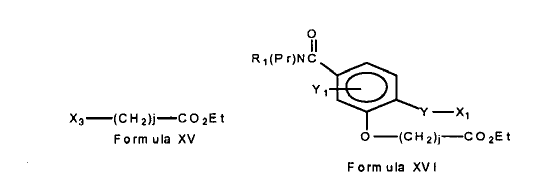
f) reducing the compound of Formula XVI to form a compound of Formula XVII, 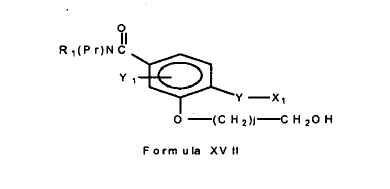
g) oxidizing the compound of Formula XVII to form a compound of Formula XVIII, 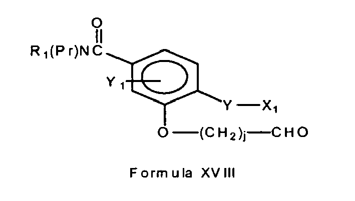
h) reacting the compound of Formula XVIII with hydroxylamine hydrochloride to form a compound of Formula XIX, 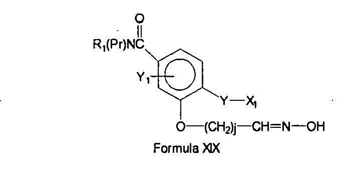
i) reacting the compound of Formula XIX with a compound of Formula XX to form a compound of Formula XXI, and 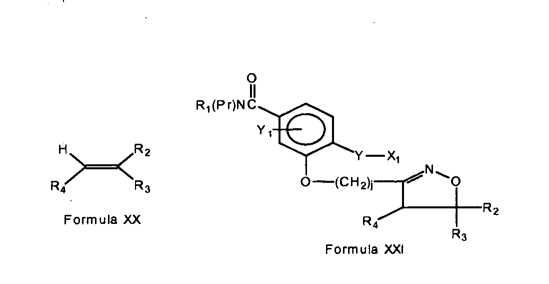
j) deprotecting the compound of Formula XXI to form a compound of Formula XXII, wherein all substituents have the same meaning as in claim 1.

14. A method for preparing a compound of Formula XXVI, 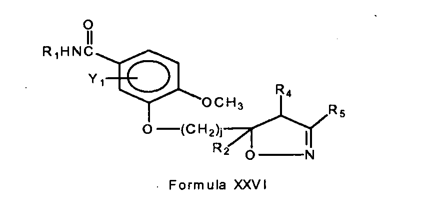 a pharmaceutically acceptable salt, pharmaceutically acceptable solvate, stereoisomer, tautomer, racemate, polymorph or N-oxide thereof comprising the steps of:
a) reacting a compound of Formula XXIII with a compound of Formula IX, 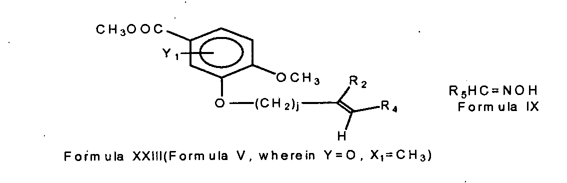 to form a compound of Formula XXIV, 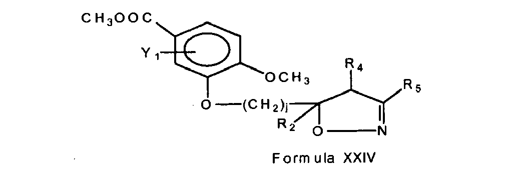
b) ester hydrolyzing the compound of Formula XXIV to form a compound of Formula XXV, and 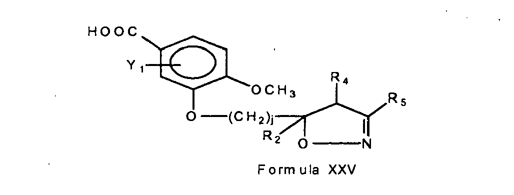
c) reacting the compound of Formula XXV with a compound of Formula VII,
R₁NH₂ Formula VII
to form a compound of Formula XXVI
wherein all substituents have the same meaning as in claim 1.

15. A method for preparing a compound of Formula XXVII, 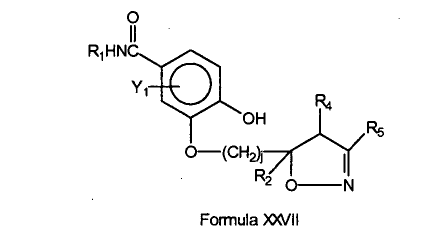 a pharmaceutically acceptable salt, pharmaceutically acceptable solvate, stereoisomer, tautomer, racemate, polymorph or N-oxide thereof comprising:
a) demethylation of a compound of Formula XXVI 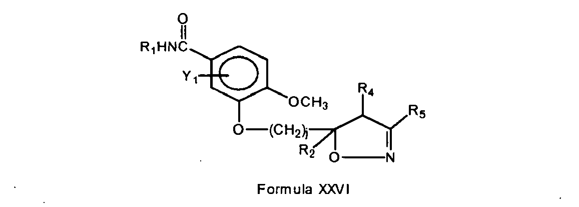 to form a compound of Formula XXVII,
wherein all substituents have the same meaning as in claim 1.

16. A method for preparing a compound of Formula XXIX, 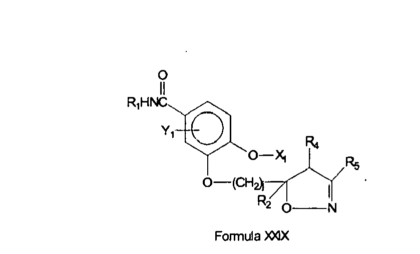 a pharmaceutically acceptable salt, pharmaceutically acceptable solvate, stereoisomer, tautomer, racemate, polymorph or N-oxide thereof comprising:
(a) reacting a compound of Formula XXVII, 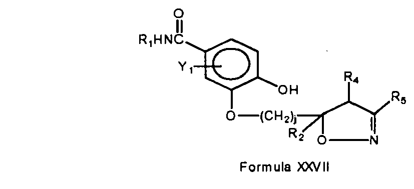 with a compound of Formula XXVIII,
X₁——h alid e Formula XXV III
to form a compound of Formula XXIX; or
(b)
(i) demethylating a compound of Formula XXIV to form a compound of Formula
XXX, 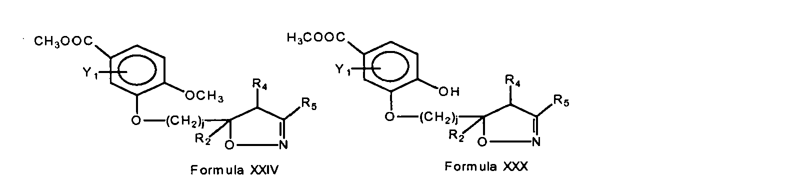
(ii) reacting the compound of Formula XXX with a compound of Formula XXVIII to form a compound of Formula XXXI, 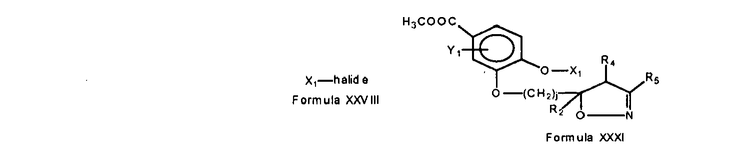
(iii) ester hydrolyzing the compound of Formula XXXI to form a compound of Formula XXXII, and 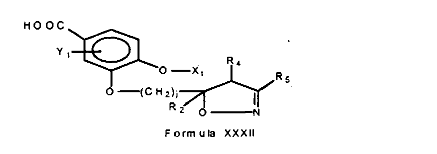
(iv) reacting the compound of Formula XXXII with a compound of Formula VII
R₁NH₂ Formula VII
to form a compound of Formula XXIX,
wherein all substituents have the same meaning as in claim 1.

## Patentansprüche

1. Verbindung mit der Struktur der Formel I 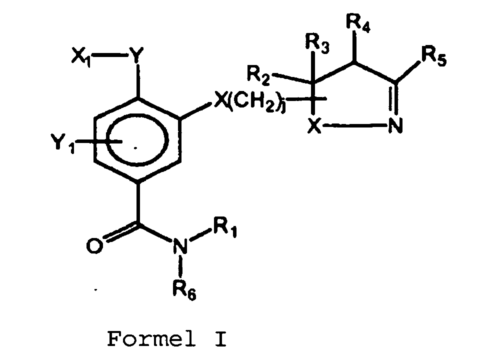 oder ein pharmazeutisch zulässiges Salz, pharmazeutisch zulässiges Solvat, Stereoisomer, Tautomer, Razemat, Polymorph oder N-Oxid davon, worin
R₁ und R₆ unabhängig voneinander Wasserstoff, Alkyl, Alkenyl, Alkinyl, (un)gesättigtes Cycloalkyl, Aryl, Aralkyl, Heterocyclyl, Heteroaryl, (Heteroaryl)alkyl oder (Heterocyclyl)alkyl sind,
X Sauerstoff, Schwefel oder NRₕ ist, worin
Rₕ Wasserstoff, Alkyl, Alkenyl, Alkinyl, (un)gesättigtes Cycloalkyl, Acyl, Aryl, Aralkyl, Heteroaryl, Heterocyclyl, (Heteroaryl)alkyl, (Heterocyclyl)alkyl, S(O)ₙR_{d} ist, worin
R_{d} Alkyl, Aryl, Heterocyclyl oder Heteroaryl und
n eine ganze Zahl von 0 bis 2 ist,
Y kein Atom, Sauerstoff, Schwefel, NRₕ ist, worin Rₕ dasselbe wie oben definiert ist,
X₁ Wasserstoff, Alkyl, Alkenyl, Alkinyl, Cycloalkyl, Aryl, Aralkyl, Heteroaryl, Heterocyclyl, (Heteroaryl)alkyl oder (Heterocyclyl)alkyl ist,
Y₁ Wasserstoff, Alkyl, Alkenyl, Alkinyl, Nitro, Cyan, Halogen, ORₕ, SRₕ, NHRₕ, COOR', COR', CONHR', S(O)ₙR_{d} ist oder Y₁, wenn
Y₁ Aryl, Heteroaryl oder Heterocyclyl ist, an einen Benzolring kondensiert sein kann, worin
Rₕ und R_{d} dasselbe wie oben definiert sind,
R' Wasserstoff, Alkyl, Alkenyl, Alkinyl, (un)gesättigtes Cycloalkyl, Aryl, Aralkyl, Heterocyclyl, Heteroaryl, (Heterocyclyl)alkyl oder (Heteroaryl)alkyl ist,
j eine ganze Zahl von 1 bis 3 ist,
R₂ und R₃ unabhängig voneinander Wasserstoff, Alkyl, Alkenyl, Alkinyl, Cycloalkyl, Nitro, Cyan, Amino, substituiertes Amino, Alkoxy, Aryloxy, COR', COOR', Aryl, Aralkyl, Heteroaryl, Heterocyclyl, (Heteroaryl)alkyl, (Heterocyclyl)alkyl, (CH₂) ₁₋₄OR''', C(=O)NRₓR_{y}, (CH₂) ₘ-C (=O) R_{z} sind, worin
R' dasselbe wie oben definiert ist,
R"' Wasserstoff, Alkyl, Alkenyl, Alkinyl, (un)gesättigtes Cycloalkyl, Aryl, Aralkyl, Heterocyclyl, Heteroaryl, (Heterocyclyl)alkyl, (Heteroaryl)alkyl oder Hydroxyl ist,
Rₓ und R_{y} unabhängig voneinander Wasserstoff, Alkyl, Alkenyl mit drei bis sechs Kohlenstoffatomen, Alkinyl mit drei bis sechs Kohlenstoffatomen, (un)gesättigtes Cycloalkyl, Aryl, Aralkyl, Heteroaryl, Heterocyclyl, (Heteroaryl)alkyl oder (Heterocyclyl)alkyl sind,
m eine ganze Zahl von 0 bis 2 ist,
R_{z} gleich Rₚ oder R_{q} ist, worin
Rₚ ein 4-12-gliedriger (un)gesättigter mono- oder bicyclischer Ring mit 1-4 aus N, O oder S ausgewählten Heteroatomen ist, wobei dieser Ring über N an (CH₂)ₘC(=O) gebunden sein kann und
R_{q} ein 4-12-gliedriger (un)gesättigter mono- oder bicyclischer Ring mit 0-4 aus N, O oder S ausgewählten Heteroatomen ist, wobei dieser Ring über C an (CH₂)ₘC(=O) gebunden sein kann,
R₄ Wasserstoff, Alkyl, Halogen, Hydroxyl, Cyan, Carboxy, Nitro, C(=O)NRₓR_{y} ist, worin Rₓ und R_{y} dasselbe wie oben definiert sind,
R₅ Wasserstoff, Alkyl, Aryl, (un)gesättigtes Cycloalkyl, Heteroaryl, Heterocyclyl, (Heteroaryl)alkyl oder (Heterocyclyl)alkyl ist, worin
R₂, R₃, R₄ oder R₅ gegebenenfalls durch eine Bindung ersetzt sind, beispielsweise dargestellt durch die Verbindungen der Formel X und der Formel XXII, worin R₃ bzw. R₅ durch die Bindung dargestellt sind.

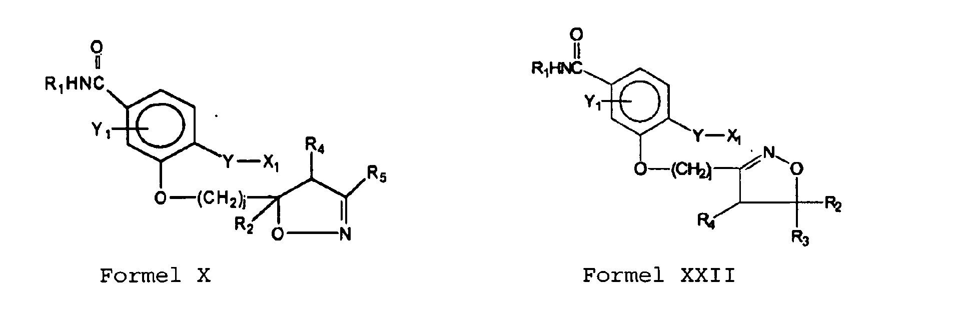

2. Verbindung nach Anspruch 1, worin R₁ 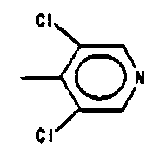 ist, R₄, R₆ und Y₁ Wasserstoff sind,
X Sauerstoff ist, Y kein Atom oder Sauerstoff ist, R₂ Methyl ist, j 1 ist, R₃ durch diese Bindung ersetzt ist, X₁ ausgewählt ist aus Wasserstoff, Cycloalkyl, Alkyl, Heterocyclyl, Heteroaryl, (Heterocyclyl)alkyl oder (Heteroaryl)alkyl und R₅ ausgewählt ist aus Alkyl, Aryl, Cycloalkyl, Heterocyclyl, Heteroaryl, (Heterocyclyl)alkyl oder (Heteroaryl)alkyl.

3. Verbindung nach Anspruch 2, worin X₁ ausgewählt ist aus Methyl, Difluormethyl, Ethyl, Propyl, Isopropyl, Cyclopropyl, Butyl, Cyclopentyl, Benzyl oder Cycloheptyl und R₅ ausgewählt ist aus Methyl, Phenyl, 3-Pyridyl, 3-Cyclopentyloxy-4-methoxyphenyl, 4-Difluormethoxyphenyl, 2-Fluorphenyl, 2,6-Difluorphenyl, 4-Fluorphenyl, 3-Fluorphenyl, 1-Phenylethyl, Cyclohexyl, p-Tolyl, 4-t-Butylphenyl, o-Tolyl, 3-Methoxyphenyl, Piperidin-1-carbonsäure-t-butylester, 3,4-Difluorphenyl, 2,4-Difluorphenyl, 4-Fluor-3-methylphenyl oder 3,5-Difluorphenyl.

4. Verbindung nach Anspruch 1, worin R₁ 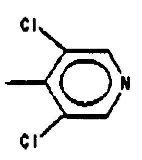 ist, R₄, R₆ und Y₁ Wasserstoff sind, X und Y Sauerstoff sind, R₃ und X₁ Methyl sind, j 1 ist, R₅ durch diese Bindung ersetzt ist und R₂ ausgewählt ist aus Cyan, -COOR' oder C(=O)NRₓR_{y}, worin R', Rₓ und R_{y} ausgewählt sind aus Wasserstoff, Alkyl, Alkenyl, Alkinyl, (un)gesättigtes Cycloalkyl, Aryl, Aralkyl, Heterocyclyl, Heteroaryl, (Heterocyclyl)alkyl oder (Heteroaryl)alkyl.

5. Verbindung nach Anspruch 4, worin R', Rₓ und R_{y} ausgewählt sind aus Methyl und Ethyl.

6. Verbindung nach Anspruch 1, ausgewählt aus
3-[3-(3-Cyclopentyloxy-4-methoxyphenyl)-5-methyl-4,5-dihydroisoxazol-5-ylmethoxy]-N-(3,5-dichlorpyridin-4-yl)-4-methoxybenzamid,
N-(3,5-Dichlor-pyridin-4-yl)-3-(3,5-dimethyl-4,5-dihydro-isoxazol-5-ylmethoxy)-4-methoxy-benzamid,
3-(5-Cyan-5-methyl-4,5-dihydroisoxazol-4-ylmethoxy)-N-(3,5-dichlorpyridin-4-yl)-4-methoxybenzamid,
4-[5-(3,5-Dichlorpyridin-4-ylcarbomoyl)-2-methoxy-phenoxymethyl]-5-methyl-4,5-dihydro-isoxazol-5-carbonsäuremethylester,
4-[5-(3,5-Dichlorpyridin-4-ylcarbomoyl)-2-methoxy-phenoxymethyl]-5-methyl-4,5-dihydroisoxazol-5-carbonsäureethylamid,
3-[5-(3,5-Dichlorpyridin-4-ylcarbomoyl)-2-methoxy-phenoxymethyl]-5-methyl-4,5-dihydroisoxazol-5-carbonsäure,
3-[5-(3,5-Dichlorpyridin-4-ylcarbomoyl)-2-methoxy-phenoxymethyl]-5-methyl-4,5-dihydroisoxazol-5-carbonsäureamid,
3-[5-(3,5-Dichlorpyridin-4-ylcarbomoyl)-2-methoxy-phenoxymethyl]-5-methyl-4,5-dihydroisoxazol-5-carbonsäuremethylamid,
3-[5-(3,5-Dichlorpyridin-4-ylcarbomoyl)-2-methoxy-phenoxymethyl]-5-methyl-4,5-dihydroisoxazol-5-carbonsäuredimethylamid,
N-(3,5-Dichlorpyridin-4-yl)-3-(5-methyl-3-phenyl-4,5-dihydro-isoxazol-5-ylmethoxy)-4-methoxybenzamid,
N-(3,5-Dichlorpyridin-4-yl)-3-(5-methyl-3-pyridin-3-yl-4,5-dihydroisoxazol-5-ylmethoxy)-4-methoxybenzamid,
N-(3,5-Dichlorpyridin-4-yl)-3-(3-(4-difluormethoxyphenyl)-5-methyl-4,5-dihydroisoxazol-5-ylmethoxy)-4-methoxybenzamid,
N-(3,5-Dichlorpyridin-4-yl)-4-difluormethoxy-3-(3,5-dimethyl-4,5-dihydroisoxazol-5-ylmethoxy)benzamid,
N-(3,5-Dichlorpyridin-4-yl)-3-(5-methyl-3-phenyl-4,5-dihydroisoxazol-5-ylmethoxy)-4-hydroxybenzamid,
N-(3,5-Dichlorpyridin-4-yl)-3-[3-(2-fluorphenyl)-5-methyl-4,5-dihydroisoxazol-5-ylmethoxy)-4-methoxybenzamid,
N-(3,5-Dichlorpyridin-4-yl)-3-[3-(2,6-difluorphenyl)-5-methyl-4,5-dihydroisoxazol-5-ylmethoxy)-4-methoxybenzamid,
N-(3,5-Dichlorpyridin-4-yl)-3-[3,5-dimethyl-4,5-dihydroisoxazol-5-ylmethoxy)-4-hydroxybenzamid,
N-(3,5-Dichlorpyridin-4-yl)-3-[3-(4-fluorphenyl)-5-methyl-4,5-dihydroisoxazol-5-ylmethoxy)-4-methoxybenzamid,
N-(3,5-Dichlorpyridin-4-yl)-3-[3-(3-fluorphenyl)-5-methyl-4,5-dihydroisoxazol-5-ylmethoxy)-4-hydroxybenzamid,
N-(3,5-Dichlorpyridin-4-yl)-3-[3-(3-fluorphenyl)-5-methyl-4,5-dihydroisoxazol-5-ylmethoxy)-4-methoxybenzamid,
N-(3,5-Dichlorpyridin-4-yl)-4-ethoxy-3-(5-methyl-3-phenyl-4,5-dihydroisoxazol-5-ylmethoxy)benzamid,
N-(3,5-Dichlorpyridin-4-yl)-4-ethoxy-3-[3-(3-fluorphenyl)-5-methyl-4,5-dihydroisoxazol-5-ylmethoxy]benzamid,
N-(3,5-Dichlorpyridin-4-yl)-3-(5-methyl-3-phenyl-4,5-dihydroisoxazol-5-ylmethoxy)-4-propoxybenzamid,
N-(3,5-Dichlorpyridin-4-yl)-3-[3-(3-fluorphenyl)-5-methyl-4,5-dihydroisoxazol-5-ylmethoxy]-4-propoxybenzamid,
N-(3,5-Dichlorpyridin-4-yl)-4-isopropoxy-3-(5-methyl-3-phenyl-4,5-dihydroisoxazol-5-ylmethoxy)benzamid,
N-(3,5-Dichlorpyridin-4-yl)-3-[3-(3-fluorphenyl)-5-methyl-4,5-dihydroisoxazol-5-ylmethoxy]-4-isopropoxybenzamid,
4-Cyclopropoxy-N-(3,5-dichlorpyridin-4-yl)-3-(5-methyl-3-phenyl-4,5-dihydroisoxazol-5-ylmethoxy)benzamid,
4-Cyclopropoxy-N-(3,5-dichlorpyridin-4-yl)-3-[3-(3-fluorphenyl)-5-methyl-4,5-dihydroisoxazol-5-ylmethoxy]-benzamid,
4-Butoxy-N-(3,5-dichlorpyridin-4-yl)-3-(5-methyl-3-phenyl-4,5-dihydroisoxazol-5-ylmethoxy)benzamid,
4-Butoxy-N-(3,5-dichlorpyridin-4-yl)-3-[3-(3-fluorphenyl)-5-methyl-4,5-dihydroisoxazol-5-ylmethoxy]benzamid,
4-Cyclopentyloxy-N-(3,5-dichlorpyridin-4-yl)-3-(5-methyl-3-phenyl-4,5-dihydroisoxazol-5-ylmethoxy)benzamid,
4-Cyclopentyloxy-N-(3,5-dichlorpyridin-4-yl)-3-[3-(3-fluorphenyl)-5-methyl-4,5-dihydro-isoxazol-5-ylmethoxy]-benzamid,
N-(3,5-Dichlorpyridin-4-yl)-3-(3,5-dimethyl-4,5-dihydroisoxazol-5-ylmethoxy)-4-ethoxybenzamid,
N-(3,5-Dichlorpyridin-4-yl)-3-(3,5-dimethyl-4,5-dihydroisoxazol-5-ylmethoxy)-4-propoxybenzamid,
N-(3,5-Dichlorpyridin-4-yl)-4-isopropoxy-3-(5-methyl-3-pyridin-3-yl-4,5-dihydroisoxazol-5-ylmethoxy)benzamid,
N-(3,5-Dichlorpyridin-4-yl)-3-(3,5-dimethyl-4,5-dihydroisoxazol-5-ylmethoxy)-4-isopropoxybenzamid,
4-Cyclopropoxy-N-(3,5-dichlorpyridin-4-yl)-3-(5-methyl-3-pyridin-3-yl-4,5-dihydroisoxazol-5-ylmethoxy)benzamid,
4-Cyclopropoxy-N-(3,5-dichlorpyridin-4-yl)-3-(3,5-dimethyl-4,5₋dihydroisoxazol-5-ylmethoxy)benzamid,
4-Butoxy-N-(3,5-dichlorpyridin-4-yl)-3-(5-methyl-3-pyridin-3-yl-4,5-dihydroisoxazol-5-ylmethoxy)benzamid,
4-Butoxy-N-(3,5-dichlorpyridin-4-yl)-3-(3,5-dimethyl-4,5-dihydroisoxazol-5-ylmethoxy)benzamid,
4-Cyclopentyloxy-N-(3,5-dichlorpyridin-4-yl)-3-(5-methyl-3-pyridin-3-yl-4,5-dihydro-isoxazol-5-ylmethoxy)benzamid,
4-Cyclopentyloxy-N-(3,5-dichlorpyridin-4-yl)-3-(3,5-dimethyl-4,5-dihydroisoxazol-5-ylmethoxy)benzamid,
N-(3,5-Dichlorpyridin-4-yl)-4-hydroxy-3-(5-methyl-3-pyridin-3-yl-4,5-dihydroisoxazol-5-ylmethoxy)benzamid,
N-(3,5-Dichlorpyridin-4-yl)-4-methoxy-3-[5-methyl-3-(1-phenylethyl)-4,5-dihydroisoxazol-5-ylmethoxy]benzamid,
3-(3-Cyclohexyl-5-methyl-4,5-dihydroisoxazol-5-ylmethoxy)-N-(3,5-dichlorpyridin-4-yl)-4-methoxybenzamid,
N-(3,5-Dichlorpyridin-4-yl)-4- methoxy-3-(5-methyl-3-p-tolyl-4,5-dihydroisoxazol-5-ylmethoxy)benzamid,
3-[3-(4-tert-Butylphenyl)-5-methyl-4,5-dihydroisoxazol-5-ylmethoxy]-N-(3,5-dichlorpyridin-4-yl)-4-methoxybenzamid,
N-(3,5-Dichlorpyridin-4-yl)-4-methoxy-3-(5-methyl-3-o-tolyl-4,5-dihydroisoxazol-5-ylmethoxy)benzamid,
N-(3,5-Dichlorpyridin-4-yl)-4-methoxy-3-[3-(3-methoxyphenyl)-5-methyl-4,5-dihydroisoxazol-5-ylmethoxy]benzamid,
2-{5-[5-(3,5-Dichlorpyridin-4-ylcarbamoyl)-2-methoxyphenoxymethyl]-5-methyl-4,5-dihydroisoxazol-3-yl}-piperidin-1-carbonsäure-tert-butylester,
N-(3,5-Dichlorpyridin-4-yl)-3-[3-(3,4-difluorphenyl)-5-methyl-4,5-dihydro-isoxazol-5-ylmethoxy]-4-methoxybenzamid,
N-(3,5-Dichlorpyridin-4-yl)-3-[3-(2,4-difluorphenyl)-5-methyl-4,5-dihydroisoxazol-5-ylmethoxy]-4-methoxybenzamid,
N-(3,5-Dichlorpyridin-4-yl)-3-[3-(4-fluor-3-methylphenyl)-5-methyl-4,5-dihydroisoxazol-5-ylmethoxy]-4-methoxybenzamid,
N-(3,5-Dichlorpyridin-4-yl)-3-[3-(3,5-difluorphenyl)-5-methyl-4,5-dihydroisoxazol-5-ylmethoxy]-4-methoxybenzamid,
4-Benzyloxy-N-(3,5-dichlorpyridin-4-yl)-3-(3,5-dimethyl-4,5-dihydroisoxazol-5-ylmethoxy)benzamid,
4-Cycloheptyloxy-N-(3,5-dichlorpyridin-4-yl)-3-(3,5-dimethyl-4,5-dihydroisoxazol-5-ylmethoxy)benzamid,
3-[3-(3-Cyclopentyloxy-4-methoxyphenyl)-5-methyl-4,5-dihydroisoxazol-5-ylmethoxy]-N-(3,5-dichlorpyridin-4-yl)-benzamid,
oder ein pharmazeutisch zulässiges Salz, pharmazeutisch zulässiges Solvat, Stereoisomer, Tautomer, Razemat, Polymorph oder N-Oxid davon.

7. Pharmazeutische Zusammensetzung, enthaltend eine therapeutisch wirksame Menge einer oder mehrerer Verbindungen nach Anspruch 1 und einen oder mehrere Träger, Hilfsstoffe oder Verdünner.

8. Pharmazeutische Zusammensetzung nach Anspruch 7, ferner einen oder mehrere aus Kortikosteroiden, Beta-Agonisten, Leukotrien-Agonisten, 5-Lipoxygenaseinhibitoren, Chemokininhibitoren oder Muskarinrezeptorantagonisten ausgewählte therapeutische Wirkstoffe enthaltend.

9. Verbindung nach Anspruch 1 zur Verwendung bei der Behandlung, Verhütung, Hemmung oder Unterdrückung eines entzündlichen Zustands oder einer entzündlichen Erkrankung bei einem Patienten.

10. Pharmazeutische Zusammensetzung nach Anspruch 7 zur Verwendung bei der Behandlung, Verhütung, Hemmung oder Unterdrückung eines entzündlichen Zustands oder einer entzündlichen Erkrankung bei einem Patienten.

11. Verbindung der Formel I und einer oder mehrere pharmazeutisch zulässige Träger, Hilfsstoffe oder Verdünner zur Verwendung bei der Behandlung, Verhütung, Hemmung oder Unterdrückung von AIDS, Asthma, Arthritis, Bronchitis, chronisch-obstruktive Lungenerkrankung (COPD), Psoriasis, allergische Rhinitis, Schock, atopische Dermatitis, Morbus Crohn, Atemnot-Syndrom des Erwachsenen (ARDS), eosinophiles Granulom, allergische Konjunktivitis, Arthrose, Colitis ulcerosa oder anderer entzündlicher Erkrankungen bei einem Patienten.

12. Verfahren zur Herstellung einer Verbindung der Formel X, 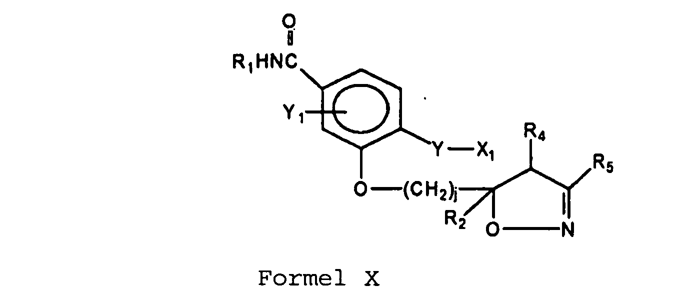 seines pharmazeutisch zulässigen Salzes, pharmazeutisch zulässigen Solvats, Stereoisomers, Tautomers, Razemats, Polymorphs oder N-Oxids davon, umfassend die Schritte
a) Oxidieren einer Verbindung der Formel II 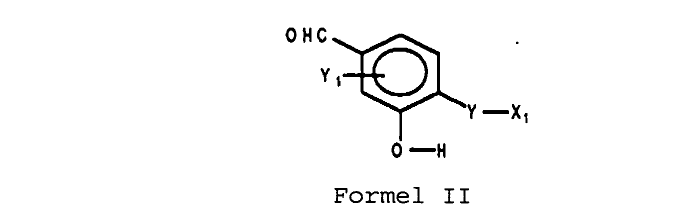 unter Bildung einer Verbindung der Formel II(a) 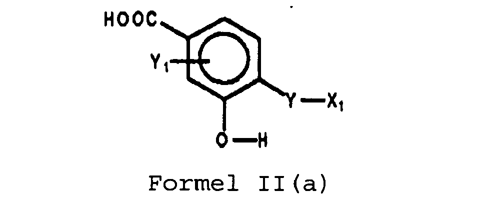
b) Verestern der Verbindung der Formel II(a) unter Bildung einer Verbindung der Formel III, 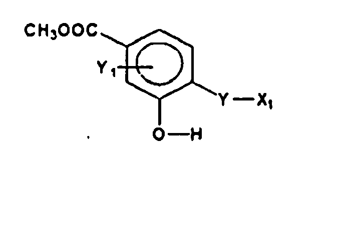 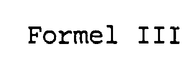
c) Umsetzung der Verbindung der Formel III mit einer Verbindung der Formel IV (worin X₃ Halogen ist) 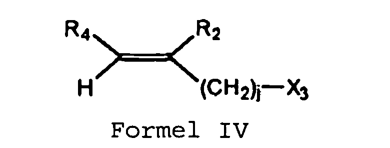 unter Bildung einer Verbindung der Formel V, 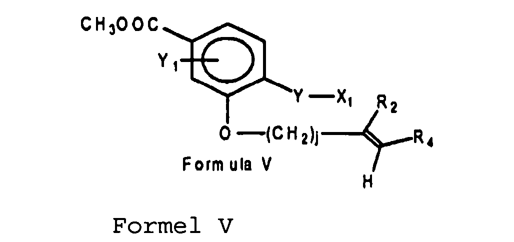
d) Esterhydrolyse der Verbindung der Formel V unter Bildung
einer Verbindung der Formel VI, 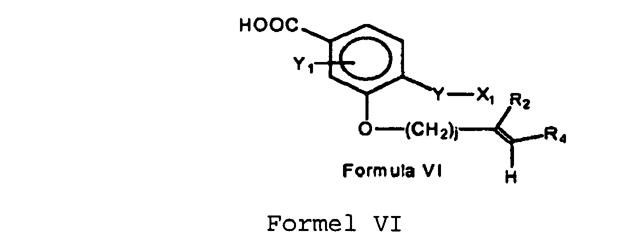
e) Umsetzung der Verbindung der Formel VI mit einer Verbindung der Formel VII unter Bildung einer Verbindung der Formel VIII,
R₁NH₂ Formel VII 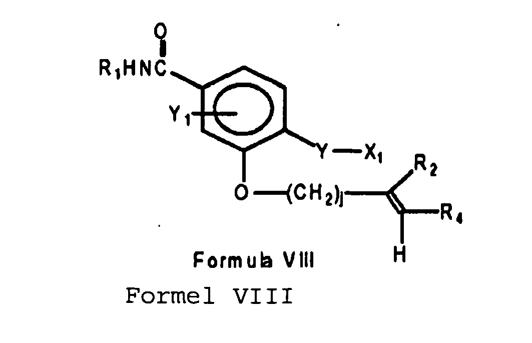
f) Umsetzung der Verbindung der Formel VIII mit einer Verbindung der Formel XI
R₅HC=NOH Formel IX
unter Bildung einer Verbindung der Formel X,
worin alle Substituenten dieselbe Bedeutung wie in Anspruch 1 haben.

13. Verfahren zur Herstellung einer Verbindung der Formel XXII 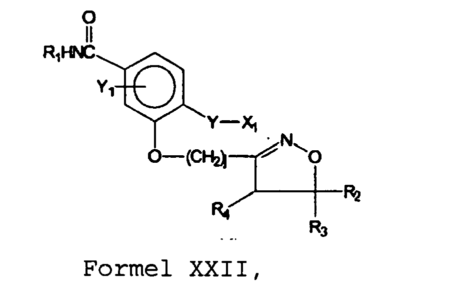 eines pharmazeutisch zulässigen Salzes, pharmazeutisch zulässigen Solvats, Stereoisomers, Tautomers, Razemats, Polymorphs oder N-Oxids davon, umfassend die Schritte
a) Schützen einer Verbindung der Froeml II(a) 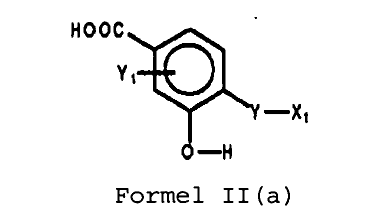 mit einer Verbindung der Formel RX₃ (worin X Halogen sein kann und R eine Hydroxyl schützende Gruppe ist) unter Bildung einer Verbindung der Formel XI, 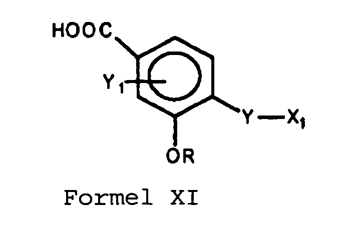
b) Umsetzen der Verbindung der Formel XI mit einer Verbindung der Formel VII unter Bildung einer Verbindung der Formel XII,
R₁NH₂ Formel VII 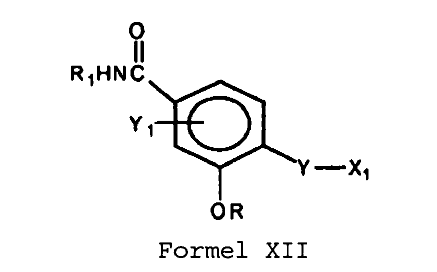
c) Schutzgruppenspaltung von der Verbindung der Formel XII unter Bildung der Verbindung der Formel XIII, 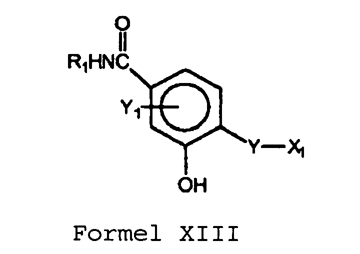
d) N-Schützen der Verbindung der Formel XIII unter Bildung einer Verbindung der Formel XIV (worin Pr eine Amino schützende Gruppe ist), 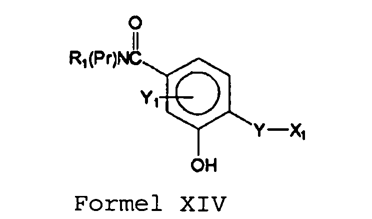
e) Umsetzen der Verbindung der Formel XIV mit einer Verbindung der Formel XV (worin X₃ dasselbe wie früher definiert ist) unter Bildung einer Verbindung der Formel XVI, 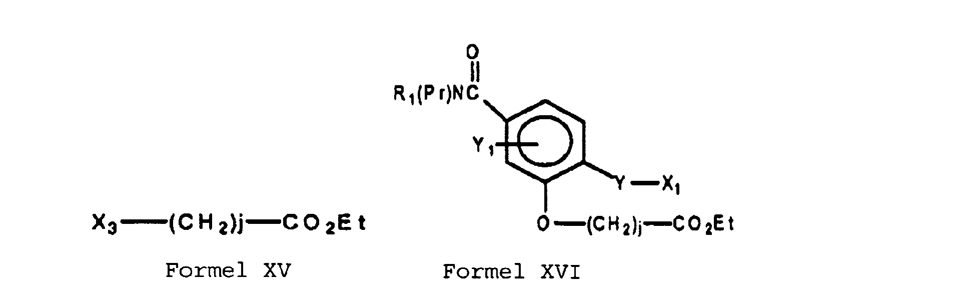
f) Reduzieren der Verbindung der Formel XVI unter Bildung einer Verbindung der Formel XVII, 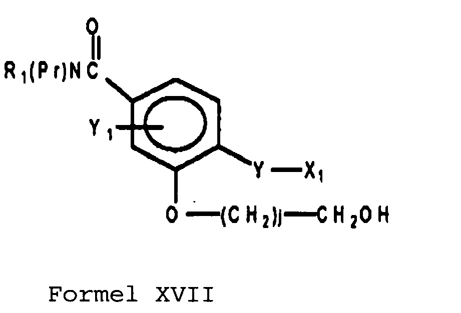
g) Oxydieren der Verbindung der Formel XVII unter Bildung einer Verbindung der Formel XVIII, 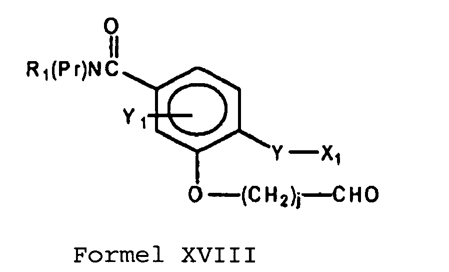
h) Umsetzen der Verbindung der Formel XVIII mit Hydroxylaminhydrochlorid unter Bildung einer Verbindung der Formel XIX, 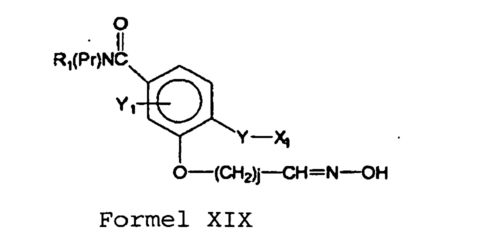
i) Umsetzen der Verbindung der Formel XIX mit einer Verbindung der Formel XX unter Bildung einer Verbindung der Formel XXI und 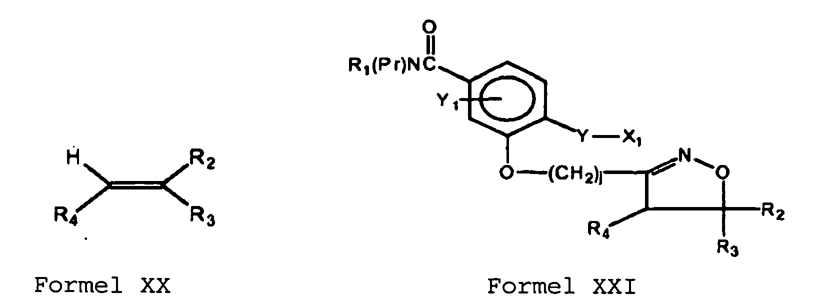
j) Schutzgruppenspaltung der Verbindung der Formel XXI unter Bildung einer Verbindung der Formel XXII, worin alle Substituenten dieselbe Bedeutung wie in Anspruch 1 haben.

14. Verfahren zur Herstellung einer Verbindung der Formel XXVI, 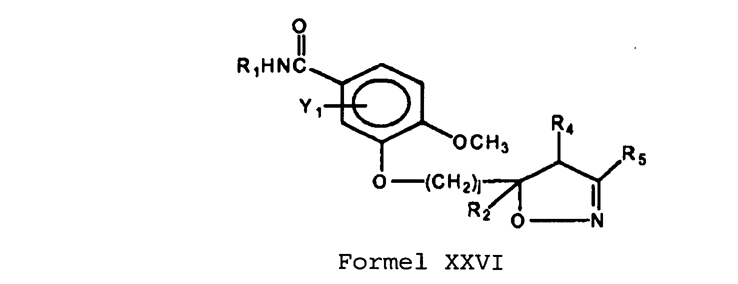 eines pharmazeutisch zulässigen Salzes, pharmazeutisch zulässigen Solvats, Stereoisomers, Tautomers, Razemats, Polymorphs oder N-Oxids davon, umfassend die Schritte
a) Umsetzen einer Verbindung der Formel XXIII mit einer Verbindung der Formel IX 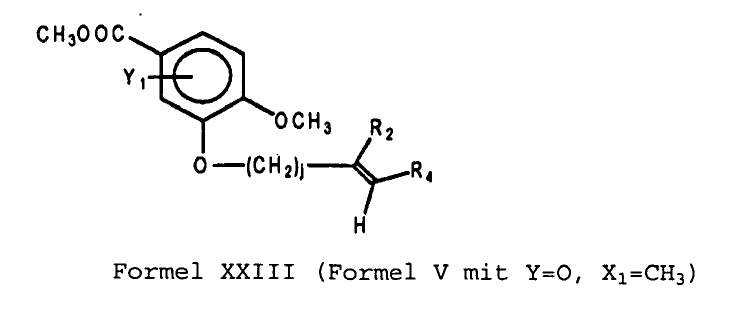 R₅HC=NOH Formel IX
unter Bildung einer Verbindung der Formel XXIV, 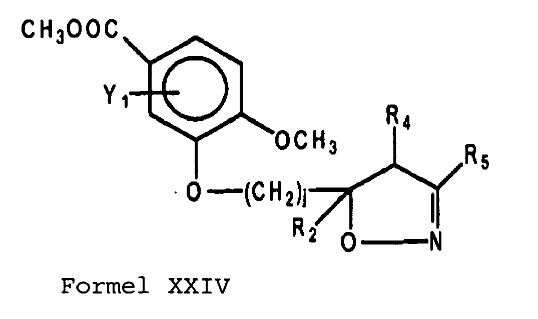
b) Esterhydrolyse der Verbindung der Formel XXIV unter Bildung
einer Verbindung der Formel XXV und 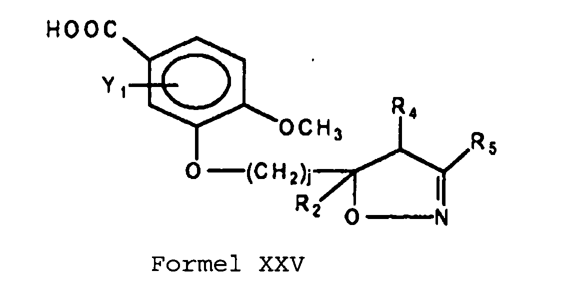
c) Umsetzung der Verbindung der Formel XXV mit einer Verbindung der Formel VII
R₁NH₂ Formel VII
unter Bildung einer Verbindung der Formel XXVI.

15. Verfahren zur Herstellung einer Verbindung der Formel XXVII 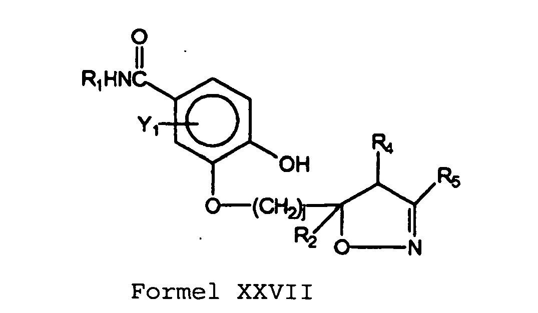 eines pharmazeutisch zulässigen Salzes, pharmazeutisch zulässigen Solvats, Stereoisomers, Tautomers, Razemats, Polymorphs oder N-Oxids davon, umfassend
a) Demethylierung einer Verbindung der Formel XXVI 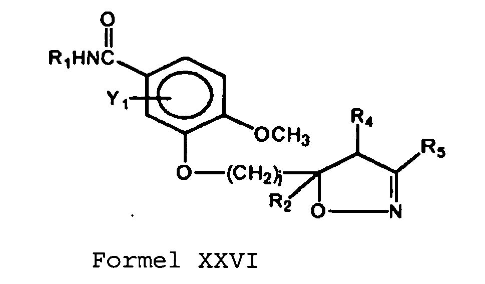 unter Bildung einer Verbindung der Formel XXVII,
wobei alle Substituenten dieselbe Bedeutung wie in Anspruch 1 haben.

16. Verfahren zur Herstellung einer Verbindung der Formel XXIX, 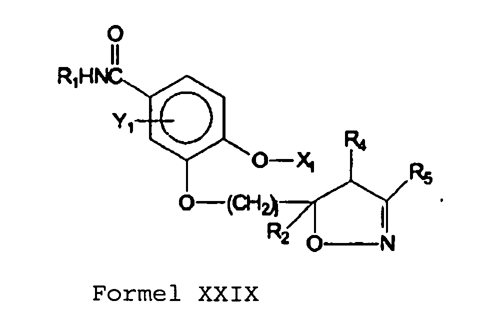 eines pharmazeutisch zulässigen Salzes, pharmazeutisch zulässigen Solvats, Stereoisomers, Tautomers, Razemats, Polymorphs oder N-Oxids davon, umfassend
a) Umsetzung einer Verbindung der Formel XXVII 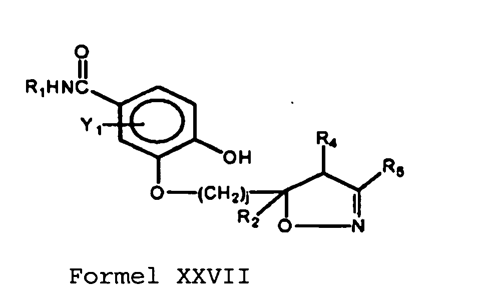 mit einer Verbindung der Formel XXVIII
X₁-h a lid e Formel XXVIII
unter Bildung einer Verbindung der Formel XXIX oder
b)
(i) Demethylieren einer Verbindung der Formel XXIV unter Bildung einer Verbindung der Formel XXX, 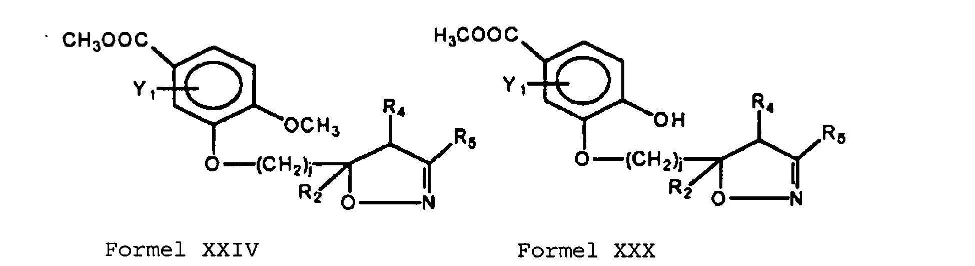
(ii) Umsetzen der Verbindung der Formel XXX mit einer Verbindung der Formel XXVIII unter Bildung einer Verbindung der Formel XXXI, 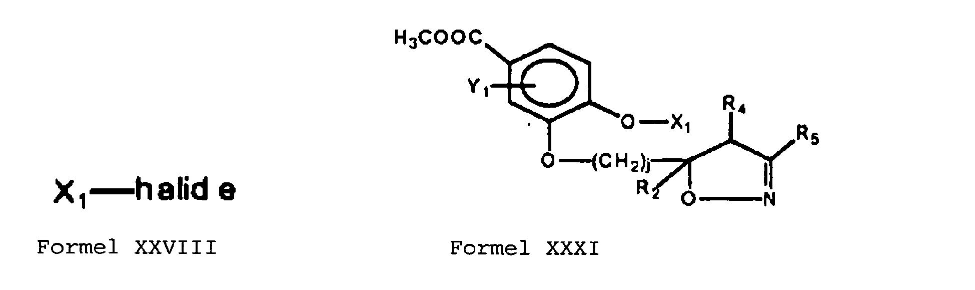
(iii) Esterhydrolyse der Verbindung der Formel XXXI unter Bildung einer Verbindung der Formel XXXII und 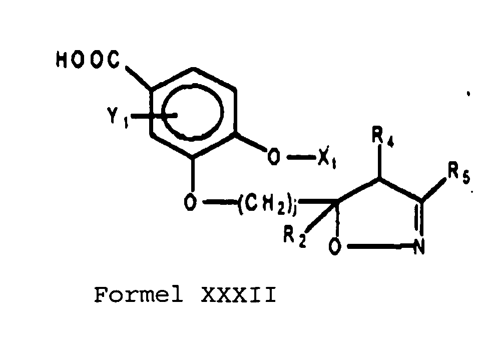
(iv) Umsetzen der Verbindung der Formel XXXII mit einer Verbindung der Formel VII
R₁NH₂ Formel VII
unter Bildung einer Verbindung der Formel XXIX,
worin alle Substituenten dieselbe Bedeutung wie in Anspruch 1 haben.

## Revendications

1. Composé ayant la structure de formule I : 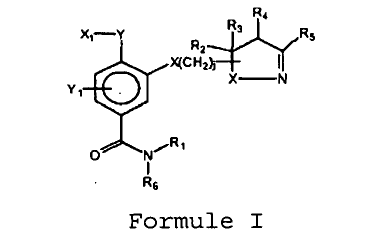 ou un sel acceptable au plan pharmaceutique, un
solvate acceptable au plan pharmaceutique, un stéréoisomère, un tautomère, un racémate, un polymorphe ou un N-oxyde de ce composé, dans laquelle formule :
R₁ et R₆ représentent indépendamment de l'hydrogène, un groupement alkyle, alcényle, alcynyle, cycloalkyle (in)saturé, aryle, aralkyle, hétérocyclyle, hétéroaryle, (hétéroaryl)alkyle ou (hétérocyclyl)- alkyle,
X représente de l'oxygène, du soufre ou NRₕ, où
Rₕ représente de l'hydrogène, un groupement alkyle, alcényle, alcynyle, cycloakyle (in)saturé, acyle, aryle, aralkyle, hétéroaryle, hétérocyclyle, (hétéroaryl) alkyle, (hétérocyclyl) alkyle, S(O)ₙR_{d}, où
R_{d} représente un groupement alkyle, aryle, hétérocyclyle ou hétéroaryle, et
n est un entier de 0 à 2,
Y ne représente pas un atome ou représente de l'oxygène, du soufre ou NRₕ, où Rₕ est tel que défini ci-dessus,
X₁ représente de l'hydrogène, un groupement alkyle, alcényle, alcynyle, cycloalkyle, aryle, aralkyle, hétéroaryle, hétérocyclyle, (hétéroaryl)alkyle ou (hétérocyclyl)alkyle,
Y₁ représente de l'hydrogène, un groupement alkyle, alcényle, alcynyle, nitro, cyano, halogène, ORₕ, SRₕ, NHRₕ, COOR' , COR' , CONHR' , S(O)ₙR_{d}, ou Y₁ peut être fusionné à un anneau de benzène si Y représente un groupement aryle, hétéroaryle ou hétérocyclyle, où
Rₕ et R_{d} sont tels que définis ci-dessus, et
R' représente de l'hydrogène, un groupement alkyle, alcényle, alcynyle, cycloalkyle (in)saturé, aryle, aralkyle, hétérocyclyle, hétéroaryle, (hétéro- cyclyl)alkyle ou (hétéroaryl)alkyle,
j est un entier de 1 à 3,
R₂ et R₃ représentent indépendamment de l'hydrogène, un groupement alkyle, alcényle, alcynyle, cycloalkyle, nitro, cyano, amino, amino substitué, alcoxy, aryloxy, COR', COOR', aryle, aralkyle, hétéroaryle, hétérocyclyle, (hétéroaryl)alkyle, (hétérocyclyl) alkyle, (CH₂)₁₋₄OR''', C (=O) NRₓR_{y} ou (CH₂)ₘ-C(=O)R_{z}, où
R' est tel que défini ci-dessus,
R "' représente de l'hydrogène, un groupement alkyle, alcényle, alcynyle, cycloalkyle (in)saturé, aryle, aralkyle, hétérocyclyle, hétéroaryle, (hétéro- cyclyl)alkyle, (hétéroaryl)alkyle ou hydroxy,
Rₓ et R_{y} représentent indépendamment de l'hydrogène, un groupement alkyle, alcényle de trois à six atomes
m de carbone, alcynyle de trois à six atomes de carbone, cycloalkyle (in)saturé, aryle, aralkyle, hétéroaryle, hétérocyclyle, (hétéroaryl)alkyle ou (hétérocyclyl)alkyle, représente un entier de 0 à 2, et
R_{z} représente Rₚ ou R_{q}, où
Rₚ représente un anneau monocyclique ou bicyclique (in)saturé à 4 à 12 éléments contenant 1 à 4 hétéroatomes sélectionnés parmi N, O ou S, où ledit anneau peut être fixé à (CH₂)ₘC(=O) par N, et
R_{q} est un anneau monocyclique ou bicyclique à 4 à 12 éléments contenant 0 à 4 hétéroatomes sélectionnés dans le groupe constitué de N, O ou S, où ledit anneau peut être fixé à (CH₂)ₘC(=O) par C,
R₄ représente de l'hydrogène, un groupement alkyle, halogène, hydroxy, cyano, carboxy, nitro, C(=O)NRₓR_{y}, où
Rₓ et R_{y} sont tels que définis ci-dessus, et
R₅ représente de l'hydrogène, un groupement alkyle, aryle, aralkyle, cycloakyle (in)saturé, hétéroaryle, hétérocyclyle, (hétéroaryl)alkyle, ou (hétérocyclyl)alkyle,
dans laquelle R₂, R₃, R₄ ou R₅ sont éventuellement remplacés par une liaison représentée, par exemple, par les composés de formule X et de formule XXII, où R₃ et R₅ sont remplacés respectivement par la liaison :

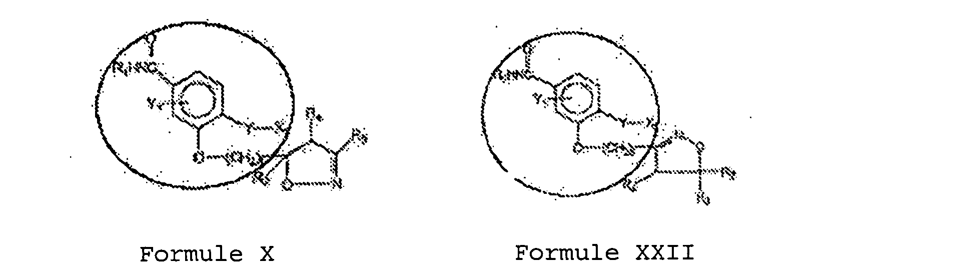

2. Composé selon la revendication 1, dans lequel R₁
représente 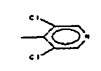 , R₄, R₆ et Y₁ représentent de l'hydrogène,
X représente de l'oxygène, Y ne représente pas un
atome ou représente de l'oxygène, R₂ représente un groupement méthyle, j est 1, R₃ est remplacé par ladite liaison, X₁ est sélectionné parmi de l'hydrogène, un groupement cycloalkyle, alkyle, hétérocyclyle, hétéroaryle, (hétérocyclyl)alkyle ou (hétéroaryl)alkyle et R₅ est sélectionné parmi les groupements alkyle, aryle, cycloalkyle, hétérocyclyle, hétéroaryle, (hétérocyclyl)alkyle ou (hétéroaryl)alkyle.

3. Composé selon la revendication 2, dans lequel X₁ est sélectionné parmi les groupements méthyle, difluorométhyle, éthyle, propyle, isopropyle, cyclopropyle, butyle, cyclopentyle, benzyle ou cycloheptyle et R₅ est sélectionné parmi les groupements méthyle, phényle, 3-pyridyle, 3-cyclopentyloxy-4-méthoxy-phényle, 4-difluoro-méthoxy-phényle, 2-fluorophényle, 2,6-difluorophényle, 4-fluorophényle, 3-fluorophényle, 1-phényléthyle, cyclohexyle, p-tolyle, 4-tert-butylphényle, O-tolyle, 3-méthoxyphényle, ester tert-butylique d'acide pipéridine-1-carboxylique, 3,4-difluorophényle, 2,4-difluorophényle, 4-fluoro-3-méthylphényle ou 3,5-difluorophényle.

4. Composé selon la revendication 1, dans lequel R₁
représente 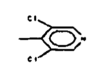 , R₄, R₆ et Y₁ représentent de l'hydrogène, X et Y représentent de l'oxygène, R₃ et X₁ représentent un groupement méthyle, j est 1, R₅ est remplacé par ladite liaison et R₂ est sélectionné parmi le groupement cyano, -COOR' ou C (=O) NRₓR_{y}, où R', Rₓ et R_{y} sont sélectionnés parmi l'hydrogène, un groupement alkyle, alcényle, alcynyle, cycloalkyle (in)saturé, aryle, aralkyle, hétérocyclyle, hétéroaryle, (hétérocyclyl)alkyle ou (hétéroaryl)alkyle.

5. Composé selon la revendication 4, dans lequel R', Rₓ et R_{y} sont sélectionnés parmi les groupements méthyle et éthyle.

6. Composé selon la revendication 1, sélectionné parmi les groupements suivants :
3-[3-(3-cyclopentyloxy-4-méthoxy-phényl)-5-méthyl-4,5-dihydro-isoxazol-5-ylméthoxy]-N-(3,5-dichloro-pyridin-4-yl)-4-méthoxy-benzamide,
N-(3,5-dichloro-pyridin-4-yl)-3-(3,5-diméthyl-4,5-dihydro-isoxazol-5-ylméthoxy)-4-méthoxy-benzamide,
3-(5-cyano-5-méthyl-4,5-dihydro-isoxazol-4-ylméthoxy)-N-(3,5-dichloro-pyridin-4-yl)-4-méthoxy-benzamide,
ester méthylique d'acide 4-[5-(3,5-dichloro-pyridin-4-ylcarbamoyl)-2-méthoxy-phénoxyméthyl]-5-méthyl-4,5-dihydro-isoxazole-5-carboxylique,
amide éthylique d'acide 4-[5-(3,5-dichloro-pyridin-4-ylcarbamoyl)-2-méthoxy-phénoxyméthyl]-5-méthyl-4,5-dihydro-isoxazole-5-carboxylique,
acide 3-[5-(3,5-dichloro-pyridin-4-ylcarbamoyl)-2-méthoxy-phénoxyméthyl]-5-méthyl-4,5-dihydroisoxazole-5-carboxylique,
amide d'acide 3-[5-(3,5-dichloro-pyridin-4-ylcarbamoyl)-2-méthoxy-phénoxyméthyl]-5-méthyl-4,5-dihydro-isoxazole-5-carboxylique,
amide méthylique d'acide 3-[5-(3,5-dichloro-pyridin-4-ylcarbamoyl)-2-méthoxy-phénoxyméthyl]-5-méthyl-4,5-dihydro-isoxazole-5-carboxylique,
amide diméthylique d'acide 3-[5-(3,5-dichloro-pyridin-4-ylcarbamoyl)-2-méthoxy-phénoxyméthyl]-5-méthyl-4,5-dihydro-isoxazole-5-carboxylique,
N-(3,5-dichloro-pyridin-4-yl)-3-(5-méthyl-3-phényl-4,5-dihydro-isoxazol-5-ylméthoxy)-4-méthoxy-benzamide,
N-(3,5-dichloro-pyridin-4-yl)-3-(5-méthyl-3-pyridin-3-yl-4,5-dihydro-isoxazol-5-ylméthoxy)-4-méthoxy-benzamide,
N-(3,5-dichloro-pyridin-4-yl)-3-(3-(4-difluorométhoxyphényl)-5-méthyl-4,5-dihydro-isoxazol-5-ylméthoxy)-4-méthoxy-benzamide,
N-(3,5-dichloro-pyridin-4-yl)-4-difluorométhoxy-3-(3,5-diméthyl-4,5-dihydro-isoxazol-5-ylméthoxy)-benzamide,
N-(3,5-dichloro-pyridin-4-yl)-3-(5-méthyl-3-phényl-4,5-dihydro-isoxazol-5-ylméthoxy)-4-hydroxy-benzamide,
N-(3,5-dichloro-pyridin-4-yl)-3-[3-(2-fluoro-phényl)-5-méthyl-4,5-dihydro-isoxazol-5-ylméthoxy)-4-méthoxy-benzamide,
N-(3,5-dichloro-pyridin-4-yl)-3-[3-(2,6-difluorophényl)-5-méthyl-4,5-dihydro-isoxazol-5-ylméthoxy)-4-méthoxy-benzamide,
N-(3,5-dichloro-pyridin-4-yl)-3-[3,5-diméthyl-4,5-dihydro-isoxazol-5-ylméthoxy)-4-hydroxy-benzamide,
N-(3,5-dichloro-pyridin-4-yl)-3-[3-(4-fluoro-phényl)-5-méthyl-4,5-dihydro-isoxazol-5-ylméthoxy)-4-méthoxy-benzamide,
N-(3,5-dichloro-pyridin-4-yl)-3-[3-(3-fluoro-phényl)-5-méthyl-4,5-dihydro-isoxazol-5-ylméthoxy)-4-hydroxy-benzamide,
N-(3,5-dichloro-pyridin-4-yl)-3-[3-(3-fluoro-phényl)-5-méthyl-4,5-dihydro-isoxazol-5-ylméthoxy)-4-méthoxy-benzamide,
N-(3,5-dichloro-pyridin-4-yl)-4-éthoxy-3-(5-méthyl-3-phényl-4,5-dihydro-isoxazol-5-ylméthoxy)benzamide,
N-(3,5-dichloro-pyridin-4-yl)-4-éthoxy-3-[3-(3-fluorophényl)-5-méthyl-4,5-dihydro-isoxazol-5-ylméthoxy]benzamide,
N-(3,5-dichloro-pyridin-4-yl)-3-(5-méthyl-3-phényl-4,5-dihydro-isoxazol-5-ylméthoxy)-4-propoxy-benzamide,
N-(3,5-dichloro-pyridin-4-yl)-3-[3-(3-fluoro-phényl)-5-méthyl-4,5-dihydro-isoxazol-5-ylméthoxy]-4-propoxy-benzamide,
N-(3,5-dichloro-pyridin-4-yl)-4-isopropoxy-3-(5-méthyl-3-phényl-4,5-dihydro-isoxazol-5-ylméthoxy)-benzamide,
N-(3,5-dichloro-pyridin-4-yl)-3-[3-(3-fluoro-phényl)-5-méthyl-4,5-dihydro-isoxazol-5-ylméthoxy]-4-isopropoxy-benzamide,
4-cyclopropoxy-N-(3,5-dichloro-pyridin-4-yl)-3-(5-méthyl-3-phényl-4,5-dihydro-isoxazol-5-ylméthoxy)-benzamide,
4-cyclopropoxy-N-(3,5-dichloro-pyridin-4-yl)-3-[3-(3-fluoro-phényl)-5-méthyl-4,5-dihydro-isoxazol-5-ylméthoxy]benzamide,
4-butoxy-N-(3,5-dichloro-pyridin-4-yl)-3-(5-méthyl-3-phényl-4,5-dihydro-isoxazol-5-ylméthoxy)benzamide,
4-butoxy-N-(3,5-dichloro-pyridin-4-yl)-3-[3-(3-fluorophényl)-5-méthyl-4,5-dihydro-isoxazol-5-ylméthoxy]benzamide,
4-cyclopentyloxy-N-(3,5-dichloro-pyridin-4-yl)-3-(5-méthyl-3-phényl-4,5-dihydro-isoxazol-5-ylméthoxy)-benzamide,
4-cyclopentyloxy-N-(3,5-dichloro-pyridin-4-yl)-3-[3-(3-fluoro-phényl)-5-méthyl-4,5-dihydro-isoxazol-5-ylméthoxy]benzamide,
N-(3,5-dichloro-pyridin-4-yl)-3-(3,5-diméthyl-4,5-dihydro-isoxazol-5-ylméthoxy)-4-éthoxy-benzamide,
N-(3,5-dichloro-pyridin-4-yl)-3-(3,5-diméthyl-4,5-dihydro-isoxazol-5-ylméthoxy)-4-propoxy-benzamide,
N-(3,5-dichloro-pyridin-4-yl)-4-isopropoxy-3-(5-méthyl-3-pyridin-3-yl-4,5-dihydro-isoxazol-5-ylméthoxy)benzamide,
N-(3,5-dichloro-pyridin-4-yl)-3-(3,5-diméthyl-4,5-dihydro-isoxazol-5-ylméthoxy)-4-isopropoxy-benzamide,
4-cyclopropoxy-N-(3,5-dichloro-pyridin-4-yl)-3-(5-méthyl-3-pyridin-3-yl-4,5-dihydro-isoxazol-5-ylméthoxy)benzamide,
4-cyclopropoxy-N-(3,5-dichloro-pyridin-4-yl)-3-(3,5-diméthyl-4,5-dihydro-isoxazol-5-ylméthoxy)-benzamide,
4-butoxy-N-(3,5-dichloro-pyridin-4-yl)-3-(5-méthyl-3-pyridin-3-yl-4,5-dihydro-isoxazol-5-ylméthoxy)-benzamide,
4-butoxy-N-(3,5-dichloro-pyridin-4-yl)-3-(3,5-diméthyl-4,5-dihydro-isoxazol-5-ylméthoxy)-benzamide,
4-cyclopentyloxy-N-(3,5-dichloro-pyridin-4-yl)-3-(5-méthyl-3-pyridin-3-yl-4,5-dihydro-isoxazol-5-ylméthoxy)benzamide,
4-cyclopentyloxy-N-(3,5-dichloro-pyridin-4-yl)-3-(3,5-diméthyl-4,5-dihydro-isoxazol-5-ylméthoxy)-benzamide,
N-(3,5-dichloro-pyridin-4-yl)-4-hydroxy-3-(5-méthyl-3-pyridin-3-yl-4,5-dihydro-isoxazol-5-ylméthoxy)-benzamide,
N-(3,5-dichloro-pyridin-4-yl)-4-méthoxy-3-[5-méthyl-3-(1-phényl-éthyl)-4,5-dihydro-isoxazol-5-ylméthoxy]benzamide,
3-(3-cyclohexyl-5-méthyl-4,5-dihydro-isoxazol-5-ylméthoxy)-N-(3,5-dichloro-pyridin-4-yl)-4-méthoxy-benzamide,
N-(3,5-dichloro-pyridin-4-yl)-4-méthoxy-3-(5-méthyl-3-p-tolyl-4,5-dihydro-isoxazol-5-ylméthoxy)-benzamide,
3-[3-(4-tert-butyl-phényl)-5-méthyl-4,5-dihydro-isoxazol-5-ylméthoxy]-N-(3,5-dichloro-pyridin-4-yl)-4-méthoxy-benzamide,
N-(3,5-dichloro-pyridin-4-yl)-4-méthoxy-3-(5-méthyl-3-O-tolyl-4,5-dihydro-isoxazol-5-ylméthoxy)-benzamide,
N-(3,5-dichloro-pyridin-4-yl)-4-méthoxy-3-[3-(3-méthoxy-phényl)-5-méthyl-4,5-dihydro-isoxazol-5-ylméthoxy]benzamide,
ester tert-butylique d'acide 2-{5-[5-(3,5-dichloro-pyridin-4-ylcarbamoyl)-2-méthoxy-phénoxyméthyl]-5-méthyl-4,5-dihydro-isoxazol-3-yl)-pipéridine-1-carboxylique,
N-(3,5-dichloro-pyridin-4-yl)-3-[3-(3,4-difluorophényl)-5-méthyl-4,5-dihydro-isoxazol-5-ylméthoxy]-4-méthoxy-benzamide,
N-(3,5-dichloro-pyridin-4-yl)-3-[3-(2,4-difluorophényl)-5-méthyl-4,5-dihydro-isoxazol-5-ylméthoxy]-4-méthoxy-benzamide,
N-(3,5-dichloro-pyridin-4-yl)-3-[3-(4-fluoro-3-méthylphényl)-5-méthyl-4,5-dihydro-isoxazol-5-ylméthoxy]-4-méthoxy-benzamide,
N-(3,5-dichloro-pyridin-4-yl)-3-[3-(3,5-difluorophényl)-5-méthyl-4,5-dihydro-isoxazol-5-ylméthoxy]-4-méthoxy-benzamide,
4-benzyloxy-N-(3,5-dichloro-pyridin-4-yl)-3-(3,5-diméthyl-4,5-dihydro-isoxazol-5-ylméthoxy)-benzamide,
4-cycloheptyloxy-N-(3,5-dichloro-pyridin-4-yl)-3-(3,5-diméthyl-4,5-dihydro-isoxazol-5-ylméthoxy)-benzamide,
3-[3-(3-cyclopentyloxy-4-méthoxy-phényl)-5-méthyl-4,5-dihydro-isoxazol-5-ylméthoxy]-N-(3,5-dichloro-pyridin-4-yl)benzamide,
ou un sel acceptable au plan pharmaceutique, un solvate acceptable au plan pharmaceutique, un stéréoisomère, un tautomère, un racémate, un polymorphe ou un N-oxyde de ces composés.

7. Composition pharmaceutique comprenant une quantité efficace au plan thérapeutique d'un ou plusieurs composés selon la revendication 1 et d'un ou plusieurs véhicules, excipients ou diluants acceptables au plan pharmaceutique.

8. Composition pharmaceutique selon la revendication 7, comprenant en outre un ou plusieurs des agents thérapeutiques sélectionnés parmi les corticostéroïdes, les bêta-agonistes, les antagonistes de leukotriène, les inhibiteurs de 5-lipoxygénase, les inhibiteurs de chimiokine ou des antagonistes de récepteurs muscariniques.

9. Composé selon la revendication 1 pour usage dans le traitement, la prévention, l'inhibition ou la suppression d'un état inflammatoire ou d'une maladie chez un patient.

10. Composition pharmaceutique selon la revendication 7 pour usage dans le traitement, la prévention, l'inhibition ou la suppression d'un état inflammatoire ou d'une maladie chez un patient.

11. Composé de formule I et un ou plusieurs véhicules, excipients ou diluants acceptables au plan pharmaceutique pour usage dans le traitement, la prévention, l'inhibition ou la suppression des maladies suivantes : SIDA, asthme, arthrite, bronchite, maladie pulmonaire obstructive chronique (COPD), psoriasis, rhinite allergique, choc, dermatite atopique, maladie de Crohn, syndrome de détresse respiratoire chez l'adulte (ARDS), granulome éosinophile, conjonctivite allergique, ostéoarthrite, colite ulcérative ou autres maladies inflammatoires chez un patient.

12. Procédé pour préparer un composé de formule X : 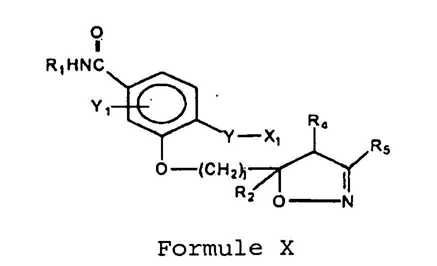 d'un sel acceptable au plan pharmaceutique, d'un
solvate acceptable au plan pharmaceutique, d'un stéréoisomère, d'un tautomère, d'un racémate, d'un polymorphe ou d'un N-oxyde de ce composé, comprenant les étapes consistant à :
a) oxyder un composé de formule II, 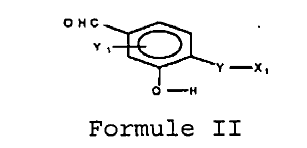 pour former un composé de formule II(a), 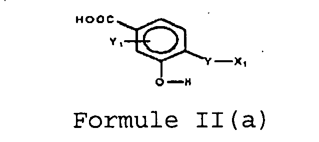
b) estérifier le composé de formule II(a) pour former un composé de formule III, 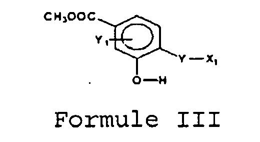
c) faire réagir le composé de formule III avec un composé de formule IV (dans laquelle X₃ est un halogène) 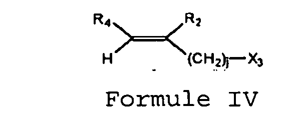 pour former un composé de formule V : 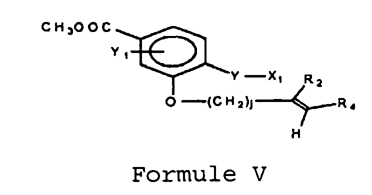
d) hydrolyser l'ester du composé de formule V pour former un composé de formule VI : 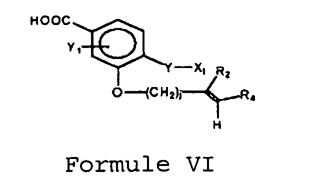
e) faire réagir le composé de formule VI avec un composé de formule VII pour former un composé de formule VIII : 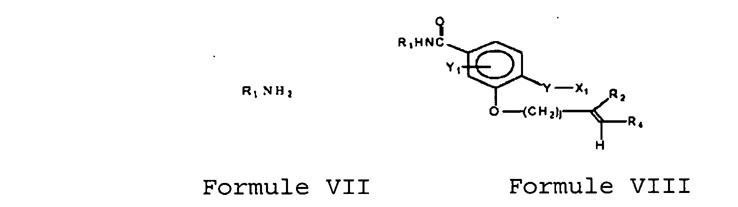
f) faire réagir le composé de formule VIII avec un
composé de formule IX :
R₅HC=NOH Formule IX
pour former un composé de formule X, dans laquelle
tous les substituants ont la même signification que dans la revendication 1.

13. Procédé pour préparer un composé de formule XXII : 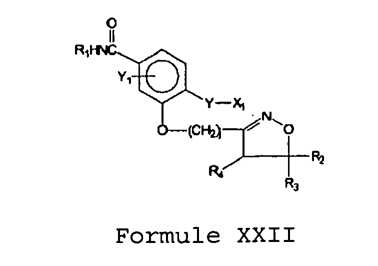 d'un sel acceptable au plan pharmaceutique, d'un
solvate acceptable au plan pharmaceutique, d'un stéréoisomère, d'un tautomère, d'un racémate, d'un polymorphe ou d'un N-oxyde de ce composé, comprenant les étapes consistant à :
a) protéger un composé de formule II(a) : 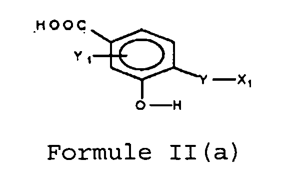 avec un composé de formule RX₃ (dans laquelle X₃ peut
être un halogène et R représente un groupement de protection hydroxy) pour former un composé de formule XI : 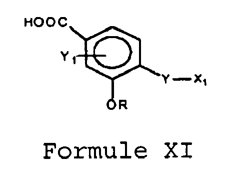
b) faire réagir le composé de formule XI avec un composé de formula VII pour former un composé de formule XI : 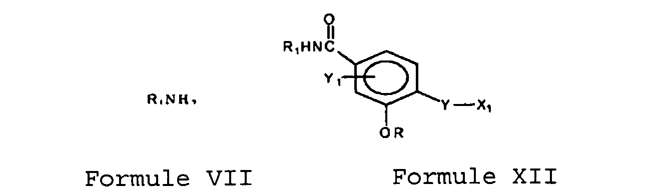
c) déprotéger le composé de formule XII pour former un composé de formule VIII : 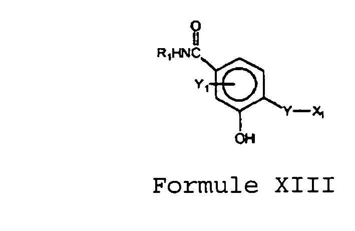
d) N-protéger le composé de formule XIII pour former un composé de formule XIV (dans laquelle Pr est un
groupement de protection amino), 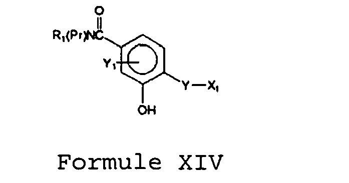
e) faire réagir le composé de Formule XIV avec un composé de formule XV (dans laquelle X₃ est tel que défini plus haut) pour former un composé de formule XVI : 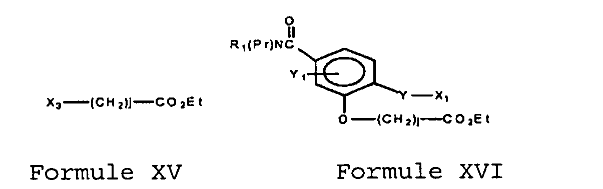
f) réduire le composé de formule XVI pour former un composé de formule XVII : 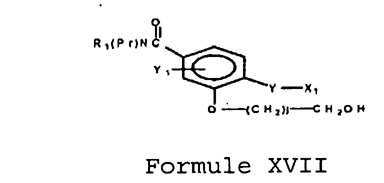
g) oxyder le composé de formule XVII pour former un composé de formule XVIII : 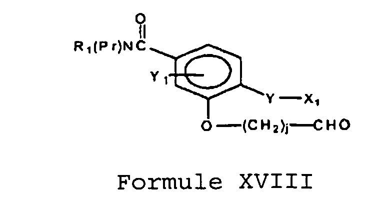
h) faire réagir le composé de formule XVIII avec du chlorhydrate d'hydroxylamine pour former un composé de formule XIX : 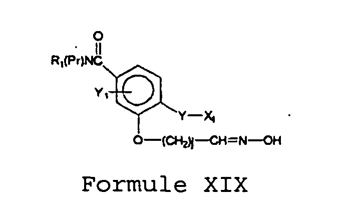
i) faire réagir le composé de formule XIX avec un composé de formule XX pour former un composé de formule XXI : 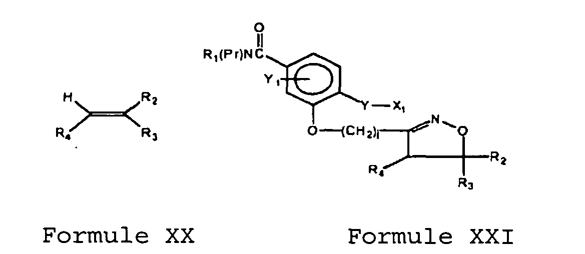
j) déprotéger le composé de formule XXI pour former un composé de formule XXII, dans laquelle tous les substituants ont la même signification que dans la revendication 1.

14. Procédé pour préparer un composé de formule XXVI : 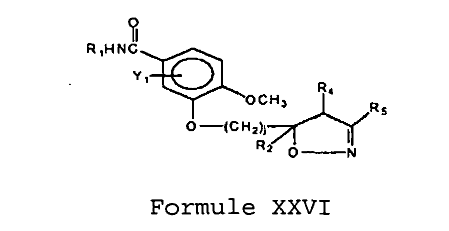 d'un sel acceptable au plan pharmaceutique, d'un
solvate acceptable au plan pharmaceutique, d'un stéréoisomère, d'un tautomère, d'un racémate, d'un polymorphe ou d'un N-oxyde de ce composé, comprenant les étapes consistant à :
a) faire réagir un composé de formule XXIII avec un composé de formule IX : 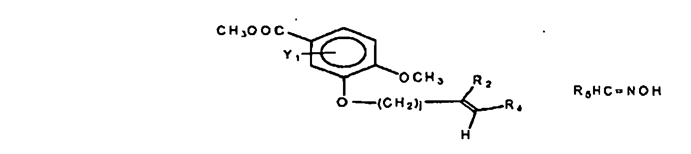 Formule XXIII (Formule V, Formule IX
dans laquelle Y=O, X₁=CH₃) pour former un composé de formule XXIV : 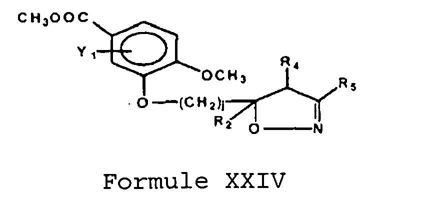
b) hydrolyser l'ester du composé de formule XXIV pour former un composé de formule XXV : 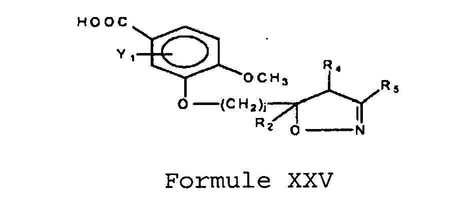
c) faire réagir le composé de formule XXV avec un composé de formule VII :
R₁NH₂ Formule VII
pour former un composé de formule XXVI, dans laquelle
tous les substituants ont la même signification que dans la revendication 1.

15. Procédé pour préparer un composé de formule XXVII : 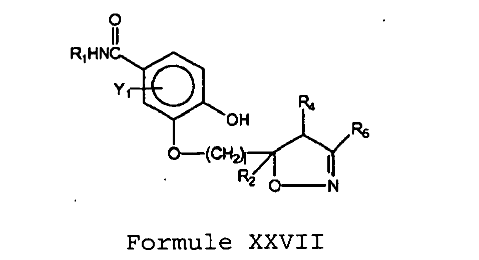 d'un sel acceptable au plan pharmaceutique, d'un
solvate acceptable au plan pharmaceutique, d'un stéréoisomère, d'un tautomère, d'un racémate, d'un polymorphe ou d'un N-oxyde de ce composé, comprenant les étapes consistant à :
a) déméthyler un composé de formule XXVI : 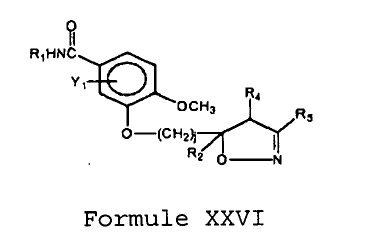 pour former un composé de formule XXVII, dans laquelle
tous les substituants ont la même signification que dans la revendication 1.

16. Procédé pour préparer un composé de formule XXIX : 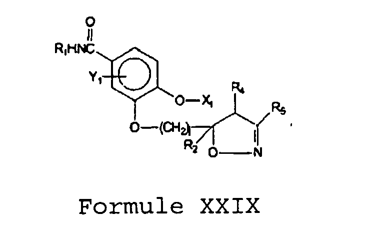 d'un sel acceptable au plan pharmaceutique, d'un
solvate acceptable au plan pharmaceutique, d'un stéréoisomère, d'un tautomère, d'un racémate, d'un polymorphe ou d'un N-oxyde de ce composé, comprenant les étapes consistant à :
(a) faire réagir un composé de formule XXVII : 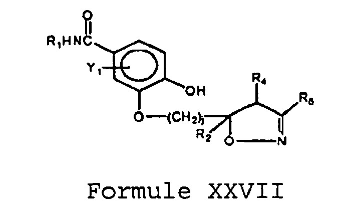 avec un composé de formule XXVIII :
X₁-halogénure Formule XXVIII
pour former un composé de formule XXIX ; ou
(b)
(i) déméthyler un composé de formule XXIV pour former un composé de formule XXX : 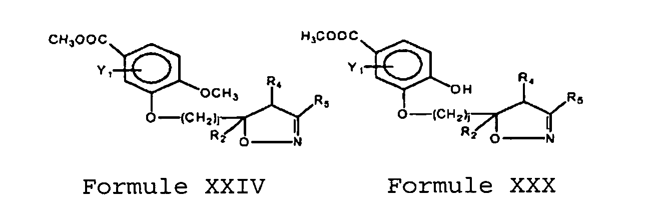
(ii) faire réagir le composé de formule XXX avec un composé de formula XXVIII pour former un composé de formule XXXI : 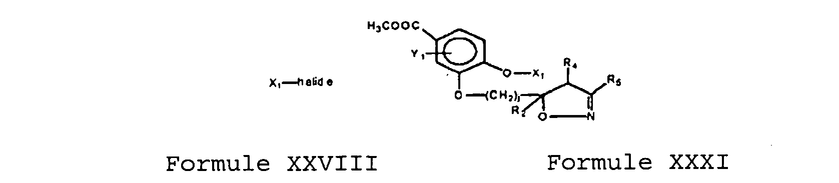
(iii) hydrolyser l'ester du composé de formule XXXI pour former un composé de formule XXXII : 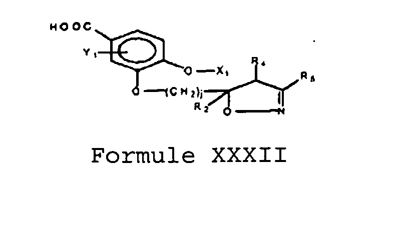
(iv) faire réagir le composé de formule XXXII avec un composé de formule VII :
R₁NH₂ Formule VII
pour former un composé de formule XXIX, dans laquelle
tous les substituants ont la même signification que dans la revendication 1.
